(19) Europäisches Patentamt
European Patent Office
Office européen des brevets

(11) **EP 2 970 477 B1**

(12) # EUROPEAN PATENT SPECIFICATION

(45) Date of publication and mention
of the grant of the patent:
**04.10.2023 Bulletin 2023/40**

(21) Application number: **14717954.3**

(22) Date of filing: **14.03.2014**

(51) International Patent Classification (IPC):
***C07K 16/28*** *(2006.01)* ***C07K 16/32*** *(2006.01)*
***C07K 16/46*** *(2006.01)*

(52) Cooperative Patent Classification (CPC):
**C07K 16/28; A61P 1/16; A61P 9/10; A61P 11/00;
A61P 11/08; A61P 13/12; A61P 19/04;
A61P 29/00; A61P 31/04; A61P 31/12;
A61P 33/00; A61P 33/02; A61P 35/00;
A61P 35/02; A61P 37/06;** (Cont.)

(86) International application number:
**PCT/US2014/029253**

(87) International publication number:
**WO 2014/144722 (18.09.2014 Gazette 2014/38)**

(54) **BISPECIFIC-FC MOLECULES**

BISPEZIFISCHE FC-MOLEKÜLE

MOLÉCULES FC BISPÉCIFIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **15.03.2013 US 201361791424 P**

(43) Date of publication of application:
**20.01.2016 Bulletin 2016/03**

(73) Proprietor: **Amgen Inc.**
**Thousand Oaks, California 91320-1799 (US)**

(72) Inventors:
• **BORGES, Luis G.**
**Redwood City**
**CA 94603 (US)**
• **BAEUERLE, Patrick A.**
**82131 Gauting (DE)**
• **YAN, Wei**
**Sammamish**
**Washington 98075 (US)**
• **MICHAELS, Mark L.**
**Encino**
**California 91316 (US)**

(74) Representative: **Schiweck Weinzierl Koch
Patentanwälte Partnerschaft mbB
Ganghoferstraße 68 B
80339 München (DE)**

(56) References cited:
WO-A1-2009/018386   WO-A1-2011/063348
WO-A1-2012/135345   WO-A1-2013/026837
WO-A1-2013/055809   WO-A2-2009/052081
WO-A2-2009/088805   WO-A2-2010/037837
WO-A2-2012/143524

• **KIPRIYANOV S M ET AL: "Effect of Domain Order
on the Activity of Bacterially Produced Bispecific
Single-chain Fv Antibodies", JOURNAL OF
MOLECULAR BIOLOGY, ACADEMIC PRESS,
UNITED KINGDOM, vol. 330, no. 1, 27 June 2003
(2003-06-27), pages 99-111, XP004445110, ISSN:
0022-2836, DOI: 10.1016/S0022-2836(03)00526-6**
• **T. YING ET AL: "Soluble Monomeric IgG1 Fc",
JOURNAL OF BIOLOGICAL CHEMISTRY, vol.
287, no. 23, 1 June 2012 (2012-06-01), pages
19399-19408, XP055063702, ISSN: 0021-9258,
DOI: 10.1074/jbc.M112.368647**

**(Cont. next page)**

- DEYEV SERGEY M ET AL: "Multivalency: the hallmark of antibodies used for optimization of tumor targeting by design", BIOESSAYS, JOHN WILEY & SONS LTD, GB, vol. 30, no. 9, 1 September 2008 (2008-09-01), pages 904-918, XP002609474, ISSN: 0265-9247, DOI: 10.1002/BIES.20805 [retrieved on 2008-08-08]
- ALT M ET AL: "Novel tetravalent and bispecific IgG-like antibody molecules combining single-chain diabodies with the immunoglobulin gamma1 Fc or CH3 region", FEBS LETTERS, ELSEVIER, AMSTERDAM, NL, vol. 454, no. 1-2, 2 July 1999 (1999-07-02), pages 90-94, XP027291954, ISSN: 0014-5793 [retrieved on 1999-07-02]
- PARK S S ET AL: "Generation and characterization of a novel tetravalent bispecific antibody that binds to hepatitis B virus surface antigens", MOLECULAR IMMUNOLOGY, PERGAMON, GB, vol. 37, no. 18, 1 December 2000 (2000-12-01), pages 1123-1130, XP002266827, ISSN: 0161-5890, DOI: 10.1016/S0161-5890(01)00027-X
- FOURNIER PHILIPPE ET AL: "Bispecific antibodies and trispecific immunocytokines for targeting the immune system against cancer: preparing for the future.", BIODRUGS : CLINICAL IMMUNOTHERAPEUTICS, BIOPHARMACEUTICALS AND GENE THERAPY FEB 2013, vol. 27, no. 1, February 2013 (2013-02), pages 35-53, XP008170163, ISSN: 1173-8804
- K. Gunasekaran ET AL: "Enhancing Antibody Fc Heterodimer Formation through Electrostatic Steering Effects: APPLICATIONS TO BISPECIFIC MOLECULES AND MONOVALENT IgG", Journal of Biological Chemistry, vol. 285, no. 25, 16 April 2010 (2010-04-16), pages 19637-19646, XP055303838, US ISSN: 0021-9258, DOI: 10.1074/jbc.M110.117382
- WEISSER N E ET AL: "Applications of single-chain variable fragment antibodies in therapeutics and diagnostics", BIOTECHNOLOGY ADVANCES, ELSEVIER PUBLISHING, BARKING, GB, vol. 27, no. 4, 1 July 2009 (2009-07-01), pages 502-520, XP026127899, ISSN: 0734-9750, DOI: 10.1016/J.BIOTECHADV.2009.04.004 [retrieved on 2009-04-15]

Remarks:
The complete document including Reference Table(s) and the Sequence Listing(s) can be downloaded from the EPO website

(52) Cooperative Patent Classification (CPC): (Cont.)
**C07K 16/00; C07K 16/2803; C07K 16/2809; C07K 16/32; C07K 16/468;** A61K 2039/505; C07K 2317/31; C07K 2317/52; C07K 2317/60; C07K 2317/622; C07K 2317/64; C07K 2317/71; C07K 2317/73; C07K 2317/92; C07K 2319/00

**Description**

**Field**

This invention is in the field of protein engineering.

**Background**

Bispecific antibodies have promise as therapeutics in a variety of indications. Bispecific antibodies having a standard IgG format can be challenging to produce because they include four different polypeptide chains. The efficacy of a smaller, more easily-produced bispecific molecule has been clinically demonstrated in non-Hodgkin's lymphoma. *See, e.g.,* Bargou et al. (2008), Science 321(5891): 974-977. Bispecific antigen binding molecules for T cell activation and re-direction to specific target cells are also disclosed in WO 2013/026837. Prolonged administration by continuous intravenous infusion was used to achieve these results because of the short *in vivo* half life of this small, single chain molecule. *Id*. Hence, there is a need in the art for bispecific therapeutics that retain similar therapeutic efficacy, that have a format that is straightforward to produce, and that have favorable pharmacokinetic properties, including a longer half-life.

**Summary**

The invention is set out in the appended set of claims.
A Bispecific-Fc (Bi-Fc) as described herein can bind to two different proteins and contains an Fc region of an antibody or a portion thereof. A Bi-Fc can have favorable pharmacokinetic properties relative to a bispecific single chain molecule lacking an Fc region. One protein bound by a Bi-Fc can be expressed on an immune effector cell such as a T cell, an NK cell, a neutrophil, or a macrophage, and the other protein can be expressed on a target cell, for example, a cancer cell, a cell infected by a pathogen, or a cell mediating a disease, such as a fibroblast causing fibrosis. The Bi-Fc molecules described herein can elicit activation of an immune effector cell in the presence of a target cell and/or killing of a target cell in the presence of an immune effector cell.
In one aspect, the invention relates to a bispecific Fc (Bi-Fc) polypeptide, which is heterodimeric and comprises

(a)

(i) a first polypeptide chain comprising an amino acid sequence having the following formula: V1-L1-V2-L2-V3-L3-V4-L4-Fc; wherein Fc is a human IgG Fc polypeptide chain which is C-terminal relative to the four immunoglobulin variable regions; wherein V1, V2, V3, and V4 are each immunoglobulin variable regions; wherein L1, L2, L3, and L4 are linkers; and wherein L4 can be present or absent; and
(ii) a second polypeptide chain that comprises a human IgG Fc polypeptide chain; or

(b)

(i) a first polypeptide chain having the following formula: Fc-L4-V1-L1-V2-L2-V3-L3-V4; wherein Fc is a human IgG Fc polypeptide chain which is N-terminal relative to the four immunoglobulin variable regions; wherein V1, V2, V3, and V4 are each immunoglobulin variable regions; wherein L1, L2, L3, and L4 are linkers; and wherein L4 can be present or absent; and
(ii) a second polypeptide chain that comprises a human IgG Fc polypeptide chain;

wherein the Bi-Fc binds to a target cell and an immune effector cell and/or mediates cytolysis of a target cell by an immune effector cell,
wherein L1 and L3 are at least 15 amino acids long,
wherein L2 is less than 12 amino acids long,
wherein either V1 is a VH region and V2 is a VL region or vice versa,
wherein either V3 is a VH region and V4 is a VL region or vice versa,
wherein the Bi-Fc binds to human and/or cynomolgus CD3ε,
wherein the Fc polypeptide chain(s) comprise(s) an insertion of the amino acid sequence of any of SEQ ID NOs:36-47 between positions 384 and 385 of each Fc polypeptide chain, wherein positions 384 and 385 are positions assigned according to the EU numbering scheme.

V1 and V2 can bind to a target cell or an immune effector cell when they are part of an IgG and/or an scFv antibody, and V3 and V4 can bind to a target cell or an immune effector cell when they are part of an IgG and/or an scFv antibody. The Fc polypeptide chain in the first polypeptide chain can comprise a heterodimerizing alteration, and the Fc polypeptide chain in the second polypeptide chain can comprise another heterodimerizing alteration. The heterodimerizing alteration in the first polypeptide chain can be a charge pair substitution, and the heterodimerizing alteration in the second polypeptide chain can be a charge pair substitution. The first polypeptide chain can comprise the charge pair substitutions K409D, or K409E and K392D or K392E, and the second polypeptide chain can comprise the charge pair substitutions D399K or D399R and D356K, or D356R; or the second polypeptide chain can comprise the charge pair substitutions K409D, or K409E and K392D or K392E, and the first polypeptide chain can comprise the charge pair substitutions D399K or D399R and D356K, or D356R. The Fc polypeptide chains of the first and second polypeptide chains arehuman IgG Fc polypeptide chains, such as IgG1, IgG2, IgG3, or IgG4 Fc polypeptide chains. The Fc polypeptide chains of the first and second polypeptide chains can comprise one or more alterations that inhibit(s) Fc gamma receptor (Fc$\gamma$R) binding or enhance(s) ADCC. The Fc polypeptide chains of the first and second polypeptide chains comprise, for example, L234A, L235A, and any substitution at N297.

In a further aspect, is the invention relates to a bispecific Fc (Bi-Fc) polypeptide, which is heterodimeric comprising:

(i) a first polypeptide chain having following formula: V1-L1-V2-L2-V3-L3-V4-L4-Fc, wherein Fc is a human IgG Fc polypeptide chain, which is C-terminal relative to the four immunoglobulin variable regions, wherein V1, V2, V3, and V4 are each immunoglobulin variable regions that have different amino acid sequences, wherein L1, L2, L3, and L4 are linkers, and wherein L4 can be present or absent; and (ii) a second polypeptide chain comprising a human IgG Fc polypeptide chain; wherein L1 and L3 are at least 15 amino acids long and L2 is less than 12 amino acids long; wherein either V1 is a VH region and V2 is a VL region or V1 is a VL region and V2 is a VH region; wherein either V3 is a VH region and V4 is a VL region or V3 is a VL region and V4 is a VH region; wherein the Fc polypeptide chains of each of the first and second polypeptide chains each contain a heterodimerizing alteration; and wherein the Bi-Fc mediates cytolysis of a target cell displaying a target cell protein by an immune effector cell, and does not mediate cytolysis of a cell not displaying the target cell protein by the immune effector cell, and/or the Bi-Fc can bind to a target cell and to an immune effector cell, wherein the Bi-Fc binds to human and/or cynomolgus CD3$\varepsilon$, and wherein the Fc polypeptide chain(s) comprise(s) an insertion of the amino acid sequence of any of SEQ ID NOs:36-47 between positions 384 and 385 of each Fc polypeptide chain, wherein positions 384 and 385 are positions assigned according to the EU numbering scheme. The Fc polypeptide chains are human IgG Fc polypeptide chains, such as IgG1, IgG2, IgG3, or IgG4 Fc polypeptide chains. The Fc polypeptide chains of the first and second polypeptide chains can comprise one or more alteration that inhibits Fc$\gamma$R binding, such as one or more of L234A, L235A, and any substitution at N297.

In a further aspect, the invention relates to a bispecif Fc (Bi-Fc) polypeptide, which is monomeric and comprises:

(a) a polypeptide chain comprising an amino acid sequence having the following formula: V1-L1-V2-L2-V3-L3-V4-L4-Fc; wherein Fc is a human IgG Fc polypeptide chain which is C-terminal relative to the four immunoglobulin variable regions;

wherein two of V1, V2, V3, and V4 are immunoglobulin heavy chain variable (VH) regions and the other two are immunoglobulin light chain variable (VL) regions;
wherein either V1 is a VH region and V2 is a VL region or vice versa and either V3 is a VH region and V4 is a VL region or vice versa;
wherein L1, L2, L3, and L4 are linkers, wherein L2 is present and wherein L2 is not more than 12 amino acids long;
wherein L1 and L3 are each at least 15 amino acids long; and
wherein L4 can be present or absent; or

(b) a polypeptide chain comprising an amino acid sequence having the following formula: Fc-L4-V1-L1-V2-L2-V3-L3-V4; wherein Fc is a human IgG Fc polypeptide chain which is N-terminal relative to the four immunoglobulin variable regions;

wherein either V1 is a VH region and V2 is a VL region or vice versa and either V3 is a VH region and V4 is a VL region or vice versa;
wherein two of V1, V2, V3, and V4 are VH regions and the other two are VL regions;
wherein L1, L2, L3, and L4 are linkers, wherein L2 is present and wherein L2 is not more than 12 amino acids long;
wherein L1 and L3 are each at least 15 amino acids long; and
wherein L4 can be present or absent;
wherein the Bi-Fc binds to a target cell and an immune effector cell and/or mediates cytolysis of a target cell by an immune effector cell, wherein the polypeptide chain targeting the effector cell binds to human and/or cynomolgus CD3$\varepsilon$,
wherein the Fc polypeptide chain of the Bi-Fc comprises an insertion of the amino acid sequence of any of SEQ ID NOs:36-47 between positions 384 and 385, wherein these position numbers are assigned according to the EU numbering scheme. The Fc polypeptide chain is a human IgG Fc polypeptide chain, such as IgG1, IgG2, IgG3, or IgG4 Fc polypeptide chain. The Fc polypeptide chain of (a) or (b) can comprise one or more the following alterations: K392D, K392E, K409D, K409E, Y349T, L351T, L368T, L398T, F405T, Y407T, and Y407R. The Fc polypeptide chain of (a) or (b) can comprise one or more alteration that inhibits FcγR binding, such as one or more of L234A, L235A, and any substitution at N297.

The immune effector cell of any Bi-Fc described herein can be a human T cell and/or a cynomolgus monkey T cell. The effector cell protein of any Bi-Fc described herein can be part of the human and/or cynomolgus monkey TCR-CD3 complex. The effector cell protein of any Bi-Fc described herein can be the human and/or cynomolgus monkey CD3ε chain. The effector cell protein is CD3ε. In such embodiments, one VH region of the Bi-Fc can have a CDR1 having the amino acid sequence of SEQ ID NO:48, a CDR2 having the amino acid sequence of SEQ ID NO:49, and a CDR3 having the amino acid sequence of SEQ ID NO:50, and on VL region of the Bi-Fc can have a CDR1 having the amino acid sequence of SEQ ID NO:51, a CDR2 having the amino acid sequence of SEQ ID NO:52, and a CDR3 having the amino acid sequence of SEQ ID NO:53. In another such embodiment, one VH region of the Bi-Fc can have a CDR1 having the amino acid sequence of SEQ ID NO:54, a CDR2 having the amino acid sequence of SEQ ID NO:55, and a CDR3 having the amino acid sequence of SEQ ID NO:56, and on VL region of the Bi-Fc can have a CDR1 having the amino acid sequence of SEQ ID NO:57, a CDR2 having the amino acid sequence of SEQ ID NO:58, and a CDR3 having the amino acid sequence of SEQ ID NO:59.

The Bi-Fc can comprise a VH region and a VL comprising the amino acid sequences of SEQ ID NOs:7 and 8, respectively, or comprising the amino acid sequences of SEQ ID NOs:29 and 31, respectively. Alternatively such a Bi-Fc can comprise a VH region comprising an amino acid sequence at least 95% identical to SEQ ID NO:7 or SEQ ID NO:29 and a VL region comprising an amino acid sequence at least 95% identical to SEQ ID NO:8 or SEQ ID NO:31, wherein the identity region is at least 50, 60, 70, 80, 90, or 100 amino acids long.

The target cell of any Bi-Fc can be a cancer cell, a cell infected by a pathogen, or a cell that mediates disease. If the target cell is a cancer cell, the cancer can be a hematologic malignancy or a solid tumor malignancy. If the target cell is a cancer cell, the Bi-Fc can bind to a cancer cell antigen such as epidermal growth factor receptor (EGFR), EGFRvIII (a mutant form of EGFR), melanoma-associated chondroitin sulfate proteoglycan (MCSP), mesothelin (MSLN), folate receptor 1 (FOLR1), CD133, CDH19, and human epidermal growth factor 2 (HER2), among many others. If the target cell is a cell infected by a pathogen, the pathogen can be virus, including human immunodeficiency virus, hepatitis virus, human papilloma virus, or cytomegalovirus, or a bacterium of the genus Listeria, Mycobacterium, Staphylococcus, or Streptococcus. If the target cell is a cell that mediates a disease, the target cell can be a cell that mediates a fibrotic disease or an autoimmune or inflammatory disease.

Provided herein are pharmaceutical formulations comprising any of the Bi-Fc molecules described herein and a physiologically acceptable excipient.

Further provided herein are nucleic acids encoding any of the Bi-Fc described herein and vectors containing such nucleic acids, as well as host cell containing such nucleic acids and/or vectors. In another aspect, described herein is a method for making a Bi-Fc comprising culturing the host cell containing the nucleic acids or vector under conditions such that the nucleic acids are expressed, and recovering the Bi-Fc from the cell mass or the culture medium.

In another aspect, provided herein is a pharmaceutical composition for use in a method for treating a cancer patient comprising administering to the patient a therapeutically effective dose of any of the Bi-Fc molecules described herein, wherein the target cell of the Bi-Fc is a cancer cell. This pharmaceutical composition for use in a method for treating a cancer can further comprise administering radiation, a chemotherapeutic agent, or a non-chemotherapeutic, anti-neoplastic agent before, after, or concurrently with the administration of the Bi-Fc. The patient can have a hematologic malignancy or a solid tumor malignancy.

In a further embodiment, described herein is a pharmaceutical composition for use in a method for treating a patient having a fibrotic disease comprising administering to the patient a therapeutically effective dose of any of the Bi-Fc molecules described herein, wherein the target cell of the Bi-Fc is a fibrotic cell . The Bi-Fc can be administered concurrently with, before, or after the administration of other therapeutics used to treat the disease. The fibrotic disease can be atherosclerosis, chronic obstructive pulmonary disease (COPD), cirrhosis, scleroderma, kidney transplant fibrosis, kidney allograft nephropathy, or a pulmonary fibrosis, including idiopathic pulmonary fibrosis.

In still another aspect, described herein is a pharmaceutical composition for use in a method for treating a patient having a disease mediated by a pathogen comprising administering to the patient a therapeutically effective dose of any of the Bi-Fc molecules described herein. The pathogen can be a virus, a bacterium, or a protozoan. The Bi-Fc can be administered concurrently with, before, or after the administration of other therapeutics used to treat the pathogen-mediated disease.

Also provided herein are pharmaceutical compositions comprising any of the Bi-Fc molecules described herein plus a physiologically acceptable excipient. Such compositions can be for use in the treatment of a cancer, an infectious disease, an autoimmune or inflammatory disease, or a fibrotic disease.

Brief Description of the Figures

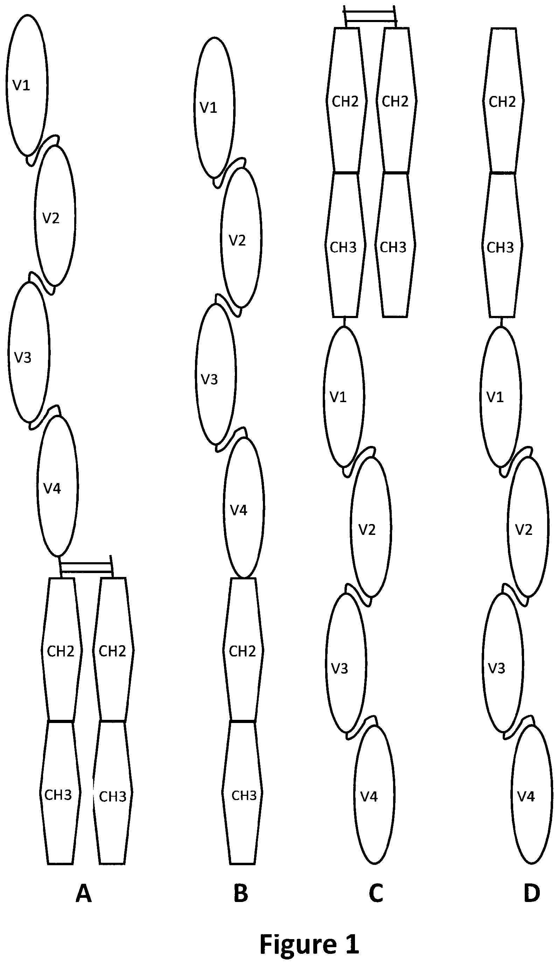

**Figure 1:** Diagrams of exemplary heterodimeric and monomeric Bi-Fc molecules. Four immunoglobulin variable regions are indicated by ovals and labeled V1, V2, V3, and V4. CH2 and CH3 regions are labeled as such and diagramed as elongated hexagons. Lines between these regions indicate linkers or a hinge region. Exemplary disulfide bridges are indicated by horizontal lines. Panels A and C depict heterodimeric Bi-Fc's, and panels B and D depict monomeric Be-Fc's.

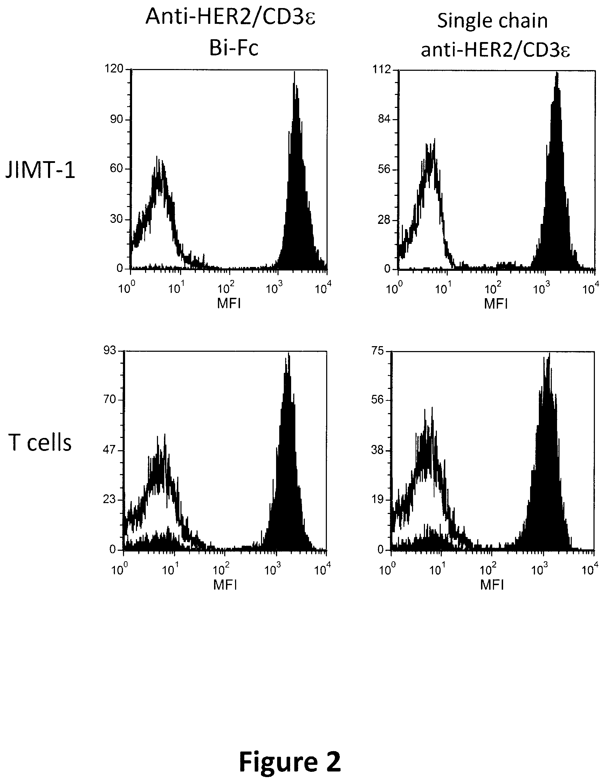

**Figure 2:** Binding of a heterodimeric Bi-Fc to target cells and immune effector cells. Methods are described in Example 2. Mean fluorescence intensity (MFI) is indicated on the x axis, and the number of cells is indicated on the y axis. The unfilled profiles represent data from cells in the absence of one of the bispecific molecules, and the solidly filled profiles represent data from cells in the presence of one of the bispecific molecules. As indicated in the figure, panels at left represent data from samples containing the heterodimeric anti-HER2/CD3ε Bi-Fc, and panels at right represent data from samples containing the single chain anti-HER2/CD3ε. Top two panels represent data from samples containing JIMT-1 cells (which express the target cell protein HER2), and bottom two panels represent data from samples containing T cells (which express the effector cell protein CD3ε).

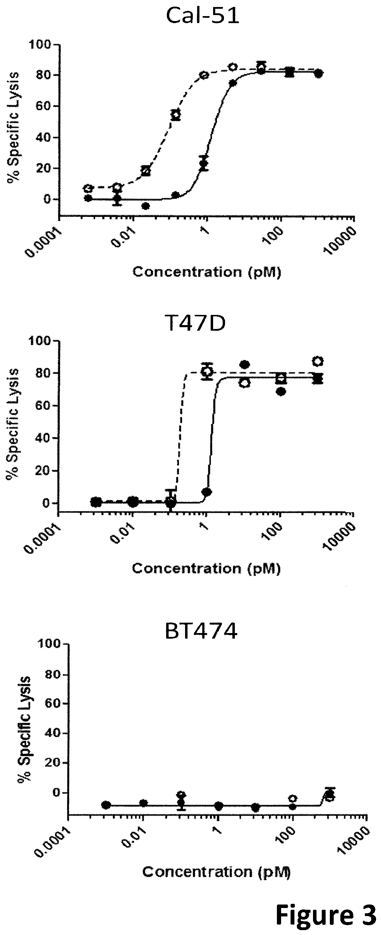

**Figure 3:** Cytolytic activity of a heterodimeric anti-FOLR1/CD3ε Bi-Fc and a single chain anti-FOLR1/CD3ε molecule. Methods are described in Example 3. The x axis in each panel indicates the concentration of the Bi-Fc or single chain molecule (pM) in each sample. The y axis in each panel indicates the percent specific lysis calculated as described in Example 3. Open circles connected by a dashed line indicate data from samples containing the single chain molecule, and filled circles connected by a solid line indicate data from the Bi-Fc molecule. The top, middle, and bottom panels, as indicated, show data from Cal-51 cells (which express FOLR1), T47D cells (which express FOLR1), and BT474 cells (which do not express FOLR1), respectively.

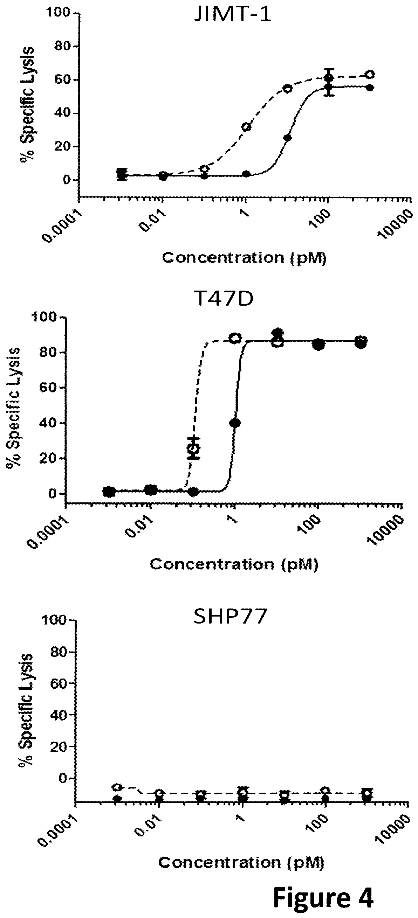

**Figure 4:** Cytolytic activity of a heterodimeric anti-HER2/CD3ε Bi-Fc and a single chain anti-HER2/CD3ε molecule. Methods are described in Example 3. The x axis in each panel indicates the concentration of the Bi-Fc or single chain molecule (pM) in each sample. The y axis in each panel indicates the percent specific lysis calculated as described in Example 3. Open circles connected by a dashed line indicate data from samples containing the single chain molecule, and filled circles connected by a solid line indicate data from the Bi-Fc molecule. The top, middle, and bottom panels, as indicated, show data from JIMT-1 cells (which express HER2), T47D cells (which express HER2), and SHP77 cells (which do not express HER2), respectively.

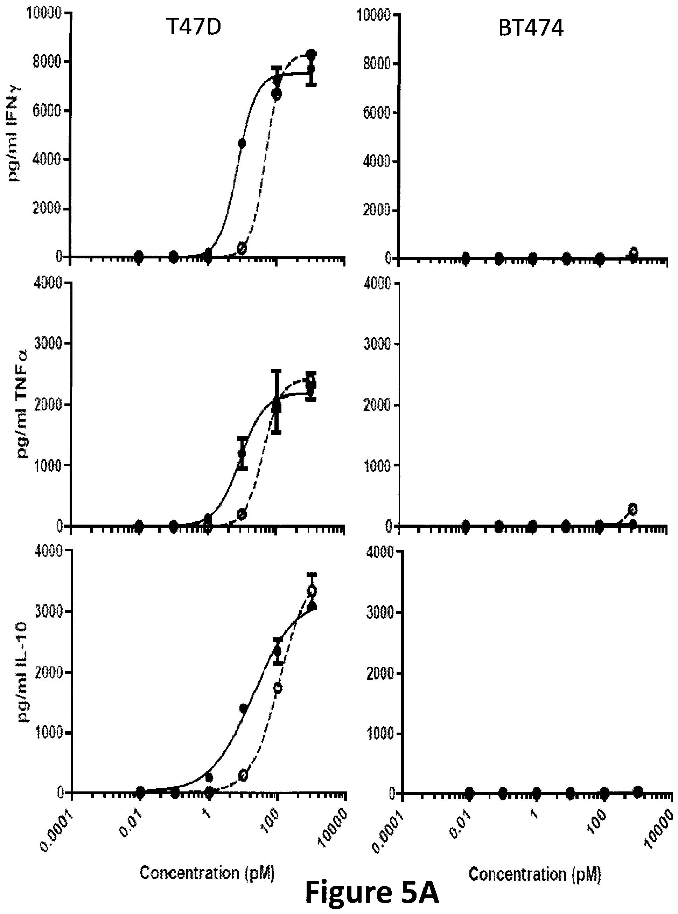

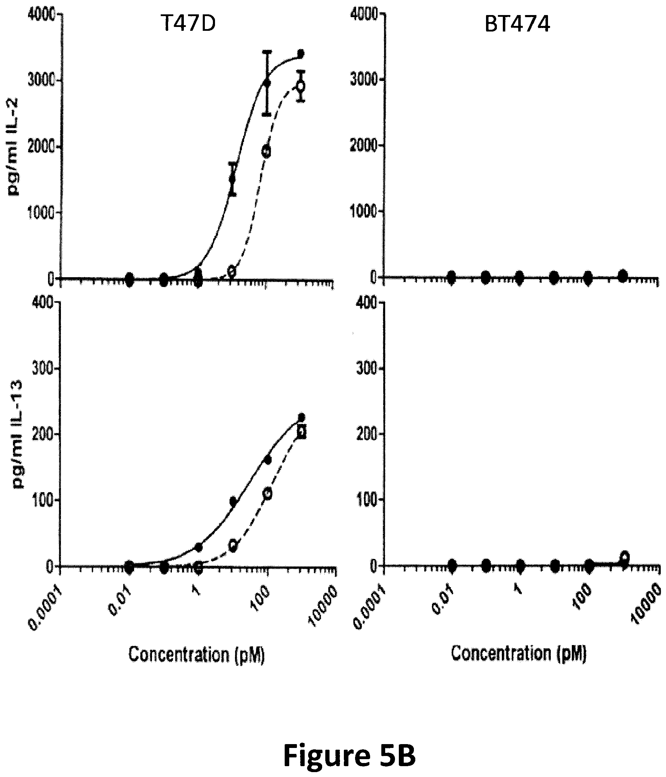

**Figure 5:** Cytokine production by T cells in the presence of a heterodimeric anti-FOLR1/CD3ε Bi-Fc or single chain molecule. Methods are described in Example 4. Open circles connected by dashed lines indicate data from assays containing the heterodimeric anti-FOLR1/CD3ε Bi-Fc, and solidly filled circles connected by solid lines indicate data from the single chain anti-FOLR1/CD3ε molecule. The x axis in each panel indicates the concentration of the Bi-Fc or single chain molecule (pM) in each assay. The y axis indicates the concentration and identity of the cytokine detected (pg/mL). Figure 5A shows data for interferon gamma (IFNγ, top), tumor necrosis factor alpha (TNFα, middle), and interleukin-10 (IL-10, bottom), and Figure 5B shows data for interleukin-2 (IL-2, top) and interleukin-13 (IL-13, bottom), as indicated. As indicated, panels on the left show data from samples containing T47D cells (which express FOLR1), and panels on the right show data from samples containing BT474 cells (which do not express FOLR1).

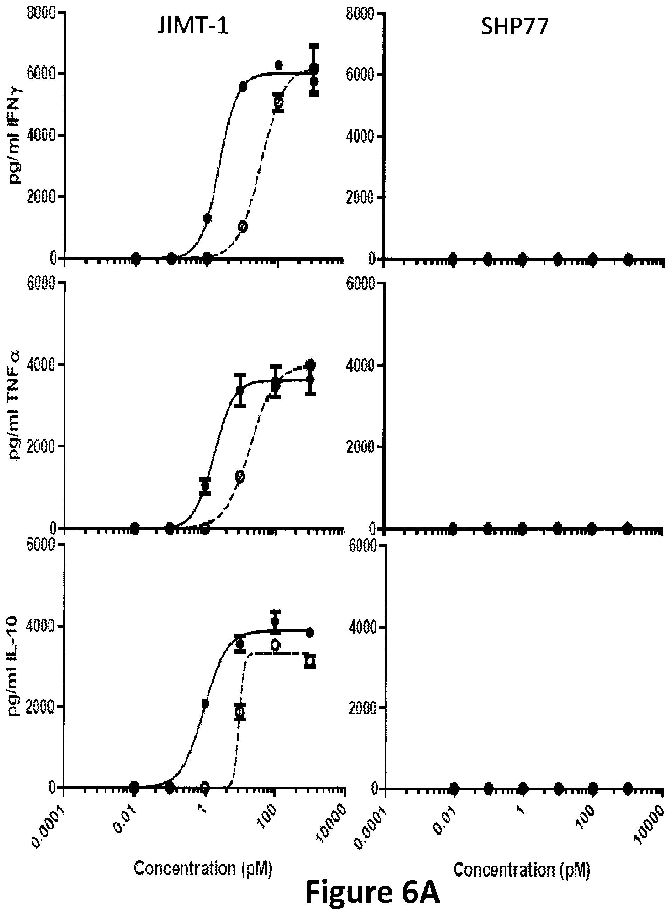

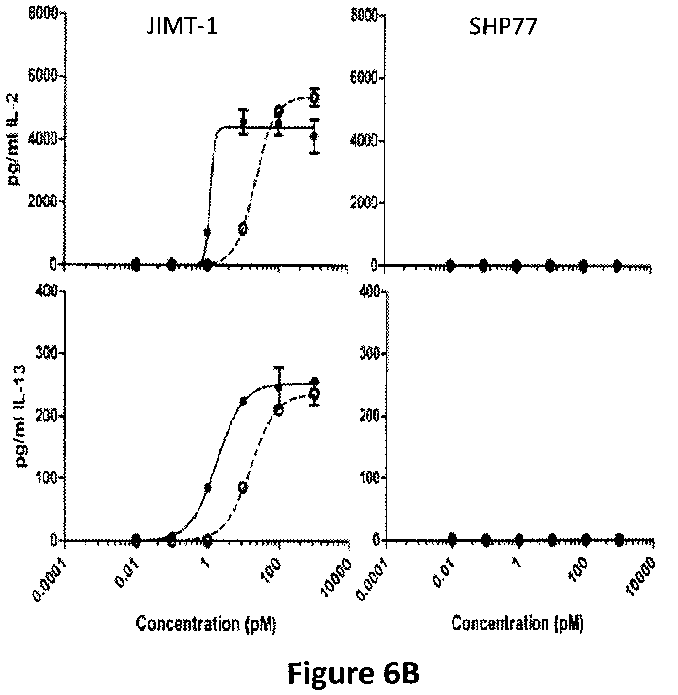

**Figure 6:** Cytokine production by T cells in the presence of an anti-HER2/CD3ε heterodimeric Bi-Fc or single chain molecule. Methods are described in Example 4. Open circles connected by dashed lines indicate data from assays containing the heterodimeric anti-HER2/CD3ε Bi-Fc, and solidly filled circles connected by solid lines indicate data from the single chain anti-HER2/CD3ε molecule. The x axis in each panel indicates the concentration of the Bi-Fc or single chain molecule (pM) in each assay. The y axis indicates the concentration and identity of the cytokine detected (pg/mL). Figure 6A data for IFNγ (top), TNFα (middle), and IL-10 (bottom), and Figure 6B shows data for IL-2 (top) and IL-13 (bottom), as indicated. As indicated, panels on the left show data from samples containing JIMT-1 cells (which express HER2), and panels on the right show data from samples containing SHP77 cells (which do not express HER2).

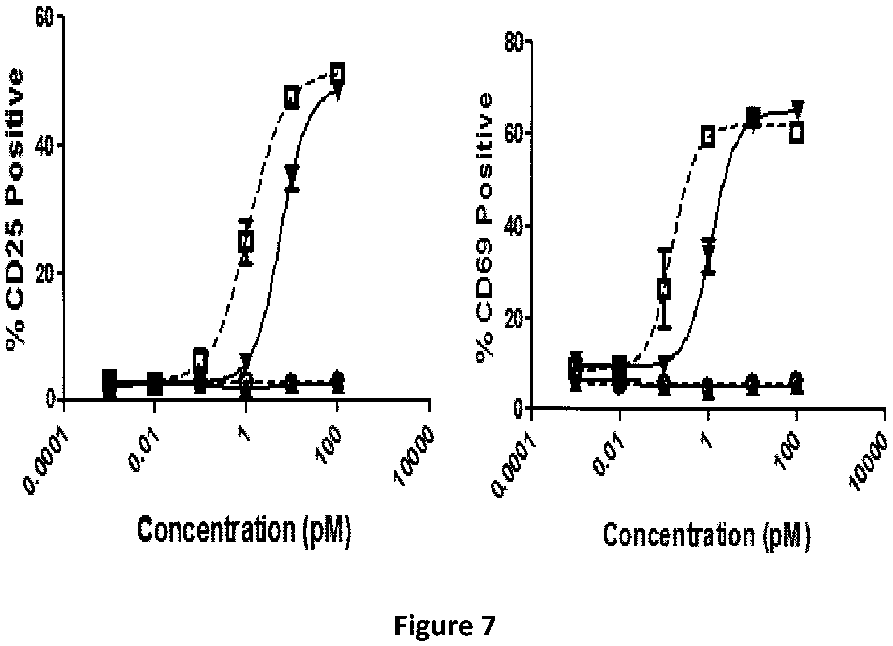

**Figure 7:** Percentage of CD25$^+$ and CD69$^+$ cells in the presence of an anti-HER2/CD3ε heterodimeric Bi-Fc or single chain molecule. Methods are described in Example 5. The x axis indicates the concentration (pM) of the anti-HER2/CD3ε heterodimeric Bi-Fc or single chain molecule. The y axis indicates the percent of CD3$^+$ T cells that are also CD25$^+$ (left panel) or CD69$^+$ (right panel) cells. Symbols indicate as follows: open squares connected by dashed line, the single chain molecule plus JIMT-1 target cells; solidly filled, downward pointing triangles connected by a solid line, the Bi-Fc molecule plus JIMT-1 target cells; open circles connected by a dashed line, the single chain molecule without JIMT-1 target cells; and solidly filled, upward pointing triangles connected by a solid line, the Bi-Fc without JIMT-1 target cells.

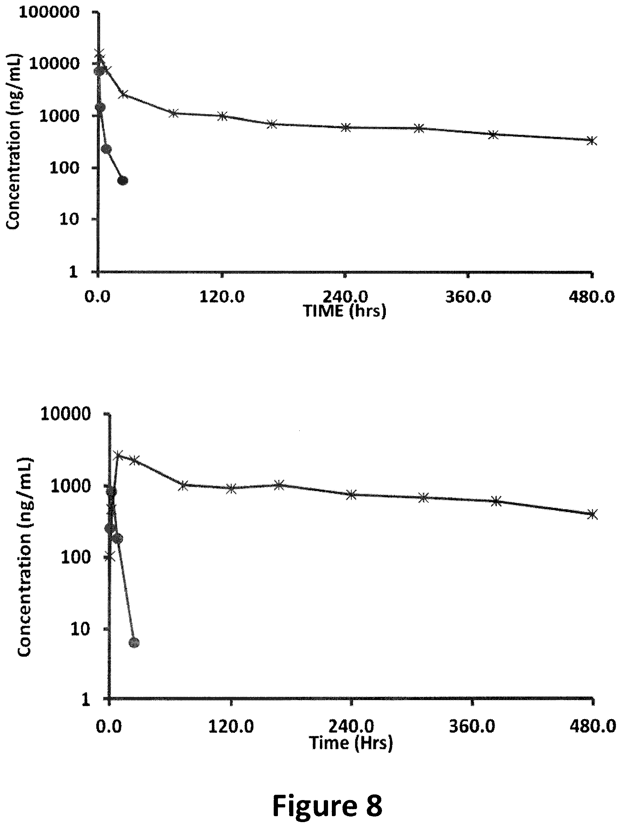

**Figure 8:** Pharmacokinetic properties of a heterodimeric Bi-Fc and a single chain bispecific molecule in mice. Methods are described in Example 6. In the top panel, a pharmacokinetic profile following an intravenous injection is shown, and below is shown the profile following a subcutaneous injection. Solidly filled circles connected by a solid line indicate data from the anti-HER2/CD3ε single chain molecule, and asterisks connected by a solid line indicate data from the heterodimeric anti-HER2 /CD3ε Bi-Fc molecule.

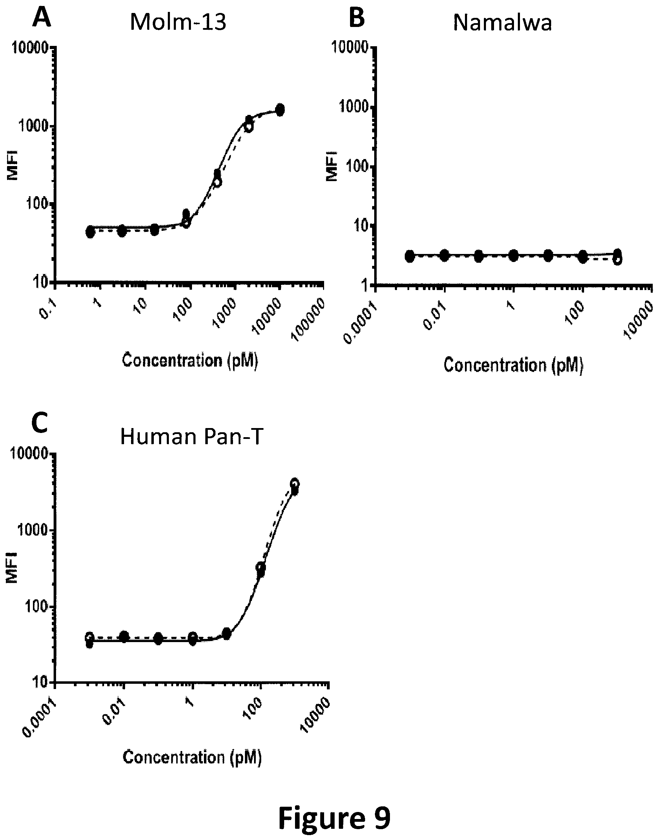

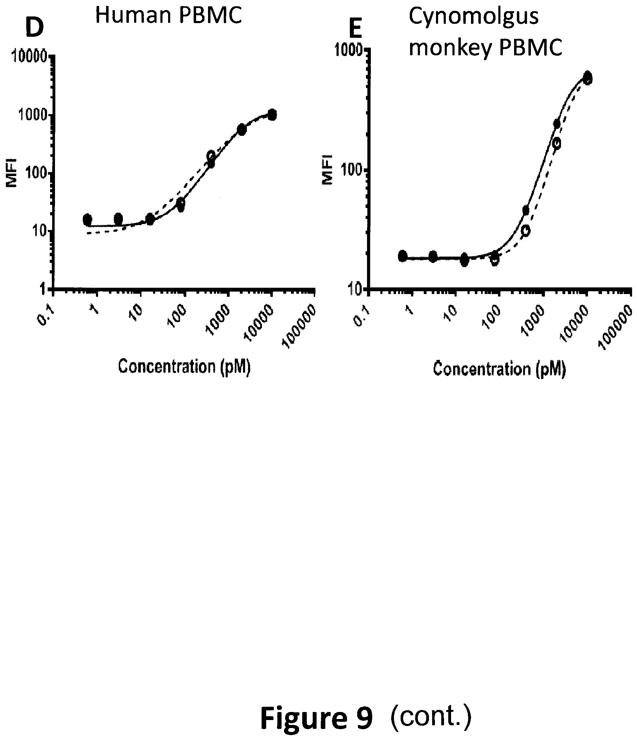

**Figure 9:** Binding of anti-CD33/CD3ε molecules to various cell types. Experimental procedures are described in Example 8. Open circles with dotted lines represent data from cultures containing the single chain anti-CD33/CD3ε, and filled circles with solid lines represent data from cultures containing the monomeric anti-CD33/CD3ε Bi-Fc. As indicated, panels A, B, C, D, and E show data on binding to Molm-13 cells, Namalwa cells, human pan T cells, human peripheral blood mononuclear cells (PBMCs), and cynomolgus monkey PBMCs, respectively.

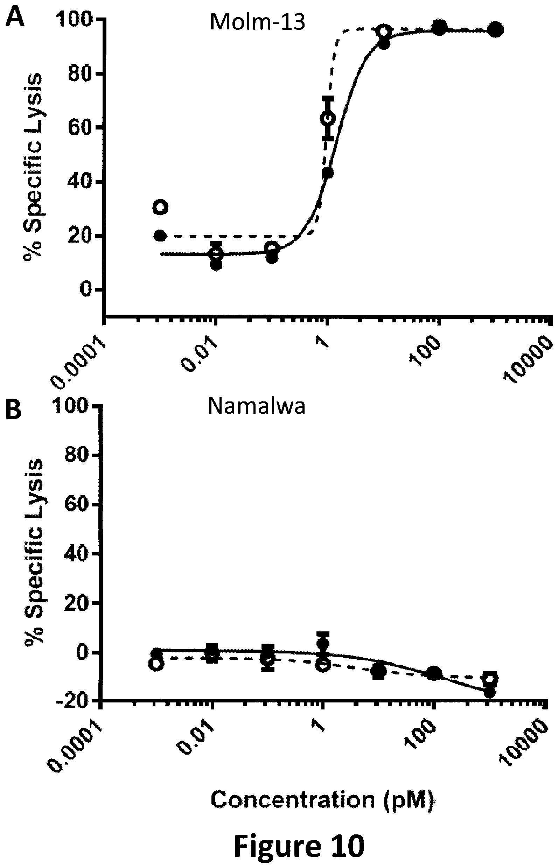

Figure 10: Lysis of Molm-13 cells, but not Namalwa cells, in the presence of PBMCs from cynomolgus monkey and a bispecific anti-CD33/CD3ε molecule. Experimental procedures are described in Example 9. Open circles with dotted lines represent data from cultures containing the single chain anti-CD33/CD3ε, and filled circles with solid lines represent data from cultures containing the monomeric anti-CD33/CD3ε Bi-Fc. Cultures contained PBMCs, a bispecific anti-CD33/CD3ε molecule, and either Molm-13 cells (panel A) or Namalwa cells (panel B).

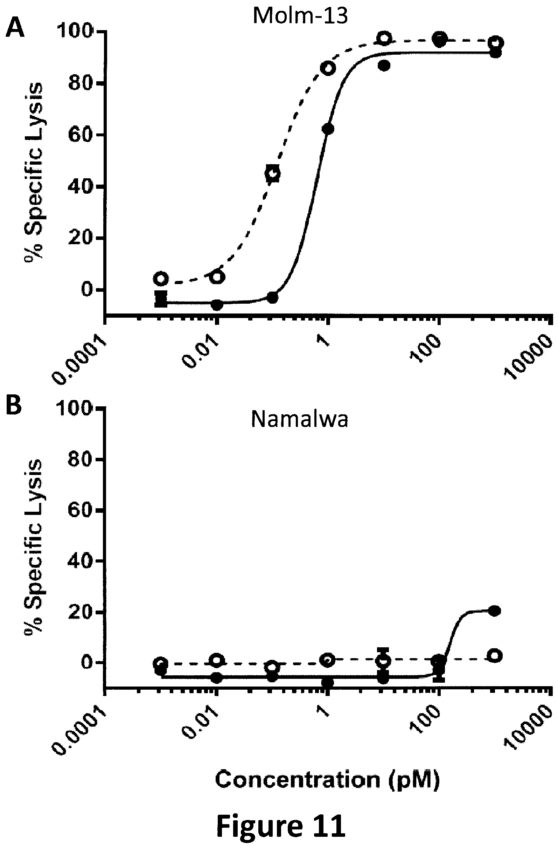

**Figure 11:** Lysis of Molm-13 cells, but not Namalwa cells, in the presence of pan T cells and a bispecific anti-CD33/CD3ε molecule. Experimental procedures are described in Example 9. Open circles with dotted lines represent data from cultures containing the single chain anti-CD33/CD3ε, and filled circles with solid lines represent data from cultures containing the monomeric anti-CD33/CD3ε Bi-Fc. Cultures contained pan T cells, a bispecific anti-CD33/CD3ε molecule, and either Molm-13 cells (panel A) or Namalwa cells (panel B).

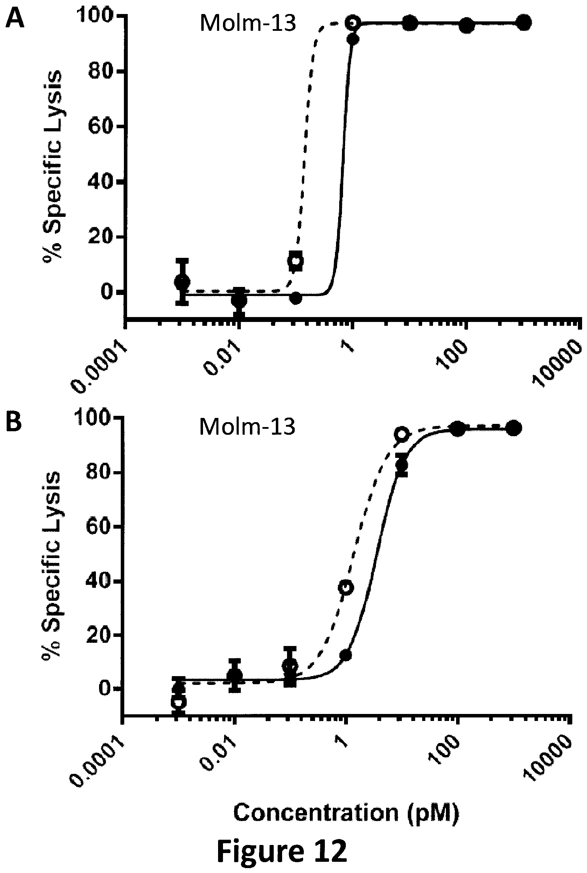

**Figure 12**: Lysis of CD33-expressing tumor cells in the presence of PBMCs and either the monomeric anti-CD33/CD3ε Bi-Fc or the single chain anti-CD33/CD3ε.

Experimental procedures are described in Example 10. The graphs show data from cultures containing an anti-CD33/CD3ε molecule and CD33-expressing Molm-13 cells, plus either human PBMCs (panel A) or cynomulgus monkey PBMCs (panel B). Open circles with dotted lines represent data from cultures containing the single chain anti-CD33/CD3ε, and filled circles with solid lines represent data from cultures containing the monomeric anti-CD33/CD3ε Bi-Fc.

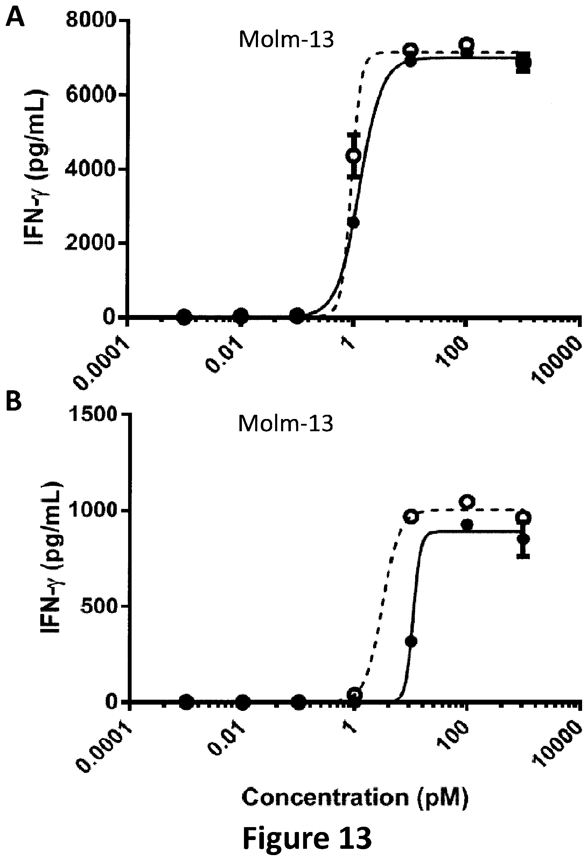

**Figure 13:** Release of interferon gamma (IFN-γ) by PBMCs in the presence of a monomeric anti-CD33/CD3ε Bi-Fc and CD33-expressing tumor cells. Experimental procedures are described in Example 10. The graphs show data from cultures containing an anti-CD33/CD3ε molecule plus CD33-expressing Molm-13 cells plus either human PBMCs (panel A) or cynomulgus monkey PBMCs (panel B). Open circles with dotted lines represent data from cultures containing the single chain anti-CD33/CD3ε, and filled circles with solid lines represent data from cultures containing the monomeric anti-CD33/CD3ε Bi-Fc.

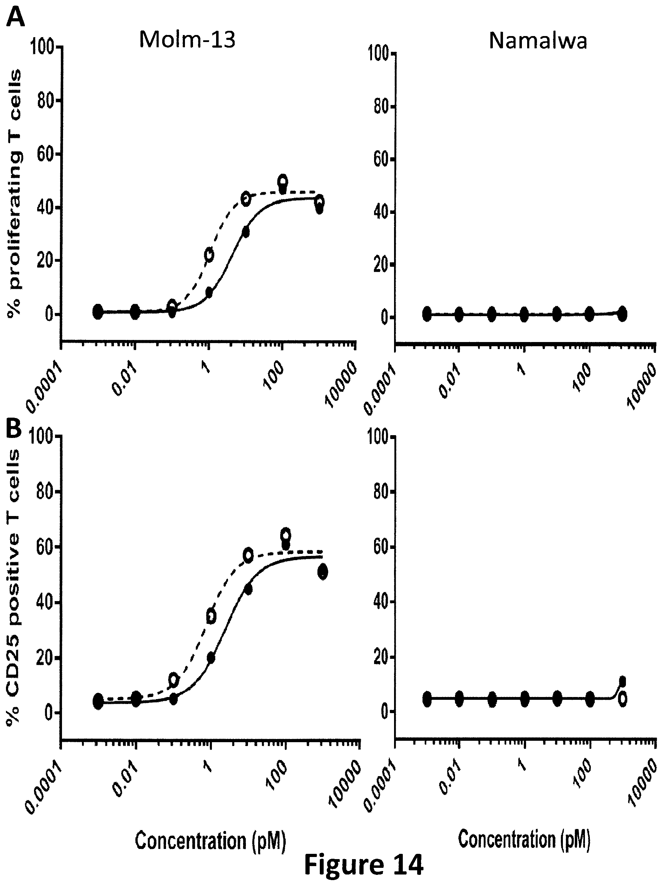

**Figure 14:** Proliferation and CD25 expression by T cells. Experimental procedures are described in Example 11. As indicated, graphs in the left column represent data from cell cultures containing Molm-13 cells (which express CD33) and pan T cells, and graphs in the right column represent data from cell cultures containing Namalwa cells (which do not express CD33) and pan T cells. As indicated, panel A shows the percent of proliferating T cells in the cultures, and panel B shows the percent of CD25 positive T cells in the culture. Open circles with dotted lines represent data from cultures containing the single chain anti-CD33/CD3ε, and filled circles with solid lines represent data from cultures containing the monomeric anti-CD33/CD3ε Bi-Fc.

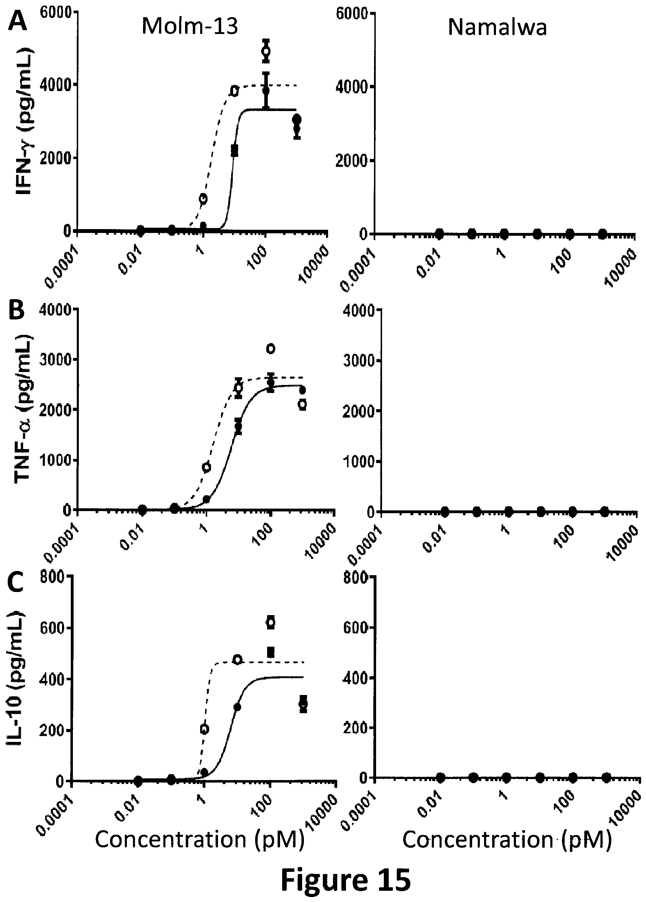

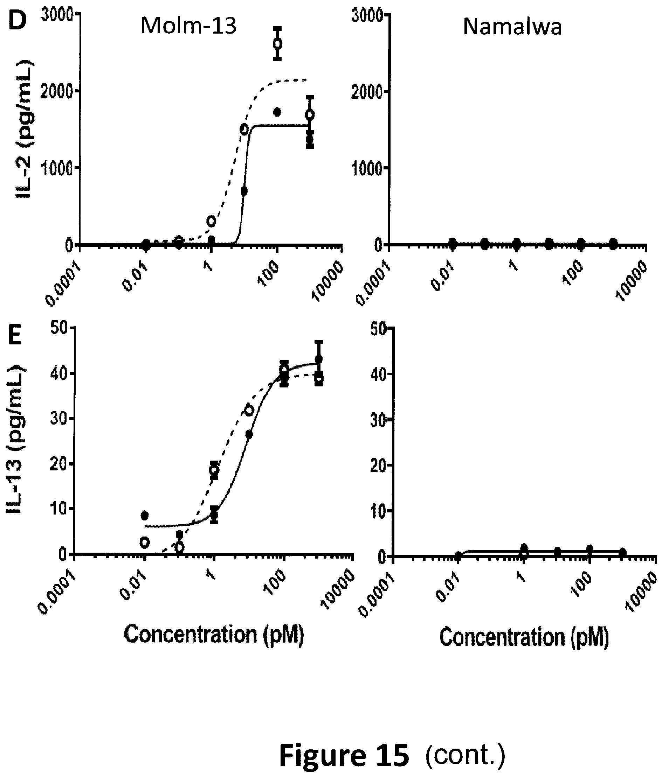

**Figure 15:** Cytokine release by T cells in the presence of a monomeric anti-CD33/CD3ε Bi-Fc and CD33-expressing tumor cells. Experimental procedures are described in Example 11. As indicated, graphs in the left column represent data from cell cultures containing Molm-13 cells (which express CD33) and pan T cells, and graphs in the right column represent data from cell cultures containing Namalwa cells (which do not express CD33) and pan T cells. Open circles with dotted lines represent data from cultures containing the single chain anti-CD33/CD3ε, and filled circles with solid lines represent data from cultures containing the monomeric anti-CD33/CD3ε Bi-Fc. The cytokine assayed is indicated at left of each panel.

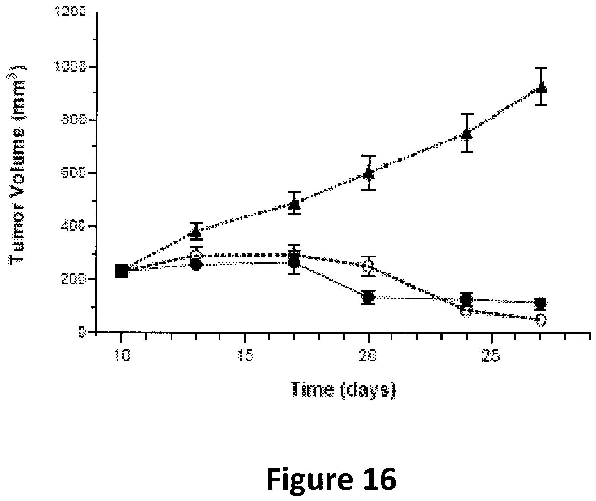

**Figure 16:** *In vivo* inhibition of tumor growth by a heterodimeric anti-FOLR1/CD3ε Bi-Fc . Methods are described in Example 13. The x axis show the time (days) elapsed since three million FOLR1-expressing, NCI-N87-luc tumor cells were implanted into the mice. The y axis shows tumor volume ($mm^3$). Symbols signify the treatment used for each group of mice as follows: vehicle (25 mM lysine-hydrochloride, 0.002% Tween 80 in 0.9% NaCl, pH 7.0), solidly filled triangle; single chain anti-FOLR1/CD3ε bispecific, solidly filled circles; and heterodimeric anti-FOLR1/CD3ε Bi-Fc, open circles.

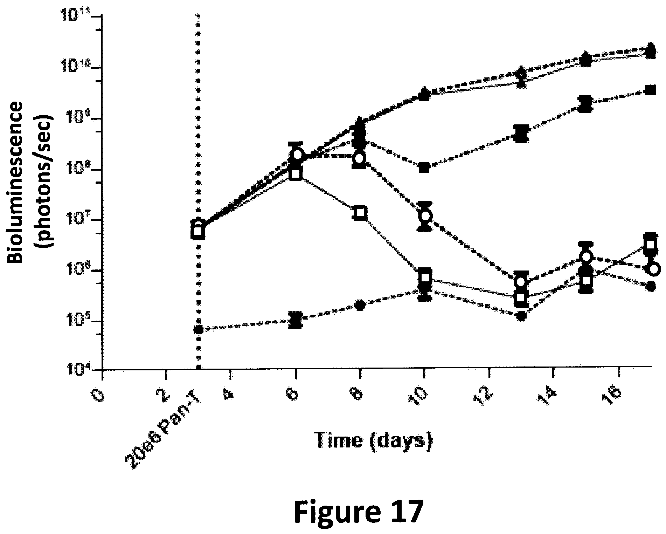

**Figure 17:** *In vivo* inhibition of tumor growth by a heterodimeric anti-CD33/CD3ε Bi-Fc and a monomeric anti-CD33/CD3ε Bi-Fc. Methods are described in Example 14. The x axis shows the time (days) elapsed since one million tumor cells were implanted subcutaneously into the right flank of each mouse. The y axis shows bioluminescence, which reflects the number of tumor cells present. The vertical dotted line indicates the time at which $20 \times 10^6$ human T cells were injected into the mice. Symbols signify the treatment used for each group of mice as follows: vehicle (25 mM lysine-hydrochloride, 0.002% Tween 80 in 0.9% NaCl, pH 7.0), solidly filled triangle; single chain anti-MEC/CD3ε bispecific, open triangle; single chain anti-CD33/CD3ε bispecific, open squares; heterodimeric anti-CD33/CD3ε Bi-Fc, open circles; monomeric anti-CD33/CD3ε Bi-Fc, solidly filled squares; and naïve animals, solidly filled circles.

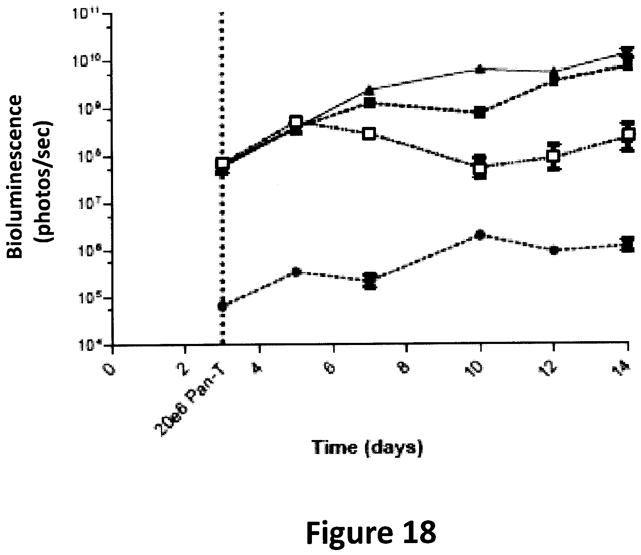

**Figure 18:** *In vivo* inhibition of tumor growth by a monomeric anti-CD33/CD3ε Bi-Fc. Methods are described in Example 15. The x axis shows the time (days) elapsed since one million tumor cells were implanted subcutaneously into the right flank of each mouse. The y axis shows tumor bioluminescence. The vertical dotted line indicates the time at which $20 \times 10^6$ human T cells were injected into the mice. Symbols signify the treatment used for each group of mice as follows: vehicle (25 mM lysine-hydrochloride, 0.002% Tween 80 in 0.9% NaCl, pH 7.0), solidly filled triangle; a monomeric anti-CD33/CD3ε Bi-Fc (N297G), solidly filled square; a monomeric anti-CD33/CD3ε Bi-Fc (N297 wild type), open squares; and naive animals, filled circles.

## Brief Description of the Sequences

| SEQ ID NO | Description |
| --- | --- |
| SEQ ID NO:1 | Amino acid sequence preceding VH CDR1 |
| SEQ ID NO:2 | Amino acid sequence preceding VH CDR2 |
| SEQ ID NO:3 | Amino acid sequence following VH CDR3 |
| SEQ ID NO:4 | Amino acid sequence following light chain CDR3 |
| SEQ ID NO:5 | Amino acid sequence of anti-HER2 VH region |
| SEQ ID NO:6 | Amino acid sequence of anit-HER2 VL region |
| SEQ ID NO:7 | Amino acid sequence of anti CD3ε VH region |
| SEQ ID NO:8 | Amino acid sequence of anti-CD3ε VL region |
| SEQ ID NO:9 | Amino acid sequence of a single chain anti-HER2/CD3ε (P136629.3) |
| SEQ ID NO:10 | Amino acid sequence of a first polypeptide chain of a heterodimeric anti-HER2/CD3ε of a Bi-Fc |
| SEQ ID NO:11 | Nucleic acid sequence encoding SEQ ID NO:10 |
| SEQ ID NO:12 | Amino acid sequence of a human IgG1 Fc polypeptide containing alterations D356K and D399K |
| SEQ ID NO:13 | Nucleic acid sequence encoding SEQ ID NO:12 |
| SEQ ID NO:14 | Amino acid sequence of a single chain anti-FOLR1/CD3ε molecule |
| SEQ ID NO:15 | Amino acid sequence of a first polypeptide chain of a heterodimeric anti-FOLR1/CD3ε molecule |

(continued)

| SEQ ID NO | Description |
|---|---|
| SEQ ID NO:16 | Nucleic acid sequence encoding SEQ ID NO:15 |
| SEQ ID NO:17 | Amino acid sequence of a linker |
| SEQ ID NO:18 | Amino acid sequence of a linker |
| SEQ ID NO:19 | Amino acid sequence of a linker |
| SEQ ID NO:20 | Amino acid sequence of a linker |
| SEQ ID NO:21 | Amino acid sequence of a linker |
| SEQ ID NO:22 | Mature amino acid sequence of CD3 epsilon chain of *Homo sapiens* |
| SEQ ID NO:23 | Mature amino acid sequence of CD3 epsilon chain of *Macaca fascicularis* |
| SEQ ID NO:24 | A portion of an epitope that is part of CD3 epsilon |
| SEQ ID NO:25 | Amino acid sequence of human IgG1 Fc region |
| SEQ ID NO:26 | Amino acid sequence of human IgG2 Fc region |
| SEQ ID NO:27 | Amino acid sequence of human IgG3 Fc region |
| SEQ ID NO:28 | Amino acid sequence of human IgG4 Fc region |
| SEQ ID NO:29 | Amino acid sequence of an anti-CD3$\epsilon$ VH region |
| SEQ ID NO:30 | Nucleic acid sequence encoding SEQ ID NO:29 |
| SEQ ID NO:31 | Amino acid sequence of an anti-CD3$\epsilon$ VL region |
| SEQ ID NO:32 | Nucleic acid sequence encoding SEQ ID NO:31 |
| SEQ ID NO:33 | Amino acid sequence of an anti-CD33/CD3$\epsilon$ single chain molecule |
| SEQ ID NO:34 | Amino acid sequence of monomeric anti-CD33/CD3$\epsilon$ Bi-Fc |
| SEQ ID NO:35 | Nucleic acid sequence encoding SEQ ID NO:34 |
| SEQ ID NO:36 | Amino acid sequence of an insertion that prolongs half life |
| SEQ ID NO:37 | Amino acid sequence of an insertion that prolongs half life |

(continued)

| SEQ ID NO | Description |
|---|---|
| SEQ ID NO:38 | Amino acid sequence of an insertion that prolongs half life |
| SEQ ID NO:39 | Amino acid sequence of an insertion that prolongs half life |
| SEQ ID NO:40 | Amino acid sequence of an insertion that prolongs half life |
| SEQ ID NO:41 | Amino acid sequence of an insertion that prolongs half life |
| SEQ ID NO:42 | Amino acid sequence of an insertion that prolongs half life |
| SEQ ID NO:43 | Amino acid sequence of an insertion that prolongs half life |
| SEQ ID NO:44 | Amino acid sequence of an insertion that prolongs half life |
| SEQ ID NO:45 | Amino acid sequence of an insertion that prolongs half life |
| SEQ ID NO:46 | Amino acid sequence of an insertion that prolongs half life |
| SEQ ID NO:47 | Amino acid sequence of an insertion that prolongs half life |
| SEQ ID NO:48 | Amino acid sequence of a VH region CDR1 of SEQ ID NO:7 |
| SEQ ID NO:49 | Amino acid sequence of a VH region CDR2 of SEQ ID NO:7 |
| SEQ ID NO:50 | Amino acid sequence of a VH region CDR3 of SEQ ID NO:7 |
| SEQ ID NO:51 | Amino acid sequence of a VL region CDR1 of SEQ ID NO:8 |
| SEQ ID NO:52 | Amino acid sequence of a VL region CDR2 of SEQ ID NO:8 |
| SEQ ID NO:53 | Amino acid sequence of a VL region CDR3 of SEQ ID NO:8 |
| SEQ ID NO:54 | Amino acid sequence of a VH region CDR1 of SEQ ID NO:29 |
| SEQ ID NO:55 | Amino acid sequence of a VH region CDR2 of SEQ ID NO:29 |
| SEQ ID NO:56 | Amino acid sequence of a VH region CDR3 of SEQ ID NO:29 |
| SEQ ID NO:57 | Amino acid sequence of a VL region CDR1 of SEQ ID NO:31 |
| SEQ ID NO:58 | Amino acid sequence of a VL region CDR2 of SEQ ID NO:31 |
| SEQ ID NO:59 | Amino acid sequence of a VL region CDR3 of SEQ ID NO:31 |

(continued)

| SEQ ID NO | Description |
|---|---|
| SEQ ID NO:60 | Amino acid sequence of a VH region CDR1 of SEQ ID NO:5 |
| SEQ ID NO:61 | Amino acid sequence of a VH region CDR2 of SEQ ID NO:5 |
| SEQ ID NO:62 | Amino acid sequence of a VH region CDR3 of SEQ ID NO:5 |
| SEQ ID NO:63 | Amino acid sequence of a VL region CDR1 of SEQ ID NO:6 |
| SEQ ID NO:64 | Amino acid sequence of a VL region CDR2 of SEQ ID NO:6 |
| SEQ ID NO:65 | Amino acid sequence of a VL region CDR3 of SEQ ID NO:6 |
| SEQ ID NO:66 | Amino acid sequence of a VH region CDR1 of SEQ ID NO:15 |
| SEQ ID NO:67 | Amino acid sequence of a VH region CDR2 of SEQ ID NO:15 |
| SEQ ID NO:68 | Amino acid sequence of a VH region CDR3 of SEQ ID NO:15 |
| SEQ ID NO:69 | Amino acid sequence of a VL region CDR1 of SEQ ID NO:15 |
| SEQ ID NO:70 | Amino acid sequence of a VL region CDR2 of SEQ ID NO:15 |
| SEQ ID NO:71 | Amino acid sequence of a VL region CDR3 of SEQ ID NO:15 |
| SEQ ID NO:72 | Amino acid sequence of a VH region CDR1 of SEQ ID NO:34 |
| SEQ ID NO:73 | Amino acid sequence of a VH region CDR2 of SEQ ID NO:34 |
| SEQ ID NO:74 | Amino acid sequence of a VH region CDR3 of SEQ ID NO:34 |
| SEQ ID NO:75 | Amino acid sequence of a VL region CDR1 of SEQ ID NO:34 |
| SEQ ID NO:76 | Amino acid sequence of a VL region CDR2 of SEQ ID NO:34 |
| SEQ ID NO:77 | Amino acid sequence of a VL region CDR3 of SEQ ID NO:34 |
| SEQ ID NO:78 | Amino acid sequence of an anti-Mec/CD3ε single chain molecule |
| SEQ ID NO:79 | Nucleic acid sequence encoding SEQ ID NO:78 |
| SEQ ID NO:80 | Amino acid sequence of the first polypeptide chain of a heterodimeric anti-CD33/CD3ε Bi-Fc |
| SEQ ID NO:81 | Nucleic acid sequence encoding SEQ ID NO:80 |

(continued)

| SEQ ID NO | Description |
|---|---|
| SEQ ID NO:82 | Amino acid sequence of the second polypeptide chain of a heterodimeric anti-CD33/CD3ε Bi-Fc, which comprises an Fc polypeptide chain |
| SEQ ID NO:83 | Nucleic acid sequence encoding SEQ ID NO:82 |
| SEQ ID NO:84 | Amino acid sequence of a monomeric anti-CD33/CD3ε Bi-Fc |
| SEQ ID NO:85 | Nucleic acids sequence encoding SEQ ID NO:84 |
| SEQ ID NO:86 | Amino acid sequence of a first polypeptide chain of a heterodimeric anti-FOLR1/CD3ε Bi-Fc molecule |
| SEQ ID NO:87 | Nucleic acid sequence encoding SEQ ID NO:86 |
| SEQ ID NO:88 | Amino acid sequence of the second polypeptide chain of the heterodimeric anti-FOLR1/CD3ε Bi-Fc molecule where SEQ ID NO:86 is the amino acid sequence of the first polypeptide chain |
| SEQ ID NO:89 | Nucleic acid sequence encoding SEQ ID NO:88 |
| SEQ ID NO:90 | Amino acid sequence of a single chain anti-FOLR1/CD3ε bispecific |
| SEQ ID NO:91 | Nucleic acid sequence encoding SEQ ID NO:90 |

## Detailed Description

Described is a new form of bispecific antibody, called herein a bispecific Fc (Bi-Fc) polypeptide, which contains one polypeptide chain or two different polypeptide chains. One chain comprises two heavy chain variable (VH) regions, two light chain variable (VL) regions, and a human IgG Fc polypeptide chain, and an optional second polypeptide chain comprises a human IgG Fc polypeptide chain. One of the proteins to which the Bi-Fc binds is expressed on the surface of an immune effector cell, such as a T cell, an NK cell, a macrophage, or a neutrophil, and the other protein to which the Bi-Fc binds is expressed on the surface of a target cell, for example a cancer cell, a cell infected by a pathogen, or a cell that mediates a disease, such as, for example, a fibrotic disease. Since a Bi-Fc has only one binding site for each of these proteins (*i.e.,* it binds each protein "monovalently," as meant herein), its binding, by itself, will not oligomerize the proteins it binds to on a cell surface. For example, if it binds to CD3 on the surface of a T cell, CD3 will not be oligomerized on the T cell surface in the absence of a target cell. Oligomerization of CD3 can cause a generalized activation of a T cell or downmodulation of CD3 on the T cell, which can be undesirable. The Bi-Fc tethers an immune effector cell to a target cell, thereby eliciting specific cytolytic activity against the target cell, rather than a generalized inflammatory response. Further, the Bi-Fc molecules have favorable pharmacokinetic properties and are not unduly complex to manufacture since they contain only one or only two different polypeptide chains.

### *Definitions*

An "**antibody**," as meant herein, is a protein containing at least one VH or VL region, in many cases both a heavy and a light chain variable region. Thus, the term "antibody" encompasses molecules having a variety of formats, including single chain Fv antibodies (scFv, which contain VH and VL regions joined by a linker), Fab, F(ab)₂', Fab', scFv:Fc antibodies (as described in Carayannopoulos and Capra, Ch. 9 in FUNDAMENTAL IMMUNOLOGY, 3rd ed., Paul, ed., Raven Press, New York, 1993, pp. 284-286) or full length antibodies containing two full length heavy and two full length light chains, such as naturally-occurring IgG antibodies found in mammals. *Id*. Such IgG antibodies can be of the IgG1, IgG2, IgG3, or IgG4 isotype and can be human or humanized antibodies. Further, the term "antibody" includes dimeric antibodies containing two heavy chains and no light chains such as the naturally-occurring antibodies found in camels and other dromedary species and sharks. *See, e.g.,* Muldermans et al, 2001, J. Biotechnol. 74:277-302; Desmyter et al, 2001, J. Biol. Chem. 276:26285-90; Streltsov et al. (2005), Protein Science 14: 2901-2909. An antibody can be "monospecific" (that is, binding to only one kind of antigen), "bispecific" (that is, binding to two different antigens), or "multispecific" (that is, binding to more than one different antigen). Further, an antibody can be monovalent, bivalent, or multivalent, meaning that it can bind to one, two, or multiple antigen molecules at once, respectively. An antibody binds "monovalently" to a particular protein when one molecule of the antibody binds to only one molecule of the protein, even though the antibody may also bind to a different protein as well. That is, an antibody binds "monovalently," as meant herein, to two different proteins when it binds to only one molecule of each protein. Such an antibody is "bispecific" and binds to each of two different proteins "monovalently." An antibody can be "monomeric," *i.e.*, comprising a single polypeptide chain. An antibody can comprise multiple polypeptide chains ("multimeric") or can comprise two ("dimeric"), three ("trimeric"), or four ("tetrameric") polypeptide chains. If multimeric, an antibody can be a homomulitmer, *i.e.,* containing more than one molecule of only one kind of polypeptide chain, including homodimers, homotrimer, or homotetramers. Alternatively, a multimeric antibody can be a heteromultimer, *i.e.,* containing more than one different kind of polypeptide chain, including heterodimers, heterotrimers, or heterotetramers. An antibody can have a variety of possible formats including, for example, monospecific monovalent antibodies (as described in International Application WO 2009/089004 and US Publication 2007/0105199) that may inhibit or activate the molecule to which they bind, bivalent monospecific or bispecific dimeric Fv-Fc, scFv-Fc, or diabody Fc, monospecific monovalent scFv-Fc/Fc's, the multispecific binding proteins and dual variable domain immunoglobulins described in US Publication 2009/0311253, the heterodimeric bispecific antibodies described herein, and the many formats for bispecific antibodies described in Chapters 1, 4, 5, 6, 7, 8, 9, 10, 11, 12, 13, 14 of BISPECIFIC ANTIBODIES, Kontermann, ed., Springer, 2011, among many other possible antibody formats.

A "**Bi-Fc**," as meant herein, comprises a first polypeptide chain and, optionally, a second polypeptide chain. In many embodiments, a Bi-Fc comprises both a first and a second polypeptide chain. In some embodiments, a Bi-Fc is a monomer comprising only the first polypeptide chain. The first polypeptide chain comprises two VH regions and two VL regions that are separated by linkers and a human IgG Fc polypeptide chain. The Fc polypeptide chain can be N-terminal or C-terminal relative to the four immunoglobulin variable regions, and it can be joined to the variable regions via a linker. This linker can be present or absent. The second polypeptide chain, if present, comprises an Fc polypeptide chain. Thus, a Bi-Fc can be a monomer or a heterodimer. A Bi-Fc can bind to an immune effector cell via an effector cell protein and to a target cell via a target cell protein and can mediate cytolysis of a target cell by an immune effector cell.

Monomeric Fc polypeptides are described in detail in United States Patent Application Publication 2012/244578. A monomeric Bi-Fc can comprise an altered Fc polypeptide chain that is more stable as a monomer than a naturally-occurring Fc polypeptide chain. Briefly, such monomers can comprise an altered human IgG Fc polypeptide comprising the following alterations: (1) K409D, K409E,; (2) K392D, K392E,; and (3) F405T or Y349T. In alternate embodiments, positions 409 and 392 are not altered, and other alterations are present, which can include one or more or the alterations described in the definition of "heterodimerizing alterations" below. Such "heterodimerizing alterations" are described below and in US Patent 8,592,562. Alterations of amino acids within an Fc polypeptide chain are denoted as follows. The amino acid normally present a given position is named in one letter code, followed the position numbered according to EU numbering (as shown in Table 2 below), followed by the amino acid replacing the amino acid normally present at that position. For example, the designation "N297G" means that the asparagine normally present at position 297 has been changed to glycine. Further, the Fc polypeptide chain portion of a monomeric Bi-Fc may lack a hinge region (as defined in connection with Table 2 below) or may have deleted or altered cysteine residues in its hinge region.

A "**cancer cell antigen**," as meant herein, is a protein expressed on the surface of a cancer cell. Some cancer cell antigens are also expressed on some normal cells, and some are specific to cancer cells. Cancer cell antigens can be highly expressed on the surface of a cancer cell. There are a wide variety of cancer cell antigens. Examples of cancer cell antigens include, without limitation, the following human proteins: epidermal growth factor receptor (EGFR), EGFRvIII (a mutant form of EGFR), melanoma-associated chondroitin sulfate proteoglycan (MCSP), mesothelin (MSLN), folate receptor 1 (FOLR1), CD33, CDH19, and human epidermal growth factor 2 (HER2), among many others.

"**Chemotherapy**," as used herein, means the treatment of a cancer patient with a "**chemotherapeutic agent**" that has cytotoxic or cytostatic effects on cancer cells. A "**chemotherapeutic agent**" specifically targets cells engaged in cell division and not cells that are not engaged in cell division. Chemotherapeutic agents directly interfere with processes that are intimately tied to cell division such as, for example, DNA replication, RNA synthesis, protein synthesis, the assembly, disassembly, or function of the mitotic spindle, and/or the synthesis or stability of molecules that play a role in these processes, such as nucleotides or amino acids. A chemotherapeutic agent therefore has cytotoxic or cytostatic effects on both cancer cells and other cells that are engaged in cell division. Chemotherapeutic agents are well-known in the art and include, for example: alkylating agents (*e.g.* busulfan, temozolomide, cyclophosphamide, lomustine (CCNU), methyllomustine, streptozotocin, *cis*-diamminedi-chloroplatinum, aziridinylbenzo-quinone, and thiotepa); inorganic ions (*e.g.* cisplatin and carboplatin); nitrogen mustards (*e.g.* melphalan hydrochloride, ifosfamide, chlorambucil, and mechlorethamine HCl); nitrosoureas (*e.g.* carmustine (BCNU)); anti-neoplastic antibiotics (*e.g.* adriamycin (doxorubicin), daunomycin, mitomycin C, daunorubicin, idarubicin, mithramycin, and bleomycin); plant derivatives (*e.g.* vincristine, vinblastine, vinorelbine, paclitaxel, docetaxel, vindesine, VP-16, and VM-26); antimetabolites (*e.g.* methotrexate with or without leucovorin, 5-fluorouracil with or without leucovorin, 5-fluorodeoxyuridine, 6-mercaptopurine, 6-thioguanine, cytarabine, 5-azacytidine, hydroxyurea, deoxycoformycin, gemcitabine, and fludarabine); podophyllotoxins (*e.g.* etoposide, irinotecan, and topotecan); as well as actinomycin D, dacarbazine (DTIC), mAMSA, procarbazine, hexamethylmelamine, pentamethylmelamine, L-asparaginase, and mitoxantrone, among many known in the art. *See e.g.* Cancer: Principles and Practice of Oncology, 4th Edition, DeVita et al., eds., J.B. Lippincott Co., Philadelphia, PA (1993). Alkylating agents and nitrogen mustard act by alkylating DNA, which restricts uncoiling and replication of strands. Methotrexate, cytarabine, 6-mercaptopurine, 5-fluorouracil, and gemcitabine interfere with nucleotide synthesis. Plant derivatives such a paclitaxel and vinblastine are mitotic spindle poisons. The podophyllotoxins inhibit topoisomerases, thus interfering with DNA replication. Antibiotics doxorubicin, bleomycin, and mitomycin interfere with DNA synthesis by intercalating between the bases of DNA (inhibiting uncoiling), causing strand breakage, and alkylating DNA, respectively. Other mechanisms of action include carbamoylation of amino acids (lomustine, carmustine), and depletion of asparagine pools (asparaginase). Merck Manual of Diagnosis and Therapy, 17th Edition, Section 11, Hematology and Oncology, 144. Principles of Cancer Therapy, Table 144-2 (1999). Specifically included among chemotherapeutic agents are those that directly affect the same cellular processes that are directly affected by the chemotherapeutic agents listed above.

A drug is "**concurrently**" administered with a Bi-Fc if it is administered in the same general time frame as the Bi-Fc, optionally, on an ongoing basis. For example, if a patient is taking Drug A once a week on an ongoing basis and a Bi-Fc once every six months on an ongoing basis, Drug A and the Bi-Fc are concurrently administered, whether or not they are ever administered on the same day. Similarly, if the Bi-Fc is taken once per week on an ongoing basis and Drug A is administered only once or a few times on a daily basis, Drug A and the Bi-Fc are concurrently administered as meant herein. Similarly, if both Drug A and the Bi-Fc are administered for short periods of time either once or multiple times within a one month period, they are administered concurrently as meant herein as long as both drugs are administered within the same month.

A "**conservative amino acid substitution**," as meant herein, is a substitution of an amino acid with another amino acid with similar properties. Properties considered include chemical properties such as charge and hydrophobicity. Table 1 below lists substitutions for each amino acid that are considered to be conservative substitutions as meant herein.

**Table 1: Conservative Amino Acid Substitutions**

| Original Residue | Conservative Substitutions |
|---|---|
| Ala | Val, Leu, Ile |
| Arg | Lys, Gln, Asn |

| Original Residue | Conservative Substitutions |
|---|---|
| Asn | Gln |
| Asp | Glu |
| Cys | Ser, Ala |
| Gln | Asn |
| Glu | Asp |
| Gly | Pro, Ala |
| His | Asn, Gln, Lys, Arg |
| Ile | Leu, Val, Met, Ala, Phe, Norleucine |
| Leu | Norleucine, Ile, Val, Met, Ala, Phe |
| Lys | Arg, Gln, Asn |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |

(continued)

| Original Residue | Conservative Substitutions |
|---|---|
| Val | Ile, Met, Leu, Phe, Ala, Norleucine |

As meant herein, an "**Fc region**" is a dimer consisting of two polypeptide chains joined by one or more disulfide bonds, each chain comprising part or all of a hinge domain plus a CH2 and a CH3 domain. Each of the polypeptide chains is referred to as an "**Fc polypeptide chain**." In some embodiments an "Fc polypeptide chain" may lack a hinge region, especially where the Fc polypeptide chain is intended to be monomeric as in a monomeric Bi-Fc. To distinguish the two Fc polypeptide chains in an Fc region, in some instances one is referred to herein as an "**A chain**" and the other is referred to as a "**B chain**." More specifically, the Fc regions (or Fc polypeptide chain in monomeric Bi-Fc's) contemplated for use with the present invention are IgG Fc regions (or Fc polypeptide chains), which can be human IgG1, IgG2, IgG3, or IgG4 Fc regions. Among human IgG1 Fc regions, at least two allelic types are known. In other embodiments, the amino acid sequences of the two Fc polypeptide chains can vary from those of a mammalian Fc polypeptide by no more than 10 amino acid substitutions, insertions, and/or deletions of a single amino acid per 100 amino acids relative to the sequence of a mammalian Fc polypeptide amino acid sequence. In some embodiments, such variations can be "heterodimerizing alterations" that facilitate the formation of heterodimers over homodimers and/or inhibit the formation of homodimers, an Fc alteration that extends half life, an alteration that inhibits Fc gamma receptor (FcγR) binding, and/or an alteration that enhances ADCC.

An "Fc alteration that extends half life," as meant herein is an alteration within an Fc polypeptide chain that lengthens the *in vivo* half life of a protein that contains the altered Fc polypeptide chain as compared to the half life of a similar protein containing the same Fc polypeptide, except that it does not contain the alteration. Such alterations can be included in an Fc polypeptide chain that is part of a Bi-Fc. The alterations M252Y, S254T, and T256E (methionine at position 252 changed to tyrosine; serine at position 254 changed to threonine; and threonine at position 256 changed to glutamic acid; numbering according to EU numbering as shown in Table 2) are Fc alterations that extend half life and can be used together, separately or in any combination. These alterations and a number of others are described in detail in U.S. Patent 7,083,784. Similarly, M428L and N434S are Fc alterations that extend half life and can be used together, separately or in any combination. These alterations and a number of others are described in detail in U.S. Patent Application Publication 2010/0234575 and U.S. Patent 7,670,600. In addition, any substitution at one of the following sites can be considered an Fc alteration that extends half life as meant here: 250, 251, 252, 259, 307, 308, 332, 378, 380, 428, 430, 434, 436. Each of these alterations or combinations of these alterations can be used to extend the half life of a heterodimeric or monomeric Bi-Fc antibody as described herein. Other alterations that can be used to extend half life are described in detail in International Application PCT/US2012/070146 filed December 17, 2012 (published as WO 2013/096221). The embodiments described in this application include insertions between positions 384 and 385 (EU numbering as shown in Table 2) that extend half life, including the following amino acid sequences: GGCVFNMFNCGG (SEQ ID NO:36), GGCHLPFAVCGG (SEQ ID NO:37), GGCGHEYMWCGG (SEQ ID NO:38), GGCWPLQDYCGG(SEQ ID NO:39), GGCMQMNKWCGG (SEQ ID NO:40), GGCDGRTKYCGG (SEQ ID NO:41), GGCALYPTNCGG (SEQ ID NO:42), GGCGKHWHQCGG (SEQ ID NO:43), GGCHSFKHFCGG (SEQ ID NO:44), GGCQGMWTWCGG (SEQ ID NO:45), GGCAQQWHHEYCGG (SEQ ID NO:46), and GGCERFHHACGG (SEQ ID NO:47), among others. Heterodimeric or monomeric Bi-Fc antibodies contain such insertions.

An "**Fc alteration that is unfavorable to homodimer formation**," includes any alteration in an Fc polypeptide chain such that the Fc polypeptide chain has decreased ability to form homodimers compared to a wild type Fc polypeptide chain. Such alterations are described in detail in U.S. Patent Application Publication US2012/0244578. Examples of such alterations include, without limitation, the following, which can be used individually or in any combination: K392D, K392E, K439D, K439E, K370D, K370E, E357K, and E357R. Such alterations can be included in an Fc polypeptide chain that is part of a Bi-Fc, especially in embodiments where the Bi-Fc is a monomer. In some embodiments, such alterations occur in the CH3 region of the Fc polypeptide chain and comprise an alteration such that one or more charged amino acids in the wild type amino acid sequence are replaced with amino acids electrostatically unfavorable to CH3 homodimer formation, and/or one or more hydrophobic interface residues are replaced with a small polar amino acid, such as, for example, asparagine, cysteine, glutamine, serine, or threonine. More specifically, for example, a charged amino acid, *e.g.,* lysine at position 392 and/or position 409, can be replaced with a neutral or oppositely charged amino acid, for example aspartate or glutamate. This can also occur at any other charged amino acid within the Fc polypeptide chain. Alternatively or in addition, one or more hydrophobic interface residues selected from the group consisting of Y349, L351, L368, V397, L398, F405, and Y407 can be replaced with a small polar amino acid. Further, the Fc polypeptide chain can have one or more mutated cysteine residues to prevent disulfide bond formation. Particularly useful cysteine mutations in this regard are those in the hinge region of the Fc polypeptide chain. Such cysteines can be deleted or substituted with other amino acids. For monomeric Bi-Fc's, the hinge region can be entirely absent.

"Heterodimerizing alterations" generally refer to alterations in the A and B chains of an Fc region that facilitate the formation of heterodimeric Fc regions, that is, Fc regions in which the A chain and the B chain of the Fc region do not have identical amino acid sequences. Such alterations can be included in an Fc polypeptide chain that is part of a Bi-Fc. Heterodimerizing alterations can be asymmetric, that is, an A chain having a certain alteration can pair with a B chain having a different alteration. These alterations facilitate heterodimerization and disfavor homodimerization. Whether hetero- or homo-dimers have formed can be assessed by size differences as determined by polyacrylamide gel electrophoresis in some situations or by other appropriate means (such as molecular tags or binding by antibodies that recognize certain portions of the heterodimer) in situations where size is not a distinguishing characteristic. One example of such paired heterodimerizing alterations are the so-called "knobs and holes" substitutions. *See, e.g.,* US Patent 7,695,936 and US Patent Application Publication 2003/0078385. As meant herein, an Fc region that contains one pair of knobs and holes substitutions, contains one substitution in the A chain and another in the B chain. For example, the following knobs and holes substitutions in the A and B chains of an IgG1 Fc region have been found to increase heterodimer formation as compared with that found with unmodified A and B chains: 1) Y407T in one chain and T366Y in the other; 2) Y407A in one chain and T366W in the other; 3) F405A in one chain and T394W in the other; 4) F405W in one chain and T394S in the other; 5) Y407T in one chain and T366Y in the other; 6) T366Y and F405A in one chain and T394W and Y407T in the other; 7) T366W and F405W in one chain and T394S and Y407A in the other; 8) F405W and Y407A in one chain and T366W and T394S in the other; and 9) T366W in one polypeptide of the Fc and T366S, L368A, and Y407V in the other. As explained above, this way of notating mutations can be explained as follows. The amino acid (using the one letter code) normally present at a given position in the CH3 region using the EU numbering system (which is presented in Edelman et al. (1969), Proc. Natl. Acad. Sci. 63: 78-85; *see* also Table 2 below) is followed by the EU position, which is followed by the alternate amino acid that is present at that position. For example, Y407T means that the tyrosine normally present at EU position 407 is replaced by a threonine. Alternatively or in addition to such alterations, substitutions creating new disulfide bridges can facilitate heterodimer formation. *See, e.g.,* US Patent Application Publication 2003/0078385. Such alterations in an IgG1 Fc region include, for example, the following substitutions: Y349C in one Fc polypeptide chain and S354C in the other; Y349C in one Fc polypeptide chain and E356C in the other; Y349C in one Fc polypeptide chain and E357C in the other; L351C in one Fc polypeptide chain and S354C in the other; T394C in one Fc polypeptide chain and E397C in the other; or D399C in one Fc polypeptide chain and K392C in the other. Similarly, substitutions changing the charge of a one or more residue, for example, in the $C_H3$-$C_H3$ interface, can enhance heterodimer formation as explained in WO 2009/089004. Such substitutions are referred to herein as "charge pair substitutions," and an Fc region containing one pair of charge pair substitutions contains one substitution in the A chain and a different substitution in the B chain. General examples of charge pair substitutions include the following: 1) K409D or K409E in one chain plus D399K or D399R in the other; 2) K392D or K392E in one chain plus D399K or D399R in the other; 3) K439D or K439E in one chain plus E356K or E356R in the other; and 4) K370D or K370E in one chain plus E357K or E357R in the other. In addition, the substitutions R355D, R355E, K360D, or K360R in both chains can stabilize heterodimers when used with other heterodimerizing alterations. Specific charge pair substitutions can be used either alone or with other charge pair substitutions. Specific examples of single pairs of charge pair substitutions and combinations thereof include the following: 1) K409E in one chain plus D399K in the other; 2) K409E in one chain plus D399R in the other; 3) K409D in one chain plus D399K in the other; 4) K409D in one chain plus D399R in the other; 5) K392E in one chain plus D399R in the other; 6) K392E in one chain plus D399K in the other; 7) K392D in one chain plus D399R in the other; 8) K392D in one chain plus D399K in the other; 9) K409D and K360D in one chain plus D399K and E356K in the other; 10) K409D and K370D in one chain plus D399K and E357K in the other; 11) K409D and K392D in one chain plus D399K, E356K, and E357K in the other; 12) K409D and K392D on one chain and D399K on the other; 13) K409D and K392D on one chain plus D399K and E356K on the other; 14) K409D and K392D on one chain plus D399K and D357K on the other; 15) K409D and K370D on one chain plus D399K and D357K on the other; 16) D399K on one chain plus K409D and K360D on the other; and 17) K409D and K439D on one chain plus D399K and E356K on the other. Any of the these heterodimerizing alterations can be used in the Fc regions of the heterodimeric bispecific antibodies described herein.

An "alteration that inhibits FcγR binding," as meant herein, is one or more insertions, deletions, or substitutions within an Fc polypeptide chain that inhibits the binding of FcγRIIA, FcγRIIB, and/or FcγRIIIA as measured, for example, by an ALPHALISA®-based competition binding assay (PerkinElmer, Waltham, MA). Such alterations can be included in an Fc polypeptide chain that is part of a Bi-Fc. More specifically, alterations that inhibit Fc gamma receptor (FcγR) binding include L234A, L235A, or any alteration that inhibits glycosylation at N297, including any substitution at N297. In addition, along with alterations that inhibit glycosylation at N297, additional alterations that stabilize a dimeric Fc region by creating additional disulfide bridges are also contemplated. Further examples of alterations that inhibit FcγR binding include a D265A alteration in one Fc polypeptide chain and an A327Q alteration in the other Fc polypeptide chain. Some such mutations are described in, *e.g.,* Xu et al. (2000), Cellular Immunol. 200: 16-26.

An "alteration that enhances ADCC," as meant herein is one or more insertions, deletions, or substitutions within an Fc polypeptide chain that enhances antibody dependent cell-mediated cytotoxicity (ADCC). Such alterations can be included in an Fc polypeptide chain that is part of a Bi-Fc. Many such alterations are described in International Patent Application Publication WO 2012/125850. Such alterations can be included in an Fc polypeptide chain that is part of a heterodimeric bispecific antibody as described herein. ADCC assays can be performed as follows. Cell lines that express high and lower amounts of a cancer cell antigen on the cell surface can be used as target cells. These target cells can be labeled with carboxyfluorescein succinimidyl ester (CFSE) and then washed once with phosphate buffered saline (PBS) before being deposited into 96-well microtiter plates with V-shaped wells. Purified immune effector cells, for example T cells, NK cells, macrophages, neutrophils can be added to each well. A monospecific antibody that binds to the cancer antigen and contains the alteration(s) being tested and an isotype-matched control antibody can be diluted in a 1:3 series and added to the wells. The cells can be incubated at 37 °C with 5% $CO_2$ for 3.5 hrs. The cells can be spun down and re-suspended in 1x FACS buffer (1x phosphate buffered saline (PBS) containing 0.5% fetal bovine serum (FBS)) with the dye TO-PRO®-3 iodide (Molecular Probes, Inc. Corporation, Oregon, USA), which stains dead cells, before analysis by fluorescence activated cell sorting (FACS). The percentage of cell killing can be calculated using the follow formula:

*(percent tumor cell lysis with bispecific – percent tumor cell lysis without bispecific)/*

*(percent total cell lysis – percent tumor cell lysis without bispecific)*

Total cell lysis is determined by lysing samples containing effector cells and labeled target cells without a bispecific molecule with cold 80% methanol. Exemplary alterations that enhance ADCC include the following alterations in the A and B chains of an Fc region: (a) the A chain comprises Q311M and K334V substitutions and the B chain comprises L234Y, E294L, and Y296W substitutions or vice versa; (b) the A chain comprises E233L, Q311M, and K334V substitutions and the B chain comprises L234Y, E294L, and Y296W substitutions or vice versa; (c) the A chain comprises L234I, Q311M, and K334V substitutions and the B chain comprises L234Y, E294L, and Y296W substitutions or vice versa; (d) the A chain comprises S298T and K334V substitutions and the B chain comprises L234Y, K290Y, and Y296W substitutions or vice versa; (e) the A chain comprises A330M and K334V substitutions and the B chain comprises L234Y, K290Y, and Y296W substitutions or vice versa; (f) the A chain comprises A330F and K334V substitutions and the B chain comprises L234Y, K290Y, and Y296W substitutions or vice versa; (g) the A chain comprises Q311M, A330M, and K334V substitutions and the B chain comprises L234Y, E294L, and Y296W substitutions or vice versa; (h) the A chain comprises Q311M, A330F, and K334V substitutions and the B chain comprises L234Y, E294L, and Y296W substitutions or vice versa; (i) the A chain comprises S298T, A330M, and K334V substitutions and the B chain comprises L234Y, K290Y, and Y296W substitutions or vice versa; (j) the A chain comprises S298T, A330F, and K334V substitutions and the B chain comprises L234Y, K290Y, and Y296W substitutions or vice versa; (k) the A chain comprises S239D, A330M, and K334V substitutions and the B chain comprises L234Y, K290Y, and Y296W substitutions or vice versa; (l) the A chain comprises S239D, S298T, and K334V substitutions and the B chain comprises L234Y, K290Y, and Y296W substitutions or vice versa; (m) the A chain comprises a K334V substitution and the B chain comprises Y296W and S298C substitutions or vice versa; (n) the A chain comprises a K334V substitution and the B chain comprises L234Y, Y296W, and S298C substitutions or vice versa; (o) the A chain comprises L235S, S239D, and K334V substitutions and the B chain comprises L234Y, K290Y, and Y296W, substitutions or vice versa; (p) the A chain comprises L235S, S239D, and K334V substitutions and the B chain comprises L234Y, Y296W, and S298C substitutions or vice versa; (q) the A chain comprises Q311M and K334V substitutions and the B chain comprises L234Y, F243V, and Y296W substitutions or vice versa; (r) the A chain comprises Q311M and K334V substitutions and the B chain comprises L234Y, K296W, and S298C substitutions or vice versa; (s) the A chain comprises S239D and K334V substitutions and the B chain comprises L234Y, K290Y, and Y296W substitutions or vice versa; (t) the A chain comprises S239D and K334V substitutions and the B chain comprises L234Y, Y296W, and S298C substitutions or vice versa; (u) the A chain comprises F243V and K334V substitutions and the B chain comprises L234Y, K290Y, and Y296W, substitutions or vice versa; (v) the A chain comprises F243V and K334V substitutions and the B chain comprises L234Y, Y296W, and S298C substitutions or vice versa; (w) the A chain comprises E294L and K334V substitutions and the B chain comprises L234Y, K290Y, and Y296W substitutions or vice versa; (x) the A chain comprises E294L and K334V substitutions and the B chain comprises L234Y, Y296W, and S298C substitutions or vice versa; (y) the A chain comprises A330M and K334V substitutions and the B chain comprises L234Y and Y296W substitutions or vice versa; or (z) the A chain comprises A330M and K334V substitutions and the B chain comprises K290Y and Y296W substitutions or vice versa.

An "**IgG antibody,**" as meant herein, is an antibody consisting essentially of two immunoglobulin IgG heavy chains and two immunoglobulin light chains, which can be kappa or lambda light chains. More specifically, the heavy chains contain a VH region, a CH1 region, a hinge region, a CH2 region, and a CH3 region in that order, while the light chains contain a VL region followed by a CL region. Numerous sequences of such immunoglobulin regions are known in the art. *See, e.g.,* Kabat et al. in SEQUENCES OF IMMUNOLOGICAL INTEREST, Public Health Service N.I.H.,

Bethesda, MD, 1991. Close variants of a known and/or naturally-occurring IgG antibody comprising no more than 10 amino acid substitutions, insertions, and/or deletions of a single amino acid per 100 amino acids relative to a known or naturally occurring sequence of an immunoglobulin IgG heavy and/or light chain are encompassed within what is meant by an IgG antibody.

An "**immune effector cell**," as meant herein, is a cell that is involved in the mediation of a cytolytic immune response, including, for example, T cells, NK cells, macrophages, or neutrophils. The heterodimeric or monomeric Bi-Fc antibodies described herein bind to an antigen that is part of a protein expressed on the surface of an immune effector cell. Such proteins are referred to herein as "**effector cell proteins**."

An "**immunoglobulin heavy chain**," as meant herein, consists essentially of a VH region, a CH1 region, a hinge region, a CH2 region, a CH3 region in that order, and, optionally, a region downstream of the CH3 region in some isotypes. Close variants of an immunoglobulin heavy chain containing no more than 10 amino acid substitutions, insertions, and/or deletions of a single amino acid per 100 amino acids relative to a known or naturally occurring immunoglobulin heavy chain amino acid sequence are encompassed within what is meant by an immunoglobulin heavy chain.

A "**immunoglobulin light chain**," as meant herein, consists essentially of a light chain variable region (VL) and a light chain constant domain (CL). Close variants of an immunoglobulin light chain containing no more than 10 amino acid substitutions, insertions, and/or deletions of a single amino acid per 100 amino acids relative to a known or naturally occurring immunoglobulin light chain amino acid sequence are encompassed within what is meant by an immunoglobulin light chain.

An "**immunoglobulin variable region**," as meant herein, is a VH region, a VL region, or a variant thereof. Close variants of an immunoglobulin variable region containing no more than 10 amino acid substitutions, insertions, and/or deletions of a single amino acid per 100 amino acids relative to a known or naturally occurring immunoglobulin variable region amino acid sequence are encompassed within what is meant by an immunoglobulin variable region. Many examples of VH and VL regions are known in the art, such as, for example, those disclosed by Kabat et al in SEQUENCES OF IMMUNOLOGICAL INTEREST, Public Health Service N.I.H., Bethesda, MD, 1991. Based on the extensive sequence commonalities in the less variable portions of the VH and VL regions, the position within a sequence of more variable regions, and the predicted tertiary structure, one of skill in the art can recognize an immunoglobulin variable region by its sequence. *See, e.g.,* Honegger and Plückthun (2001), J. Mol. Biol. 309: 657-670.

An immunoglobulin variable region contains three hypervariable regions, known as complementarity determining region 1 (CDR1), complementarity determining region 2 (CDR2), and complementarity determining region 3 (CDR3). These regions form the antigen binding site of an antibody. The CDRs are embedded within the less variable framework regions (FR1-FR4). The order of these subregions within an immunoglobulin variable region is as follows: FR1-CDR1-FR2-CDR2-FR3-CDR3-FR4. Numerous sequences of immunoglobulin variable regions are known in the art. *See, e.g.,* Kabat et al., SEQUENCES OF PROTEINS OF IMMUNOLOGICAL INTEREST, Public Health Service N.I.H., Bethesda, MD, 1991.

CDRs can be located in a VH region sequence in the following way. CDR1 starts at approximately residue 31 of the mature VH region and is usually about 5-7 amino acids long, and it is almost always preceded by a Cys-Xxx-Xxx-Xxx-Xxx-Xxx-Xxx-Xxx-Xxx (SEQ ID NO:1) (where "Xxx" is any amino acid). The residue following the heavy chain CDR1 is almost always a tryptophan, often a Trp-Val, a Trp-Ile, or a Trp-Ala. Fourteen amino acids are almost always between the last residue in CDR1 and the first in CDR2, and CDR2 typically contains 16 to 19 amino acids. CDR2 may be immediately preceded by Leu-Glu-Trp-Ile-Gly (SEQ ID NO:2) and may be immediately followed by Lys/Arg-Leu/Ile/Val/Phe/Thr/Ala-Thr/Ser/Ile/Ala. Other amino acids may precede or follow CDR2. Thirty two amino acids are almost always between the last residue in CDR2 and the first in CDR3, and CDR3 can be from about 3 to 25 residues long. A Cys-Xxx-Xxx almost always immediately precedes CDR3, and a Trp-Gly-Xxx-Gly (SEQ ID NO:3) almost always follows CDR3.

Light chain CDRs can be located in a VL region in the following way. CDR1 starts at approximately residue 24 of the mature antibody and is usually about 10 to 17 residues long. It is almost always preceded by a Cys. There are almost always 15 amino acids between the last residue of CDR1 and the first residue of CDR2, and CDR2 is almost always 7 residues long. CDR2 is typically preceded by Ile-Tyr, Val-Tyr, Ile-Lys, or Ile-Phe. There are almost always 32 residues between CDR2 and CDR3, and CDR3 is usually about 7 to 10 amino acids long. CDR3 is almost always preceded by Cys and usually followed by Phe-Gly-Xxx-Gly (SEQ ID NO:4).

A "linker," as meant herein, is a peptide that links two polypeptides, which can be two immunoglobulin variable regions or a variable region and an Fc polypeptide chain in the context of a Bi-Fc antibody. In some embodiments, a linker can be a peptide no more than 12, 11, 10, 9, 8, 7, 6, or 5 amino acids long. In other embodiments, a linker can be 20-30, or 30-40 amino acids long. In other embodiments, a linker can be about, 2, 3, 4, 5, 6, 7, 8, 9, 10,11, 12, 13, 14, 15, 16, 17, 18, 19, 20, 21, 22, 23, 24, 25, 26, 27, 28, 29, or 30 amino acids long. Exemplary linkers include, for example, the amino acid sequences TVAAP (SEQ ID NO:17), ASTKGP (SEQ ID NO:18), GGGGSGGGGS (SEQ ID NO:19), GGGGSAAA (SEQ ID NO:20), GGGGSGGGGSGGGGS (SEQ ID NO:21), and AAA, among many others.

A Bi-Fc "mediates cytolysis of a target cell by an immune effector cell," as meant herein, when addition of an amount from 0.001 pM to 20000 pM of the Bi-Fc to a cell cytolysis assay as described in the section below entitled "Target Cell Cytolysis Assays" and in Example 3 effectively elicits cytolysis of the target cells.

"**Non-chemotherapeutic anti-neoplastic agents**" are chemical agents, compounds, or molecules having cytotoxic or cytostatic effects on cancer cells other than chemotherapeutic agents. Non-chemotherapeutic antineoplastic agents may, however, be targeted to interact directly with molecules that indirectly affect cell division such as cell surface receptors, including receptors for hormones or growth factors. However, non-chemotherapeutic antineoplastic agents do not interfere directly with processes that are intimately linked to cell division such as, for example, DNA replication, RNA synthesis, protein synthesis, or mitotic spindle function, assembly, or disassembly. Examples of non-chemotherapeutic anti-neoplastic agents include inhibitors of Bcl2, inhibitors of farnesyltransferase, anti-estrogenic agents such as tamoxifen, anti-androgenic compounds, interferon, arsenic, retinoic acid, retinoic acid derivatives, antibodies targeted to tumor-specific antigens, and inhibitors of the Bcr-Abl tyrosine kinase (*e.g.,* the small molecule STI-571 marketed under the trade name GLEEVEC™ by Novartis, New York and New Jersey, USA and Basel, Switzerland), among many possible non-chemotherapeutic anti-neoplastic agents.

"**Percent identity**" of one amino acid or nucleic acid sequence with another can be determined using a computer program. An exemplary computer program is the Genetics Computer Group (GCG; Madison, WI) Wisconsin package version 10.0 program, GAP (Devereux et al (1984), Nucleic Acids Res. 12: 387-95). The preferred default parameters for the GAP program includes: (1) The GCG implementation of an unary comparison matrix (containing a value of 1 for identities and 0 for non-identities) for nucleotides, and the weighted amino acid comparison matrix of Gribskov and Burgess, ((1986) Nucleic Acids Res. 14: 6745) as described in Atlas of Polypeptide Sequence and Structure, Schwartz and Dayhoff, eds., National Biomedical Research Foundation, pp. 353-358 (1979) or other comparable comparison matrices; (2) a penalty of 8 for each gap and an additional penalty of 2 for each symbol in each gap for amino acid sequences, or a penalty of 50 for each gap and an additional penalty of 3 for each symbol in each gap for nucleotide sequences; (3) no penalty for end gaps; and (4) no maximum penalty for long gaps. Other programs used by those skilled in the art of sequence comparison can also be used.

In connection with comparisons to determine sequence identity of polynucleotides or polypeptides, an "**identity region**" is the portion of the polynucleotide or polypeptide sequence that is matched, partially or exactly, with another polynucleotide or polypeptide by the computer program GAP (Devereux et al. (1984), Nucleic Acids Res. 12: 387-95) using the parameters stated below. For example, when a polypeptide of 20 amino acids is aligned with a considerably longer protein, the first 10 amino acids match the longer protein exactly, and the last 10 amino acids do not match the longer protein at all, the identity region is 10 amino acids. If, on the other hand, the first and last amino acids of the 20 amino acid polypeptide match the longer protein, and eight other matches are scattered between, the identity region is 20 amino acids long. However, long stretches in either aligned strand without identical or conservatively substituted amino acids or identical nucleotides of at least, for example, 20 amino acids or 60 nucleotides constitute an endpoint of an identity region, as meant herein.

A "**target cell**" is a cell that a Bi-Fc binds to and that is involved in mediating a disease. In some cases, a target cell can be a cell that is ordinarily involved in mediating an immune response, but is also involved in the mediation of a disease. For example in B cell lymphoma, a B cell, which is ordinarily involved in mediating immune response, can be a target cell. In some embodiments, a target cell is a cancer cell, a cell infected with a pathogen, or a cell involved in mediating an autoimmune or inflammatory disease, for example a fibrotic disease. The Bi-Fc can bind to the target cell via binding to an antigen on a "**target cell protein**," which is a protein that is displayed on the surface of the target cell, possibly a highly expressed protein.

"**Tumor burden**" refers to the number of viable cancer cells, the number of tumor sites, and/or the size of the tumor(s) in a patient suffering from a cancer. A reduction in tumor burden can be observed, for example, as a reduction in the amount of a tumor-associated antigen or protein in a patient's blood or urine, a reduction in the number of tumor cells or tumor sites, and/or a reduction in the size of one or more tumors.

A "**therapeutically effective amount**" of a Bi-Fc or any other drug is an amount that has the effect of, for example, reducing or eliminating the tumor burden of a cancer patient or reducing or eliminating the symptoms of any disease condition that the protein is used to treat. A therapeutically effective amount need not completely eliminate all symptoms of the condition, but may reduce severity of one or more symptoms or delay the onset of more serious symptoms or a more serious disease that can occur with some frequency following the treated condition.

"**Treatment**" of any disease mentioned herein encompasses an alleviation of at least one symptom of the disease, a reduction in the severity of the disease, or the delay or prevention of disease progression to more serious symptoms that may, in some cases, accompany the disease or lead to at least one other disease. Treatment need not mean that the disease is totally cured. A useful therapeutic agent needs only to reduce the severity of a disease, reduce the severity of one or more symptoms associated with the disease or its treatment, or delay the onset of more serious symptoms or a more serious disease that can occur with some frequency following the treated condition.

When it is said that a named VH/VL pair of immunoglobulin variable regions can bind to a target cell or and/or an immune effector cell "**when they are part of an IgG and/or scFv antibody**," it is meant that an IgG antibody that contains the named VH region in both heavy chains and the named VL region in both light chains and/or an scFv antibody containing these VH and VL regions can bind to the target cell and/or the immune effector cell. The binding assay described in Example 2 can be used to assess binding.

## Bi-Fc Molecules

In the most general sense, a Bi-Fc can bind monovalently to two different antigens and comprises one polypeptide chain or two different polypeptide chains having different amino acid sequences. In addition, it can bind to the neonatal Fc receptor (FcRn) at slightly acidic pH (about pH 5.5-6.0) via its Fc region. This interaction with FcRn can lenthen the half life of a molecule *in vivo.* The first polypeptide chain (which, in some cases, is the only polypeptide chain) comprises a human IgG Fc polypeptide chain and two VH regions plus two VL regions that are separated by linkers. The Fc polypeptide chain can be N-terminal or C-terminal relative to the four immunoglobulin variable regions, and it can be joined to the variable regions via a linker. The second polypeptide chain, when present, comprises a human IgG Fc polypeptide chain. A Bi-Fc can bind to an immune effector cell and a target cell and/or can mediate cytolysis of a target cell by an immune effector cell. The general structure of a Bi-Fc is diagrammed in Figure 1, which shows embodiments where the Fc polypeptide chain is C-terminal (panels A and B) and embodiments where the Fc polypeptide chain in N-terminal (panels C and D).

More particular embodiments specify the order of immunoglobulin variable regions and the length of the linkers and specify which immunoglobulin variable regions can associate to form a binding site for an effector cell protein or a target cell protein. Generally, the antigen-binding portion of an antibody includes both a VH and a VL region, referred to herein as a "**VH/VL pair**," although in some cases a VH or a VL region can bind to an antigen without a partner. *See, e.g.,* US Application Publication 2003/0114659.

In one group of embodiments, the four variable regions can be arranged in the following order: VH1-linker1-VL1-linker2-VH2-linker3-VL2, where VH1/VL1 is an antigen-binding pair and VH2/VL2 is another antigen-binding pair. In this group of embodiments, linker1 and linker3 are at least 15 amino acids long, and linker2 is less than 12 amino acids long or, in some cases, absent. In some embodiments, the VH1/VL1 pair can bind to a target cell protein, and the VH2/VL2 pair can bind to an effector cell protein. In other embodiments, the VH1/VL1 pair can bind to an effector cell protein, and the VH2/VL2 pair can bind to a target cell protein.

In another group of embodiments the four variable regions can be arranged in the following order: VL1-linker1-VH1-linker2-VL2-linker3-VH2, where VH1/VL1 is an antigen-binding pair and VH2/VL2 is an antigen-binding pair. In these embodiments, linkers is less than 12 amino acids long or absent, and linker1 and linkers are at least 15 amino acids long. In some embodiments, the VH1/VL1 pair can bind to a target cell protein, and the VH2/VL2 pair can bind to an effector cell protein. In other embodiments, the VH1/VL1 pair can bind to an effector cell protein, and the VH2/VL2 pair can bind to a target cell protein.

In another group of embodiments the four variable regions can be arranged in the following order: VH1-linker1-VL1-linker2-VL2-linker3-VH2, where VH1/VL1 is an antigen-binding pair and VH2/VL2 is an antigen-binding pair. In these embodiments, linker2 is less than 12 amino acids long or absent, and linker1 and linkers are at least 15 amino acids long. In some embodiments, the VH1/VL1 pair can bind to a target cell protein, and the VH2/VL2 pair can bind to an effector cell protein. In other embodiments, the VH1/VL1 pair can bind to an effector cell protein, and the VH2/VL2 pair can bind to a target cell protein.

In further group of embodiments the four variable regions can be arranged in the following order: VL1-linker1-VH1-linker2-VH2-linker3-VL2, where VH1/VL1 is an antigen-binding pair and VH2/VL2 is an antigen-binding pair. In these embodiments, linkers is less than 12 amino acids long or absent, and linker1 and linkers are at least 15 amino acids long. In some embodiments, the VH1/VL1 pair can bind to a target cell protein, and the VH2/VL2 pair can bind to an effector cell receptor. In other embodiments, the VH1/VL1 pair can bind to an effector cell protein, and the VH2/VL2 pair can bind to a target cell protein.

A Bi-Fc can comprise an Fc polypeptide chain of an antibody. The Fc polypeptide chain is of human origin. For example, the Fc polypeptide chain can be a human IgG1, IgG2, IgG3, or IgG4 Fc polypeptide chain. In addition, as explained above, an Fc polypeptide chain can comprise a limited number of alterations. More particularly, an Fc polypeptide chain can contain no more than 10 insertions, deletions, and/or substitutions of a single amino acid per 100 amino acids relative to a known or naturally-occurring sequence. In some embodiments, the two Fc polypeptide chains of a heterodimeric Bi-Fc contain heterodimerizing alterations, which can be, for example, charge pair substitutions. For example, the first polypeptide chain of the Bi-Fc can comprise the substitutions K409D, or K409E or K392E and the second polypeptide chain of the Bi-Fc can comprise D399K or D399R or D356R. Alternatively, the first polypeptide chain of the Bi-Fc can comprise D399K or D399R and D356K or D356R, and the second polypeptide chain of the Bi-Fc can comprise K409D, or K409E and K392D or K392E. An Fc polypeptide chain can also comprise one or more "Fc alterations unfavorable to homodimer formation" and/or one or more "Fc alterations that extend half life," as meant herein.

In monomeric embodiments of the Bi-Fc, the Bi-Fc can comprise one or more "Fc alterations that are unfavorable to homodimer formation," as defined above.

Other kinds of alterations can also be part of an Fc polypeptide chain that is part of a Bi-Fc. In one aspect, an Fc region included in a Bi-Fc can comprise one or more "alterations that inhibit the binding of an Fc gamma receptor (FcγR)" to the Fc region as defined above. In another aspect, an Fc region included in a Bi-Fc can comprise one or more "Fc alteration that extends half life," as defined above. In still another aspect, one or more "alterations that enhance ADCC" can be included in an Fc region that is part of a Bi-Fc.

In some embodiments the amino acid sequences of the Fc polypeptides can be human amino acid sequences or variants thereof that comprise not more than 10 deletions, insertions, or substitutions of a single amino acid per 100 amino acids of sequence relative to a human amino acid sequence. Alternatively, an Fc polypeptide that is part of a Bi-Fc can be 90% or 95% identical to a human IgG Fc polypeptide chain and the identity region can be at least about 50, 60, 70, 80, 90, or 100 amino acids long. The isotype of the Fc polypeptide is IgG, such as IgG1, IgG2, IgG3, or IgG4,. Table 2 below shows an alignment of the amino acid sequences of human IgG1, IgG2, IgG3, and IgG4 Fc polypeptide chain sequences.

## Table 2: Amino acid sequences of human IgG Fc regions

```
IgG1    ------------------------------------------------
IgG2    ------------------------------------------------
IgG3    ELKTPLGDTTHTCPRCPEPKSCDTPPPCPRCPEPKSCDTPPPCPRCP
IgG4    ------------------------------------------------


         225       235       245       255       265       275
          *         *         *         *         *         *
IgG1    EPKSCDKTHTCPPCPAPELLGGPSVFLFPPKPKDTLMISRTPEVTCVVVDVSHEDPEVKF
IgG2    ERKCCVE---CPPCPAPPVA-GPSVFLFPPKPKDTLMISRTPEVTCVVVDVSHEDPEVQF
IgG3    EPKSCDTPPPCPRCPAPELLGGPSVFLFPPKPKDTLMISRTPEVTCVVVDVSHEDPEVQF
IgG4    ESKYG---PPCPSCPAPEFLGGPSVFLFPPKPKDTLMISRTPEVTCVVVDVSQEDPEVQF


         285       295       305       315       325       335
          *         *         *         *         *         *
IgG1    NWYVDGVEVHNAKTKPREEQYNSTYRVVSVLTVLHQDWLNGKEYKCKVSNKALPAPIEKT
IgG2    NWYVDGMEVHNAKTKPREEQFNSTFRVVSVLTVVHQDWLNGKEYKCKVSNKGLPAPIEKT
IgG3    KWYVDGVEVHNAKTKPREEQYNSTFRVVSVLTVLHQDWLNGKEYKCKVSNKALPAPIEKT


IgG4    NWYVDGVEVHNAKTKPREEQFNSTYRVVSVLTVLHQDWLNGKEYKCKVSNKGLPSSIEKT


         345       355       365       375       385       395
          *         *         *         *         *         *
IgG1    ISKAKGQPREPQVYTLPPSRDELTKNQVSLTCLVKGFYPSDIAVEWESNGQPENNYKTTP
IgG2    ISKTKGQPREPQVYTLPPSREEMTKNQVSLTCLVKGFYPSDIAVEWESNGQPENNYKTTP
IgG3    ISKTKGQPREPQVYTLPPSREEMTKNQVSLTCLVKGFYPSDIAVEWESSGQPENNYNTTP
IgG4    ISKAKGQPREPQVYTLPPSQEEMTKNQVSLTCLVKGFYPSDIAVEWESNGQPENNYKTTP


         405       415       425       435       445
          *         *         *         *         *
IgG1    PVLDSDGSFFLYSKLTVDKSRWQQGNVFSCSVMHEALHNHYTQKSLSLSPGK   (SEQ ID NO:25)
IgG2    PMLDSDGSFFLYSKLTVDKSRWQQGNVFSCSVMHEALHNHYTQKSLSLSPGK   (SEQ ID NO:26)
IgG3    PMLDSDGSFFLYSKLTVDKSRWQQGNIFSCSVMHEALHNRFTQKSLSLSPGK   (SEQ ID NO:27)
IgG4    PVLDSDGSFFLYSRLTVDKSRWQEGNVFSCSVMHEALHNHYTQKSLSLSLGK   (SEQ ID NO:28)
```

The numbering shown in Table 2 is according the EU system of numbering, which is based on the sequential numbering of the constant region of a human IgG1 antibody. Edelman et al. (1969), Proc. Natl. Acad. Sci. 63: 78-85. Thus, it does not accommodate the additional length of the IgG3 hinge well. It is nonetheless used herein to designate positions in an Fc region because it is still commonly used in the art to refer to positions in Fc regions. The hinge regions of the IgG1, IgG2, and IgG4 Fc polypeptides extend from about position 216 to about 230. It is clear from the alignment that the IgG2 and IgG4 hinge regions are each three amino acids shorter than the IgG1 hinge. The IgG3 hinge is much longer, extending for an additional 47 amino acids upstream. The CH2 region extends from about position 231 to 340, and the CH3 region extends from about position 341 to 447.

Naturally occurring amino acid sequences of Fc polypeptides can be varied slightly. Such variations can include no more than 10 insertions, deletions, and/or substitutions of one amino acid per 100 amino acids of sequence in a known or naturally-occurring amino acid sequence of an Fc polypeptide. If there are substitutions, they can be conservative amino acid substitutions, as defined above. The Fc polypeptides on the first and second polypeptide chains of a heterodimeric Bi-Fc can differ in amino acid sequence. In some embodiments, they can include one or more "heterodimerizing alterations," "alterations that enchance ADCC," "alterations that inhibit FcγR binding," "Fc alterations that are unfavorable to homodimer formation," and/or "Fc alterations that extend half life," as defined above.

A Bi-Fc can bind to an immune effector cell through an antigen that is part of an effector cell protein and can bind to a target cell through an antigen that is part of a target cell protein. A number of possible effector cell proteins are described in detail below. Similarly, a number of possible target cell proteins is also described below. A Bi-Fc can bind to any combination of an effector cell protein and a target cell protein.

Exemplary amino acid sequences of Bi-Fc's include the following amino acid sequences: SEQ ID NOs:10 and 12 (a heterodimeric Bi-Fc); SEQ ID NOs:15 and 12 (a heterodimeric Bi-Fc); and SEQ ID NO:34 (a monomeric Bi-Fc that includes the alterations Y349T, K392D, and K409D (EU numbering) in its Fc polypeptide chain portion).

### Nucleic Acids Encoding Bi-Fc Molecules

Provided are nucleic acids encoding Bi-Fc's. Numerous nucleic acid sequences encoding immunoglobulin regions including VH, VL, hinge, CH1, CH2, CH3, and CH4 regions are known in the art. *See, e.g.,* Kabat et al. in SEQUENCES OF IMMUNOLOGICAL INTEREST, Public Health Service N.I.H., Bethesda, MD, 1991. Using the guidance provided herein, one of skill in the art could combine such nucleic acid sequences and/or other nucleic acid sequences known in the art to create nucleic acid sequences encoding Bi-Fc's. Exemplary nucleic acids encoding Bi-Fc's include (1) SEQ ID NOs:11 and 13, (2) SEQ ID NOs:16 and 13, and (3) SEQ ID NO:35.

In addition, nucleic acid sequences encoding Bi-Fc's can be determined by one of skill in the art based on the amino acid sequences provided herein and elsewhere and knowledge in the art. Besides more traditional methods of producing cloned DNA segments encoding a particular amino acid sequence, companies such as DNA 2.0 (Menlo Park, CA, USA) and BlueHeron (Bothell, WA, USA), among others, now routinely produce chemically synthesized, gene-sized DNAs of any desired sequence to order, thus streamlining the process of producing such DNAs.

### Methods of Making Bi-Fc Molecules

Bi-Fc's can be made using methods well known in the art. For example, nucleic acids encoding the one or two polypeptide chains of a Bi-Fc can be introduced into a cultured host cell by a variety of known methods, such as, for example, transformation, transfection, electroporation, bombardment with nucleic acid-coated microprojectiles, etc. In some embodiments the nucleic acids encoding a Bi-Fc can be inserted into a vector appropriate for expression in the host cells before being introduced into the host cells. Typically such vectors can contain sequence elements enabling expression of the inserted nucleic acids at the RNA and protein levels. Such vectors are well known in the art, and many are commercially available. The host cells containing the nucleic acids can be cultured under conditions so as to enable the cells to express the nucleic acids, and the resulting Bi-Fc's can be collected from the cell mass or the culture medium. Alternatively, a Bi-Fc can be produced *in vivo,* for example in plant leaves (*see, e.g.,* Scheller et al. (2001), Nature Biotechnol. 19: 573-577 and references cited therein), bird eggs (*see, e.g.,* Zhu et al. (2005), Nature Biotechnol. 23: 1159-1169 and references cited therein), or mammalian milk (*see, e.g.,* Laible et al. (2012), Reprod. Fertil. Dev. 25(1): 315).

A variety of cultured host cells can be used including, for example, bacterial cells such as *Escherichia coli or Bacilis stearothermophilus,* fungal cells such as *Saccharomyces cerevisiae* or *Pichia pastoris,* insect cells such as lepidopteran insect cells including *Spodoptera frugiperda* cells, or mammalian cells such as Chinese hamster ovary (CHO) cells, baby hamster kidney (BHK) cells, monkey kidney cells, HeLa cells, human hepatocellular carcinoma cells, or 293 cells, among many others.

### Immune Effector Cells and Effector Cell Proteins

A Bi-Fc can bind to a molecule expressed on the surface of an immune effector cell (called "**effector cell protein**" herein) and to another molecule expressed on the surface of a target cell (called a "**target cell protein**" herein). The immune effector cell can be a T cell, an NK cell, a macrophage, or a neutrophil. In some embodiments the effector cell protein is a protein included in the T cell receptor (TCR)-CD3 complex. The TCR-CD3 complex is a heteromultimer comprising a heterodimer comprising TCRα and TCRβ or TCRγ and TCRδ plus various CD3 chains from among the CD3 zeta (CD3ζ) chain, CD3 epsilon (CD3ε) chain, CD3 gamma (CD3γ) chain, and CD3 delta (CD3δ) chain. In some embodiments the effector cell protein can be the human CD3 epsilon (CD3ε) chain (the mature amino acid sequence of which is disclosed in SEQ ID NO:22), which can be part of a multimeric protein. Alternatively, the effector cell protein can be human and/or cynomolgus monkey TCRα, TCRβ, TCRδ, TCRγ, CD3β, CD3γ, CD3δ, or CD3ζ.

Moreover, in some embodiments, a Bi-Fc can also bind to a CD3ε chain from a non-human species, such as mouse, rat, rabbit, new world monkey, and/or old world monkey species. Such species include, without limitation, the following mammalian species: *Mus musculus; Rattus rattus; Rattus norvegicus;* the cynomolgus monkey, *Macaca fascicularis;* the hamadryas baboon, *Papio hamadryas;* the Guinea baboon, *Papio papio;* the olive baboon, *Papio anubis; the yellow baboon, Papio cynocephalus;* the Chacma baboon, *Papio ursinus; Callithrix jacchus; Saguinus Oedipus,* and *Saimiri sciureus.* The mature amino acid sequence of the CD3ε chain of cynomolgus monkey is provided in SEQ ID NO:23. As is known in the art of development of protein therapeutics, having a therapeutic that can have comparable activity in humans and species commonly used for preclinical testing, such as mice and monkeys, can simplify and speed drug development. In the long and expensive process of bringing a drug to market, such advantages can be critical.

In more particular embodiments, the heterodimeric bispecific antibody can bind to an epitope within the first 27 amino acids of the CD3ε chain, which may be a human CD3ε chain or a CD3ε chain from different species, particularly one of the mammalian species listed above. The epitope can contain the amino acid sequence Gln-Asp-Gly-Asn-Glu (SEQ ID NO:24). The advantages of an antibody that binds such an epitope are explained in detail in U.S. Patent Application Publication 2010/183615. The epitope to which an antibody binds can be determined by alanine scanning, which is described in, *e.g.,* U.S. Patent Application Publication 2010/183615.

Briefly, alanine scanning can be performed as follows. In a control, DNA encoding wild type CD3ε is inserted into an expression vector appropriate for the host cells, preferably a mammalian T cell line, and transfected into the host cells where it can be expressed as part of a TCR-CD3 complex. In test samples, the DNA encodes CD3ε where a single one of amino acids of CD3ε is changed to alanine. DNA constructs are made to generate a whole series of molecules in which one amino acid at a time is changed to alanine. Only one amino acid is varied from construct to construct to scan all possible amino acid positions in the extracellular domain of CD3ε involved in binding to the Bi-Fc. The Bi-Fc to be tested is made by transfecting mammalian host cells with DNA encoding the Bi-Fc and recovering the antibody from the cell culture. Binding of the Bi-Fc to the cells expressing CD3ε, either wild type or with an alanine replacement, can be assessed by standard fluorescence-activated cell sorting (FACS) methods. Samples where the CD3ε has an alanine replacement at a particular position and the binding detected is reduced or eliminated compared to that detected with a wild type CD3ε indicate that the amino acid at the altered position is normally involved in the binding of the Bi-Fc to CD3ε.

Where a T cell is the immune effector cell, effector cell proteins to which a Bi-Fc can bind include, without limitation, CD3ε, CD3γ, CD3δ, CD3ζ, TCRα, TCRβ, TCRγ, and TCRδ. Where an NK cell or a cytotoxic T cell is an immune effector cell, NKG2D, CD352, NKp46, or CD16a can, for example, be an effector cell protein. Where a CD8$^+$ T cell is an immune effector cell, 4-1BB or NKG2D, for example, can be an effector cell protein. Alternatively, a Bi-Fc could bind to other effector cell proteins expressed on T cells, NK cells, macrophages, or neutrophils.

***Target Cells and Target cell proteins Expressed on Target Cells***

As explained above, a Bi-Fc can bind to an effector cell protein and a target cell protein. The target cell protein can, for example, be expressed on the surface of a cancer cell, a cell infected with a pathogen, or a cell that mediates a disease, for example an inflammatory, autoimmune, and/or fibrotic condition. In some embodiments, the target cell protein can be highly expressed on the target cell, although high levels of expression are not necessarily required.

Where the target cell is a cancer cell, a heterodimeric bispecific antibody as described herein can bind to a cancer cell antigen as described above. A cancer cell antigen can be a human protein or a protein from another species. For example, a heterodimeric bispecific antibody may bind to a target cell protein from a mouse, rat, rabbit, new world monkey, and/or old world monkey species, among many others. Such species include, without limitation, the following species: *Mus musculus; Rattus rattus; Rattus norvegicus;* cynomolgus monkey, *Macaca fascicularis;* the hamadryas baboon, *Papio hamadryas;* the Guinea baboon, *Papio papio;* the olive baboon, *Papio anubis; the yellow baboon, Papio cynocephalus; the* Chacma baboon, *Papio ursinus, Callithrixjacchus, Saguinus oedipus,* and *Saimiri sciureus.*

In some examples, the target cell protein can be a protein selectively expressed on an infected cell. For example, in the case of a hepatitis B virus (HBV) or a hepatits C virus (HCV) infection, the target cell protein can be an envelope protein of HBV or HCV that is expressed on the surface of an infected cell. In other embodiments, the target cell protein can be gp120 encoded by human immunodeficiency virus (HIV) on HIV-infected cells.

In other aspects, a target cell can be a cell that mediates an autoimmune or inflammatory disease. For example, human eosinophils in asthma can be target cells, in which case, EGF-like module-containing mucin-like hormone receptor (EMR1), for example, can be a target cell protein. Alternatively, excess human B cells in a systemic lupus erythematosus patient can be target cells, in which case CD19 or CD20, for example, can be a target cell protein. In other autoimmune conditions, excess human Th2 T cells can be target cells, in which case CCR4 can, for example, be a target cell protein. Similarly, a target cell can be a fibrotic cell that mediates a disease such as atherosclerosis, chronic obstructive pulmonary disease (COPD), cirrhosis, scleroderma, kidney transplant fibrosis, kidney allograft nephropathy, or a pulmonary fibrosis, including idiopathic pulmonary fibrosis and/or idiotypic pulmonary hypertension. For such fibrotic conditions, fibroblast activation protein alpha (FAP alpha) can, for example, be a target cell protein.

### Target Cell Cytolysis Assays

In the Examples below, an assay for determining whether a Bi-Fc antibody as described herein can induce cytolysis of a target cell by an immune effector cell *in vitro* is described. In this assay, the immune effector cell is a T cell. The following very similar assay can be used where the immune effector cells are NK cells.

A target cell line expressing the target cell protein of interest can be labeled with 2 $\mu$M carboxyfluorescein succinimidyl ester (CFSE) for 15 minutes at 37 °C and then washed. An appropriate number of labeled target cells can then be incubated in one or more 96 well flat bottom culture plates for 40 minutes at 4 °C, with or without a bispecific protein, a control protein, or no added protein at varying concentrations. NK cells isolated from healthy human donors can be isolated using the Miltenyi NK Cell Isolation Kit II (Miltenyi Biotec, Auburn, CA) and then added to the target cells at an Effector:Target ratio of 10:1. The NK cells, which are the immune effector cells in this assay, can be used immediately post-isolation or after overnight culture at 37 °C. Plates containing tumor target cells, bispecific proteins, and immune effector cells can be cultured for 18-24 hours at 37 °C with 5% CO2. Appropriate control wells can also be set up. After the 18-24 hour assay period, all cells can be removed from the wells. A volume of a 7-AAD solution equal to the volume of the content of the wells can be added to each sample. Samples can then assayed to determine the percentage of live versus dead target cells via flow cytometry as described in the Examples below.

### Therapeutic Uses and Compositions

Any references in the description to methods of treatment refer to the compounds, pharmaceutical compositions and medicaments of the present invention for use in a method for treatment of the human (or animal) body by therapy (or for diagnosis)

Bi-Fc's can be used to treat a wide variety of conditions including, for example, various forms of cancer, infections, autoimmune or inflammatory conditions, and/or fibrotic conditions.

Provided herein are pharmaceutical compositions comprising Bi-Fc's. Such pharmaceutical compositions comprise a therapeutically effective amount of a Bi-Fc plus one or more additional components such as a physiologically acceptable carrier, excipient, or diluent. Such additional components can include buffers, carbohydrates, polyols, amino acids, chelating agents, stabilizers, and/or preservatives, among many possibilities.

In some embodiments, a Bi-Fc can be used to treat cell proliferative diseases, including cancer, which involve the unregulated and/or inappropriate proliferation of cells, sometimes accompanied by destruction of adjacent tissue and growth of new blood vessels, which can allow invasion of cancer cells into new areas, *i.e.* metastasis. Included within conditions treatable with a Bi-Fc are non-malignant conditions that involve inappropriate cell growth, including colorectal polyps, cerebral ischemia, gross cystic disease, polycystic kidney disease, benign prostatic hyperplasia, and endometriosis. A Bi-Fc can be used to treat a hematologic or solid tumor malignancy. More specifically, cell proliferative diseases that can be treated using a Bi-Fc are, for example, cancers including mesotheliomas, squamous cell carcinomas, myelomas, osteosarcomas, glioblastomas, gliomas, carcinomas, adenocarcinomas, melanomas, sarcomas, acute and chronic leukemias, lymphomas, and meningiomas, Hodgkin's disease, Sézary syndrome, multiple myeloma, and lung, non-small cell lung, small cell lung, laryngeal, breast, head and neck, bladder, ovarian, skin, prostate, cervical, vaginal, gastric, renal cell, kidney, pancreatic, colorectal, endometrial, esophageal, hepatobiliary, bone, skin, and hematologic cancers, as well as cancers of the nasal cavity and paranasal sinuses, the nasopharynx, the oral cavity, the oropharynx, the larynx, the hypolarynx, the salivary glands, the mediastinum, the stomach, the small intestine, the colon, the rectum and anal region, the ureter, the urethra, the penis, the testis, the vulva, the endocrine system, the central nervous system, and plasma cells.

Among the texts providing guidance for cancer therapy is Cancer, Principles and Practice of Oncology, 4th Edition, DeVita et al, Eds. J. B. Lippincott Co., Philadelphia, PA (1993). An appropriate therapeutic approach is chosen according to the particular type of cancer, and other factors such as the general condition of the patient, as is recognized in the pertinent field. A Bi-Fc can be used by itself or can be added to a therapy regimen using other anti-neoplastic agents in treating a cancer patient.

In some embodiments, a Bi-Fc can be administered concurrently with, before, or after a variety of drugs and treatments widely employed in cancer treatment such as, for example, chemotherapeutic agents, non-chemotherapeutic, anti-neoplastic agents, and/or radiation. For example, chemotherapy and/or radiation can occur before, during, and/or after any of the treatments described herein. Examples of chemotherapeutic agents are discussed above and include, but are not limited to, cisplatin, taxol, etoposide, mitoxantrone (Novantrone®), actinomycin D, cycloheximide, camptothecin (or water soluble derivatives thereof), methotrexate, mitomycin (e.g., mitomycin C), dacarbazine (DTIC), antineoplastic antibiotics such as adriamycin (doxorubicin) and daunomycin, and all the chemotherapeutic agents mentioned above.

A Bi-Fc can also be used to treat infectious disease, for example a chronic HBV infection, an HCV infection, an HIV infection, an Epstein-Barr virus (EBV) infection, or a cytomegalovirus (CMV) infection, among many others. A Bi-Fc can be administered by itself or can be administered concurrently with, before, or after administration of other therapeutics used to treat such infectious diseases.

A Bi-Fc can find further use in other kinds of conditions where it is beneficial to deplete certain cell types. For example, depletion of human eosinophils in asthma, excess human B cells in systemic lupus erythematosus, excess human Th2 T cells in autoimmune conditions, or pathogen-infected cells in infectious diseases can be beneficial. In a fibrotic condition, it can be useful to deplete cells forming fibrotic tissue. A Bi-Fc can be administered by itself or can be administered concurrently with, before, or after administration of other therapeutics used to treat such diseases.

Therapeutically effective doses of a Bi-Fc can be administered. The amount of Bi-Fc that constitutes a therapeutically dose may vary with the indication treated, the weight of the patient, the calculated skin surface area of the patient. Dosing of a Bi-Fc can be adjusted to achieve the desired effects. In many cases, repeated dosing may be required. For example, a Bi-Fc can be dosed twice per week, once per week, once every two, three, four, five, six, seven, eight, nine, or ten weeks, or once every two, three, four, five, or six months. The amount of a Bi-Fc administered on each day can be from about 0.0036 mg to about 450 mg. Alternatively, the dose can calibrated according to the estimated skin surface of a patient, and each dose can be from about 0.002 mg/m$^2$ to about 250 mg/m$^2$. In another alternative, the dose can be calibrated according to a patient's weight, and each dose can be from about 0. 000051 mg/kg to about 6.4 mg/kg.

A Bi-Fc, or a pharmaceutical composition containing such a molecule, can be administered by any feasible method. Protein therapeutics will ordinarily be administered by a parenteral route, for example by injection, since oral administration, in the absence of some special formulation or circumstance, would lead to fragmentation and/or hydrolysis of the protein in the acid environment of the stomach. Subcutaneous, intramuscular, intravenous, intraarterial, intralesional, or peritoneal bolus injection are possible routes of administration. A Bi-Fc can also be administered via infusion, for example intravenous or subcutaneous infusion. Topical administration is also possible, especially for diseases involving the skin. Alternatively, a Bi-Fc can be administered through contact with a mucus membrane, for example by intra-nasal, sublingual, vaginal, or rectal administration or administration as an inhalant. Alternatively, certain appropriate pharmaceutical compositions comprising a Bi-Fc can be administered orally.

**Examples**

***Example 1: Construction of anti-HER2 CD3ε and anti-FOLR1/CD3ε Bi-Fc molecules and single chain bispecific molecules***

Bi-Fc molecules were generated using methods essentially described previously. Löffler et al. (2000), Blood95(6): 2098-2103. In more detail, a construct encoding a heterodimeric anti-HER2/CD3ε Bi-Fc was made as follows. DNA fragments encoding the VH region (SEQ ID NO:5) and the VL region (SEQ ID NO:6) of an anti-HER2 IgG antibody and the VH region (SEQ ID NO:7) and VL region (SEQ ID NO:8) of anti-human CD3ε IgG antibody were amplified by PCR using forward and reverse primers and spliced together with flexible linkers. The resulting DNA fragment, which encodes a linear fusion DNA encoding two scFv's joined by a linker is referred to herein as the single chain anti-HER2/CD3ε (SEQ ID NO:9). This construct was subcloned into a mammalian expression vector for antibody production.

A heterodimeric anti-HER2/CD3ε Bi-Fc (SEQ ID NO:10) was constructed by fusing DNA encoding the single chain anti-HER2/CD3ε to DNA encoding one of the two chains of an engineered human IgG1 Fc region. Specifically, DNA encoding an Fc polypeptide chain containing two positively charged mutations (D356K/D399K, EU numbering) plus alterations that inhibit FcγR binding (L234A and L235A) was fused to the DNA encoding the single chain anti-HER2/CD3ε at the 3' end. The amino acid sequence of this anti-HER/CD3ε Bi-Fc and the nucleic acid sequence encoding it are shown in SEQ ID NO:10 and 11, respectively. The second polypeptide chain that was part of the anti-HER2/CD3ε Bi-Fc was a human IgG1 Fc polypeptide chain containing two negatively charged mutations (K392D/K409D, EU numbering) plus L234A and L235A, as shown in SEQ ID NO:12. DNA encoding this polypeptide (SEQ ID NO:13) was amplified and inserted into an appropriate vector for expression. Using similar methods, a single chain anti-FOLR1/CD3ε (SEQ ID NO: 14) and a heterodimeric anti-FOLR1/CD3ε Bi-Fc (SEQ ID NO:15) were constructed by replacing DNA encoding the anti-HER2 scFv fragment with DNA encoding an scFv fragment derived from an anti-human FOLR1 IgG antibody.

All single chain and heterodimeric Bi-Fc molecules described above were produced by transient transfection in human HEK 293-6E cells. The culture media was harvested after 6 days. The single chain anti-HER2/CD3ε and anti-FOLR1/CD3ε molecules were purified by nickel HISTRAP® (GE Healthcare Bio-Sciences, L.L.C., Uppsala, Sweden) column chromatography and eluted with a 25 to 300 mM imidizole gradient. The elution pools were further purified by size exchange chromatography (SEC) using a preparative SUPERDEX® 200 (GE Healthcare Bio-Sciences, L.L.C., Uppsala, Sweden) column, concentrated to > 1 mg/mL, and stored at -70 °C. Heterodimeric anti-HER2/CD3ε Bi-Fc and anti-FOLR1/CD3ε Bi-Fc molecules were purified using MABSELECT SURE™ (GE Healthcare Bio-Sciences, L.L.C., Uppsala, Sweden) affinity chromatography, eluting with 50 mM citrate, 1M L-Arginine, pH 3.5. The eluate was buffer-exchanged into formulation buffer by a preparative SEC with 10 mM potassium phosphate, 161 mM L-Arginine, pH 7.6 or with a solution containing acetate and sucrose with 150 mM NaCl, 161 mM L-Arginine, pH 5.2

### Example 2: Testing BiTE:Fc molecules for binding to target cells and immune effector cells

Binding of the heterodimeric anti-HER2/CD3ε Bi-Fc and single chain anti-HER2/CD3ε to T cells expressing CD3 and JIMT-1 cells expressing HER2 was assessed as follows. Human pan-T cells (purified using Pan T Cell Isolation Kit II, human, Miltenyi Biotec, Auburn, CA) or purified JIMT-1 cells were incubated for 16 hrs at 4 °C in the absence or presence of 10 μg/mL of the heterodimeric anti-HER2/CD3ε Bi-Fc or the single chain anti-HER2/CD3ε. Cell binding of the heterodimeric anti-HER2/CD3ε Bi-Fc was detected using an allophycocyanin (APC)-labeled anti-human Fc secondary antibody. The single chain anti-HER2/CD3ε, which includes a FLAG® tag, was detected using a mouse anti-FLAG® antibody followed by an APC-labeled mouse Ig-specific antibody.

In the fluorescence-activated cell sorting (FACS) histograms shown in Figure 2, the unfilled profiles represent data from cells in the absence of one of the bispecific molecules, and the solidly filled profiles represent data from cells in the presence of one of the bispecific molecules, as indicated in Figure 2 and its description. These results indicate that the heterodimeric anti-HER2/CD3ε Bi-Fc, as well as the single chain anti-HER2/CD3ε, binds to both T cells (expressing CD3ε) and to JIMT-1 cells expressing HER2.

### Example 3: Lysis of tumor cell lines in the presence of Bi-Fc's and T cells

The heterodimeric anti-HER2/CD3ε and anti-FOLR1/CD3ε Bi-Fc's and the single chain anti-HER2/CD3ε and anti-FOLR1/CD3ε molecules described above were assayed to determine their activity in a T cell-dependent cell cytolysis (TDCC) assay using tumor cells expressing HER2 or FOLR1 as target cells. Briefly, pan T cells were isolated from healthy human donors using the Pan T Cell Isolation Kit II, human (Miltenyi Biotec, Auburn, CA). The T cells were incubated with CFSE-labeled tumor target cells at a ratio of 10:1 in the presence or absence of the heterodimeric anti-HER2/CD3ε or anti- FOLR1/CD3ε Bi-Fc or the single chain anti-HER2/CD3ε or anti-FOR1/CD3ε described in Example 1 at the varying concentrations as indicated in Figures 3 and 4. As a control, some samples contained T cells and tumor target cells, but no Bi-Fc or single chain molecule.

The target cells for the anti-FOLR1/CD3ε heterodimeric Bi-Fc and single chain molecule were either Cal-51 cells (expressing about 148,000 molecules of FOLR1 per cell), T47D cells (expressing about 101,000 molecules of FOLR1 per cell), or the control cell line BT474 (which did not express detectable levels of FOLR1).

The target cells for the anti-HER2/ CD3ε heterodimeric Bi-Fc and single chain molecules were JIMT-1 cells (expressing about 181,000 molecules of HER2 per cell), T47D cells (expressing about 61,000 molecules of HER2 per cell), or the control cell line SHP77 (which did not express detectable amounts of HER2).

After 39 to 48 hours of incubation, cells were harvested, and the percent of tumor cell lysis was monitored by uptake of 7-amino-actinomycin D (7-AAD), which stains double-stranded nucleic acids. Intact cells exclude 7-AAD, whereas 7-AAD can penetrate the membranes of dead or dying cells and stain the double-stranded nucleic acids inside these cells. Percent specific lysis was calculated according to the following formula:

$$\% \ specific \ lysis = [\% \ tumor \ lysis \ with \ Bi\text{-}Fc - \% \ tumor \ cell \ lysis \ without \ bispecific \ /$$

$$\% \ of \ total \ cell \ lysis - \% \ tumor \ cell \ lysis \ without \ bispecific] \ x \ 100$$

To determine percent total cell lysis, samples containing immune effector and labeled target cells without a Bi-Fc or single chain molecule were lysed with cold 80% methanol.

Results for the anti-FOLR1/CD3ε heterodimeric Bi-Fc and single chain molecule are shown in Figure 3. Both the anti-FOLR1/CD3ε heterodimeric Bi-Fc and single chain molecule exhibited dose dependent lysis of both the Cal-51 and the T47D target cells. The $EC_{50}$ for each of these molecules in each of these cell lines is shown in Table 3 below.

### Table 3: EC$_{50}$ of Bi-Fc and single chain anti-FOLR1/CD3ε molecules

| Molecule | EC$_{50}$ (pM) | | |
|---|---|---|---|
| | Cell Line | | |
| | Cal-51 | T47D | B7474 |
| Anti-FOLR1/CD3ε Bi-Fc | 1.27 | 1.35 | NA* |
| Anti-FOLR1/CD3ε single chain | 0.087 | 0.19 | NA* |
| *NA means that there was little or no cell lysis detected. | | | |

These data indicate that both the anti-FOLR1/CD3ε heterodimeric Bi-Fc and single chain molecule can mediate lysis of cells expressing FOLR1 in the presence of T cells, but do not mediate lysis of cells not expressing FOLR1. The EC$_{50}$'s of the Bi-Fc are about 7 to 15 fold higher than those of the single chain molecule, but they are still in the pM range. Thus, both the Bi-Fc and the single chain molecule are highly potent in this assay.

Results for the anti-HER2/CD3ε heterodimeric Bi-Fc and single chain molecule are shown in Figure 4. Both the anti-HER2/CD3ε heterodimeric Bi-Fc and single chain molecule exhibited dose dependent lysis of both the JIMT-1 and the T47D target cells, but no lysis of the control SHP77 cell line (which does not express HER2). The EC$_{50}$ for each of these molecules in each of these cell lines is shown in Table 4 below.

### Table 4: EC$_{50}$ of Bi-Fc and single chain anti-HER2/CD3ε molecules

| Molecule | EC$_{50}$ (pM) | | |
|---|---|---|---|
| | Cell Line | | |
| | JIMT-1 | T47D | SHP77 |
| Anti-HER2/CD3ε Bi-Fc | 11.52 | 1.03 | NA* |
| Anti-HER2/CD3ε single chain | 1.12 | 0.12 | NA* |
| *NA means that there was little or no cell lysis detected. | | | |

These data indicate that both the anti-HER2/CD3ε heterodimeric Bi-Fc and single chain molecules can mediate lysis of cells expressing HER2 in the presence of T cells, but do not mediate lysis of cells not expressing HER2. The EC$_{50}$'s of the Bi-Fc's are about 8.6 to 10.3 fold higher than those of the single chain molecule.

### *Example 4: Release of cytokines by T cells in the presence of Bi-Fc and target cells*

The anti-HER2/CD3ε single chain and heterodimeric Bi-Fc and the anti-FOLR1/CD3ε single chain and heterodimeric Bi-Fc described above were assayed to determine whether they could stimulate the production of inflammatory cytokines by T cells. Briefly, twenty four hour cell culture supernatants from the TDCC assays like those described in Example 3 were assessed for cytokine concentrations using the Human TH1/TH2 7-Plex and Human Proinflammatory 1 4-Plex ultra Sensitive Kits from Meso Scale Diagnostics, L.L.C. Assays were performed according to the manufacturer's directions.

These results are shown in Figures 5A, 5B, 6A, and 6B. As shown in Figures 5A and 5B, the T cells secreted cytokines in the presence of the anti-FOLR1/CD3ε heterodimeric Bi-Fc or single chain in the presence of cells expressing FOLR1 (T47D, left panels), but not in the presence of cells that did not express FOLR1 (BT474, right panels). Similarly, as shown in Figure 6A and 6B, T cells secreted cytokines in the presence of the anti-HER2/CD3ε heterodimeric Bi-Fc or single chain in the presence of cells expressing HER2 (JIMT-1, left panels), but not in the presence of cells that did not express HER2 (SHP77). Thus, the secretion of interferon gamma (IFN-γ), tumor necrosis factor alpha (TNF-α), interleukin-10 (IL-10), interleukin-2 (IL-2), and interleukin-13 (IL-13) by T cells in the presence of a heterodimeric Bi-Fc or a single chain molecule was dependent on the presence of cells expressing a target cell protein. Hence, activation of the T cells by the Bi-Fc's and single chain molecules was specific in the sense that it occurred only in the presence of target cells expressing a target cell protein.

In addition, the Bi-Fc's had very potent activity in the assay, exhibiting EC$_{50}$'s in the pM range as shown in the table below.

**Table 5: EC$_{50}$'s for eliciting cytokine secretion**

| Cytokine | EC$_{50}$ (pM) | | | |
|---|---|---|---|---|
| | JIMT-1 cells | | T47D cells | |
| | anti-HER2/CD3ε Bi-Fc | anti-HER2/CD3ε single chain | anti-FOLR1/CD3ε Bi-Fc | anti-FOLR1/CD3ε single chain |
| **IFN-γ** | 32.9 | 2.1 | 48.6 | 7.5 |
| TNF-α | 19.5 | 1.8 | 41.2 | 8.8 |
| IL-10 | 9.6 | 0.9 | 110.1 | 18.4 |
| IL-2 | 22.3 | 1.2 | 67.3 | 12.9 |
| IL-13 | 16.4 | 1.8 | 126.9 | 28.1 |

Thus, even though the heterodimeric Bi-Fc is almost twice the size of the single chain molecule, it remains a very potent activator of cytokine secretion by T cells in the presence, but not in the absence of, target cells. In addition, the heterodimeric Bi-Fc and the single chain molecule show a very similar cytokine profile. The EC$_{50}$'s for cytokine secretion induced by the anti-HER2/CD3ε heterodimeric Bi-Fc were about 9 to 19 fold higher than those induced by the anti-HER2/CD3ε single chain. The EC$_{50}$'s for cytokine secretion induced by the anti-FOLR1/CD3ε Bi-Fc were about 4.5 to 6.5 fold higher than those induced the anti-FOLR1/CD3ε single chain.

**Example 5: Upregulation of T cell activation markers in the presence of Bi-Fc and target cells**

The following experiment was done to determine whether a heterodimeric Bi-Fc could activate T cells in the presence of peripheral blood mononuclear cells (PBMCs) and in the presence or absence of target cells. PBMCs from healthy donors were purified on a FICOLL™ gradient from human leukocytes purchased from Biological Specialty Corporation of Colmar, Pennsylvania. These PBMCs were incubated with the heterodimeric anti-HER2/ CD3ε Bi-Fc or the single chain anti-HER2/CD3ε bispecific molecule described above in the presence or absence of JIMT-1 cells at a 10:1 ratio. After 48 hours of incubation, non-adherent cells were removed from the wells and divided into two equal samples. All samples were stained with fluorescein isothiocynate (FITC)-conjugated anti-human CD3 antibody plus an allophycocyanin (APC)-conjugated anti-CD25 or anti-CD69 antibody. CD25 and CD69 are markers of activation of T cells.

Up-regulation of CD25 and CD69 (Figure 7) activation markers by CD3[+] peripheral T cells was observed with the heterodimeric anti-HER2/CD3ε Bi-Fc and the anti-HER2/CD3ε single chain in the presence, but not in the absence, of HER2-expressing JIMT-1 tumor target cells. These observations suggest that T cell activation by the Bi-Fc is dependent on the presence of tumor target cells expressing the target cell protein. An alternate potential path to T cell activation, that is, cross-linking by FcγR's in the presence of a Bi-Fc such as the anti-HER2/CD3ε Bi-Fc, likely is not responsible for the observed effects because the Fc region of the anti-HER2/CD3ε Bi-Fc contains alterations that inhibit binding to FcgRs and because activation of T cells is not observed in the absence of target cells expressing HER2.

**Example 6: Pharmacokinetic properties of Bi-Fc's**

In the following experiment, the single dose pharmacokinetic profiles of a heterodimeric anti-HER2/CD3ε Bi-Fc (comprising the amino acid sequences of SEQ ID NOs:10 and 12) and an anti-HER2/CD3ε single chain (comprising the amino acid sequence of SEQ ID NO:9) was assessed by intravenous and subcutaneous bolus administration in male NOD.SCID mice (Harlan, Livermore, CA). These test molecules were injected as a bolus at 1 mg/kg intravenously via the lateral tail vein in some mice or subcutaneously under the skin over the shoulders in others. Serial bleeds of approximately 0.1 mL of whole blood were collected at at each time point via retroorbital sinus puncture. Upon clotting of whole blood the samples were processed to obtain serum (-0.040 mL per sample). Serum samples were analyzed by immunoassay using the technology Gyros AB (Warren, NJ) to determine the serum concentrations of the anti-HER2/CD3ε single chain and Bi-Fc. Serum samples were collected at 0, 0.5, 2, 8, 24, 72, 120, 168, 240, 312, 384, and 480 hours. Serum samples were maintained at -70°C (±10°C) prior to analysis. Pharmacokinetic parameters were estimated from serum concentrations using non-compartmental analysis using Phoenix® 6.3 software (Pharsight, Sunnyvale, CA).

The single dose pharmacokinetic profiles of the heterodimeric Bi-Fc and the single chain molecule are shown in Figure 8. The Bi-Fc showed an extended serum half life (219 hours) compared to the single chain molecule, which was rapidly eliminated and had a half life of only 5 hours. Exposure of the Bi-Fc was characterized by an area under the curve (AUC) of 524 hr*μg/mL, as compared to 19 hr*ug/mL for the single chain molecule. The subcutaneous bioavailability of the Bi-Fc was 83%, while that of the single chain molecule was 29%. Thus, the heterodimeric Bi-Fc showed favorable single dose pharmacokinetic properties as compared to the single chain molecule.

### Example 7: Construction of a monomeric anti-CD33/CD3ε Bi-Fc

A monomeric anti-CD33/CD3ε Bi-Fc was constructed, the overall structure of which is represented by the second diagram from the left in Figure 1. Monomeric Fc polypeptide chains, containing specific alterations relative to a naturally occurring Fc polypeptide chain, are described in US Patent Application Publication 2012/0244578. Starting with a vector encoding a human monomeric IgG1 Fc polypeptide chain (which lacked a hinge region, *i.e.,* started at position 231 in the EU numbering system, and had a carboxyterminal hexa-histidine tag plus the alterations Y349T, K392D, and K409D), further mutations were introduced using Agilent's Quikchange Site-Directed Mutagenesis Kit (catalog number 200518-5) that specified the alteration N297G. Thus, the final Fc polypeptide chain began with the alanine at position 231 and continued through to the lysine at position 447, which was followed by a hexa-histidine tag (SEQ ID NO:92). It contained the following alterations: N297G, Y349T, K392D, and K409D.

DNA encoding an anti-CD33/CD3ε single chain molecule was amplified by PCR from a second vector encoding a single chain molecule containing heavy and light chain variable regions binding to CD33 followed by heavy and light chain variable regions binding to CD3ε. The amino acid sequence of this anti-CD33/CD3ε single chain is given in SEQ ID NO:33, and it is described in detail in US Patent Application 2012/244162. This DNA was attached to DNA encoding the altered Fc polypeptide chain described above using splice overhang extension by polymerase chain reaction (SOE by PCR). *See, e.g.,* Warrens et al. (1997), Gene 186: 29-35.

The amino acid sequence of the resulting monomeric anti-CD33/CD3ε Bi-Fc is provided in SEQ NO NO:34, and the nucleic acid sequence encoding it is provided in SEQ ID NO:35. DNA encoding the monomeric anti-CD33/CD3ε Bi-Fc was introduced into mammalian cells, which were cultured under conditions suitable for expression. The protein was recovered from the cell supernatant.

### Example 8: Binding of anti-CD33/CD3ε Bi-Fc to cells expressing CD3ε or CD33

Binding of the monomeric anti-CD33/CD3ε Bi-Fc and the anti-CD33/CD3ε single chain to cells expressing CD3ε, CD33, or neither was assessed. Molm-13 cells (expressing CD33), Namalwa cells (expressing neither CD33 nor CD3ε), purified human pan-T cells (expressing CD3ε), human PBMCs (expressing CD3ε), and cynomologus PBMCs (expressing CD3ε) were tested. Cells were incubated for 2 hours at 4 °C in the absence or presence of the bispecific molecules. Cell binding of the monomeric anti-CD33/CD3ε Bi-Fc and the anti-CD33/CD3ε single chain were then detected by incubating the cells with a mouse antibody that binds to the CD3-binding regions of the bispecific molecules at 10 μg/mL overnight at 4 °C, followed by an APC-labeled anti-mouse Fc secondary antibody (Jackson 115-136-071) at 5 μg/mL for 2 hrs at 4 °C. The cells were analyzed by FACS, and the mean fluorescent intensity (MFI) of the signal was determined.

Figure 9 shows the MFI detected for the various cell types in the presence of various concentrations of the bispecific molecules as follows: panel A, Molm-13 cells (expressing CD33); panel B, Namalwa cells (expressing neither CD33 nor CD3ε); panel C, human pan-T cells (expressing CD3ε); panel D, human PBMCs (expressing CD3ε); and panel E, cynomologus PBMCs (expressing CD3ε). The results demonstrate that these two bispecific molecules bind to these cells types in a similar manner, indicating that the addition of an Fc polypeptide chain to the anti-CD33/CD3ε single chain did not detectably affect its ability to bind to CD33 and CD3ε as measured by this assay.

### Example 9: Lysis of CD33-expressing tumor cells in the presence of a monomeric anti-CD33/CD3ε Bi-Fc

The following experiments were done to determine whether the monomeric anti-CD33/CD3ε Bi-Fc described above could induce lysis of CD33-expressing tumor cells in the presence of peripheral blood mononuclear cells (PBMCs). PBMC effector cells from cynomologus monkeys were obtained from SNBL USA (a subsidiary of Shin Nippon Biomedical Laboratories). In this preparation of PBMCs, 61% were CD3$^+$ T cells (data not shown). These PBMCs were incubated with carboxyfluorescein succinimidyl ester (CFSE)-labeled tumor target cells at a ratio of 10:1 in the presence and absence of the monomeric anti-CD33/CD3ε Bi-Fc or the anti-CD33/CD3ε single chain at the concentrations indicated in Figure 10. Following 40-48 hours of incubation at 37 °C, cells were harvested, and live and dead tumor cells were monitored by 7AAD uptake using flow cytometry. Percent specific lysis was calculated according to the following formula:

$$\% \text{ specific lysis} = 1 - (\text{live cell counts (with bispecific)}/\text{live cell counts (without bispecific)}) \times 100$$

These results are shown in Figure 10. Data shown in Figure 10, panel A indicate that Molm-13 cells, which express about 33,000 molecules of CD33 per cell, were lysed with both the monomeric anti-CD33/CD3ε Bi-Fc and the anti-CD33/CD3ε single chain. The concentrations for half maximal lysis ($EC_{50}$'s) were in the pM range, that is. 1.45 pM and 0.96 pM for the monomeric anti-CD33/CD3ε Bi-Fc and the anti-CD33/CD3ε single chain, respectively. Thus, the $EC_{50}$ of the monomeric Bi-Fc was less than two fold higher than that of the single chain molecule. Data shown in Figure 10, panel B indicate that there was no lysis of Namalwa cells, which do not express detectable levels of CD33. These observations suggest that the monomeric anti-CD33/CD3ε Bi-Fc is a highly specific and potent reagent capable of inducing tumor cell lysis by cynomologus monkey PBMCs.

In a second experiment, pan T effector cells isolated from human healthy donors were incubated with CFSE-labeled Molm-13 or Namalwa cells at a ratio of 10:1 in the presence and absence of the monomeric anti-CD33/CD3ε Bi-Fc or the anti-CD33/CD3ε single chain at the concentrations indicated in Figure 11. Following 40-48 hours of incubation at 37 °C, cells were harvested, and live and dead tumor cells were monitored by 7AAD uptake using flow cytometry. Percent specific lysis was calculated according to the formula given above in this example. Results are shown in Figure 11.

Specific lysis of Molm-13 cells was observed with both the monomeric anti-CD33/CD3ε Bi-Fc and the anti-CD33/CD3ε single chain. Figure 11, panel A. The $EC_{50}$'s were in the pM range, that is, 0.65 pM and 0.12 pM for the monomeric anti-CD33/CD3ε Bi-Fc and the anti-CD33/CD3ε single chain, respectively. Hence, the $EC_{50}$ for the Bi-Fc is 5 to 6 fold higher than that of the single chain molecule. There was no lysis of Namalwa cells detected with either bispecific molecule except for a small amount of lysis detected at the highest concentration of the monomeric anti-CD33/CD3ε Bi-Fc tested. Figure 11, panel. B. No lysis of Molm-13 cells occurred in the absence of T cells (data not shown). These observations suggest that the monomeric anti-CD33/CD3ε Bi-Fc, like the heterodimeric Bi-Fc's described above, is a highly specific and potent reagent capable of inducing tumor cell lysis by T cells.

***Example 10: Lysis of CD33-expressing tumor cells and release of interferon gamma from PBMCs in the presence of a monomeric anti-CD33/CD3ε Bi-Fc***

In another experiment, PBMCs isolated from healthy human donors or cynomologus monkeys (obtained from SNBL USA) were tested for their ability to lyse tumor target cells expressing CD33. In these preparations, the PBMCs were 42% CD3$^+$ T cells (human) and 30% CD3$^+$ T cells (cynomolgus monkey). PBMCs were incubated at 37°C with CFSE-labeled tumor target cells at a ratio of 5:1 in the presence and absence of the monomeric anti-CD33/CD3ε Bi-Fc or the anti-CD33/CD3ε single chain at the concentrations indicated in Figure 12. Following 67 hours of incubation at 37 °C, cells were harvested, and live and dead tumor cells were monitored by 7AAD uptake using flow cytometry. Percent specific lysis was calculated according to the formula described above. Results are shown in Figure 12.

Specific lysis of Molm-13 cells was observed with both the monomeric anti-CD33/CD3ε Bi-Fc and the anti-CD33/CD3ε single chain using human PBMCs (Figure 12, panel A) or cynomologus PBMCs (Figure 12, panel B). The concentrations for half maximal lysis ($EC_{50}$'s) were in the pM range, as shown in Table 6 below.

Table 6: $EC_{50}$'s for lysis of Molm-13 cells by PBMCs in the presence of anti-CD33/CD3ε bispecific molecules

| | $EC_{50}$ (pM) for lysis of Molm-13 cells | |
|---|---|---|
| Anti-CD33/CD3ε bispecific | Human PBMCs | Cynomolgus monkey PBMCs |
| Monomeric Bi-Fc | 0.68 | 3.55 |
| Single chain | 0.14 | 1.39 |

Molm-13 cells were not lysed in the presence of either of the bispecifics in the absence of T cells (data not shown). These data show that the $EC_{50}$'s for the monomeric Bi-Fc are in the sub-picomolar to low picomolar range and are very close to the $EC_{50}$'s of the single chain molecule using both human and cynomolgus monkey PBMCs as the effector cells.

Twenty four hour cell culture supernatants from the cell lysis assays described immediately above were assessed for cytokine concentrations using the commercially available BD OptEIA™ Human IFN-γ ELISA Kit II (BD Biosciences) and the Monkey Interferon gamma ELISA Kit (Cell Sciences). The assays were performed according to the manufacturer's directions. In the presence of Molm-13 cells, IFN-γ was released from human (Figure 13, panel A) and cynomologus monkey (Figure 13, panel B) PBMCs treated with the monomeric anti-CD33/CD3ε Bi-Fc or the anti-CD33/CD3ε single chain. These results suggest that the monomeric anti-CD33/CD3ε Bi-Fc, like the anti-CD33/CD3ε single chain, is a highly specific and potent reagent capable of mediating release of interferon gamma from PBMCs.

***Example 11: Induction of T cell proliferation, CD25 expression, and cytokine release by monomeric anti-CD33/CD3ε Bi-Fc***

Pan T effector cells isolated from human healthy donors were labeled with CFSE and incubated with tumor target cells, either Molm-13 or Namalwa cells, at a ratio of 10:1 in the presence and absence of the monomeric anti-CD33/CD3ε Bi-Fc or the anti-CD33/CD3ε single chain at the concentrations indicated in Figures 14 and 15. Following 72 hours of incubation at 37 °C, cells were harvested, and T cell proliferation and expression of CD25, a marker for activation, were analyzed by flow cytometry.

Proliferation was assessed by monitoring the numbers of cells with a decreased fluorescent signal from the CFSE dye. With each cell division following labeling of the T cells with CFSE, the intensity of the fluorescent signal from the CFSE for each individual dividing cell decreases. The percent proliferating T cells was determined by gating on CFSE-labeled T cells and comparing the number of mitotic T cells, *i.e.,* cells having a diminished fluorescent signal, with the total number of T cells. The percent CD25 positive T cells was determined by staining the cells in the co-culture with an allophycocyanin (APC)-labeled anti-human CD25 antibody and measuring the APC levels of CFSE-labeled cells using two-color flow cytometry.

Proliferation of T cells was observed in the presence of the CD33-expressing tumor cell line, Molm-13, plus either the monomeric anti-CD33/CD3ε Bi-Fc or the anti-CD33/CD3ε single chain. Figure 14, panel A. The $EC_{50}$'s were in the single digit pM range, that is, 4.27 pM in the presence of the monomeric anti-CD33/CD3ε Bi-Fc and 1.09 pM in the presence of the anti-CD33/CD3ε single chain. No proliferation of T cells was observed in the presence Namalwa cells, which do not express detectable levels of CD33. Figure 14, panel A.

T cells in the presence of Molm-13 cells and either the monomeric anti-CD33/CD3ε Bi-Fc or the anti-CD33/CD3ε single chain expressed the activation marker, CD25. Figure 14, panel B. T cells in the presence of Namalwa cells (which do not express detectable levels of CD33) and either of the bispecifics did not express CD25. Figure 14, panel B. These observations suggest that the monomeric anti-CD33/CD3ε Bi-Fc is capable of specifically inducing T activation and proliferation.

Twenty four hour cell culture supernatants from assays described immediately above were assessed for cytokine concentrations using the commercially available Human TH1/TH2 7-Plex and Human Proinflammatory 1 4-Plex Ultra-Sensitive Kits from Meso Scale Diagnostics, LLC. The assays were performed according to the manufacturer's directions. Results are shown in Figure 15.

In the presence of the CD33-expressing Molm-13 tumor cell line, interferon gamma (IFN-γ), tumor necrosis factor alpha (TNF-α), interleukin-10 (IL-10), interleukin-2 (IL-2), and interleukin-13 (IL-13) were released from T cells treated with the monomeric anti-CD33/CD3ε Bi-Fc or the anti-CD33/CD3ε single chain as shown in Figure 15, panels A, B, C, D, and E, respectively. The highest cytokine concentrations were seen with IFN-γ, TNF-α, IL-2 and IL-10 (greater than 400 pg/mL). Moderate levels of IL-13 were also observed. Cytokine secretion was not observed in the presence of the CD33-negative cell line, Namalwa. In Table 7 below, the $EC_{50}$'s for the production of the various cytokines by Molm-13 cells in the presence of either the monomeric anti-CD33/CD3ε Bi-Fc or the single chain anti-CD33/CD3ε are shown.

**Table 7: $EC_{50}$'s for cytokine production**

|  | $EC_{50}$ (pM) Molm-13 tumor cell line | |
|---|---|---|
|  | Monomeric anti-CD33/CD3ε Bi-Fc | Single chain anti-CD33/CD3ε |
| IFN-γ | 9.5 | 2.6 |
| TNF-α | 6.1 | 1.7 |
| IL-10 | 6.4 | 1.1 |
| IL-2 | 10.3 | 5.2 |
| IL-13 | 8.4 | 1.3 |

These results suggest that the monomeric anti-CD33/CD3ε Bi-Fc is a highly specific and potent scaffold capable of mediating cytokine release by T cells.

***Example 12: Cytolytic synapse formation in the presence of an anti-HER2/CD3ε single chain bispecific or an anti-HER2/CD3ε Bi-Fc***

The anti-HER2/CD3ε single chain bispecific and HER2/CD3ε Bi-Fc described in Example 1 were assayed to determine their ability to induce cytolytic synapse formation between T cells and HER2-expressing JIMT-1 tumor cells. JIMT-1 cells were distributed into 24-well poly-L-lysine-coated glass bottom culture plates ($0.5 \times 10^6$ cells/well in RPMI medium with 1% FCS and 2 g/L glucose). Following 1 hr incubation at 37 °C, JIMT-1 cells adhering to the glass wells were gently washed with warm DPBS. Freshly isolated CD8+ T cells ($1 \times 10^6$ cells per well from healthy donors) with or without 1 nM anti-HER2/CD3ε single chain bispecific or anti-HER2/CD3ε Bi-Fc were added to the tumor cells and allowed to incubate for an additional 20 minutes at 37 °C to generate cytolytic synapses. Cells adhering to the plate were washed with prewarmed DPBS and immediately fixed with 3.7% parafomaldehyde for 10 minutes. The cells were then washed with DPBS and permeabilized with 0.1% titron X-100 for 5 minutes at room temperature. A mixture of primary antibodies (5 μg/mL anti-PKCθ and 0.4 μg/mL anti-CD45) was incubated with cells overnight at 4 °C and then washed 3 times. A mixture of 8 μg/mL secondary antibodies (green for anti-CD45 and red for anti- PKCθ) were added for 3 hours at room temperature, and the plates were then washed 2X with DPBS. PCKθ is known to localize to immune synapses, while CD45 is expressed on the surface of T cells. SLOWFADE® Gold antifade reagent with DAPI (nuclear stain) (Life Technologies #536939) was added directly to glass wells and plates stored at -70 °C protected from light.

Immunofluorescence confocal microscopy showed that CD45 (green staining) was present on the surface of T cells (identified as the smaller cell type with green CD45 staining), while PKCθ (red staining) gave a focused signal at the site of synapse formation between tumor cells (identified as the larger cell type) and T cells. Cytolytic synapses between the T cells and tumor targets were observed with the anti-HER2/CD3ε single chain bispecific and anti-HER2/CD3ε Bi-Fc, but were not observed in the absence of a bispecific (data not shown). These observations suggest that cytolytic synapse formation is dependent on the presence a bispecific molecule, and the Bi-Fc can form synapses similar to those seen with the single chain bispecific molecule.

***Example 13: In vivo effects of a heterodimeric anti-FOLR1/CD3ε Bi-Fc on tumor growth***

The experiment described below demonstrates the activity of heterodimeric Bi-Fc bispecific antibody in an *in vivo* cancer model system, using FOLR1-expressing NCI-N87-luc, human gastric carcinoma cells. Although these cells do express luciferase, which can enable tumor detection by luminescence, tumor growth was monitored by physical measurement of the tumors in this experiment. NCI-N87-luc cells ($3 \times 10^6$) in 50% matrigel were implanted subcutaneously into 8 week old female NOD scid gamma (NSG) mice (day 0). On day 10, $20 \times 10^6$ activated human Pan-T cells were administered by intraperitoneal injection into each mouse. The human Pan-T cells engrafted into the mice were pre-activated and expanded using anti-CD3/CD28/CD2 antibodies on days 0 and 14 of an 18 day culture using Miltenyi T cell activation/expansion kit according to the manufacturer's directions. On day 11 and day 18, an FcγR block consisting of 10 mg/mouse GAMMAGARD [Immune Globulin Infusion (Human)] 10% (Baxter) plus 0.2 mg/mouse anti-mu FcγRII/III (clone 2.4G2) was administered IP. One hour following the first FcγR block, animals (N=10/group) received either (1) daily intraperitoneal injections of 0.05 mg/kg of an anti-FOLR1/anti-CD3ε single chain molecule (comprising the amino acid sequence of SEQ ID NO:90) or (2) two intraperitoneal injections, spaced 5 days apart of 1 mg/kg of a heterodimeric anti-FOLR1/anti-CD3ε Bi-Fc (comprising the amino acid sequences of SEQ ID NOs:86 and 88), or 25 mM Lysine-hydrochloride, 0.002% Tween 80 in 0.9% NaCl, pH 7.0 (vehicle control). Tumor volumes were measured, and animals were euthanized when their tumor reached 2000 mm³ or at the end of the study (day 27).

In vehicle-treated mice, tumors grew in all the animals tested. *See* Figure 16. In contrast, tumor growth was significantly inhibited in the mice that were treated with the single chain anti-FOLR1/CD3ε bispecific or the heterodimeric anti-FOLR1/CD3ε Bi-Fc (p<0.0001 when compared to vehicle-treated mice). Throughout the experiment, there were no significant changes in body weight of treated or untreated mice (data not shown). These data indicate that the anti-FOLR1/anti-CD3ε heterodimeric Bi-Fc can induce T cell-mediated killing of target cells *in vivo*.

***Example 14: Comparison of the in vivo effects of monomeric and heterodimeric anti-CD33/CD3 Bi-Fc's on tumor growth***

The following experiment was aimed at determining whether a monomeric Bi-Fc could kill tumor cells *in vivo*. Human pan-T cells were pre-activated and expanded in culture for use in this experiment by addition of anti-CD3/CD28/CD2 antibodies on days 0 and 14 of an 18-day culture period using a Miltenyi T cell activation/expansion kit according to the manufacturer's directions. Molm-13-luc cells ($1 \times 10^6$), which are CD33-expressing tumor cells that luminesce in the presence of D-luciferin, were injected subcutaneously (SC) into the right flank of 10 week old female NSG mice (day 0). On the third day following tumor cell inoculation, $20 \times 10^6$ of the activated human pan-T cells were administered to each mouse by IP injection. On days 4 and 11, an FcγR block as described in Example 13 was administered by IP injection. One hour following the day 4 FcγR block, the mice (N=8/group) received one of the following treatments: (1) daily intraperitoneal injections of either 0.05 mg/kg of the anti-CD33/CD3ε single chain bispecific (having the amino acid sequence of SEQ ID NO:33), 0.05 mg/kg of an anti-MEC/CD3ε single chain bispecific (having the amino acid sequence of SEQ ID NO:78; a negative control), 0.05 mg/kg of a monomeric anti-CD33/CD3ε Bi-Fc (having the amino acid sequence of SEQ ID NO:34), or 25 mM lysine-hydrochloride, 0.002% Tween 80 in 0.9% NaCl, pH 7.0 (a vehicle control) for 10 days; or (2) two IP injections, spaced 5 days apart of 1 mg/kg anti-CD33/CD3ε heterodimeric Bi-Fc (comprising the amino acid sequences of SEQ ID NOs:80 and 82).

Bioluminescent imaging was performed on Monday, Wednesday, and Friday for two weeks after dosing began with an IVIS®-200 In Vivo Imaging System (Perkin Elmer). Nine minutes before imaging, mice were given 150 mg/kg D-luciferin by IP injection. Images were collected and analyzed using LIVING IMAGE® software 2.5 (Caliper Life Sciences). Naive animals (animals not inoculated with Molm-13-luc or human pan-T cells) were used as to measure baseline bioluminescence.

Mice that received vehicle or the anti-MEC/CD3ε single chain experienced tumor growth throughout the study, and naïve control mice that received no tumor cells did not exhibit appreciable tumor cell growth. Figure 17. Mice that received either the anti-CD33/CD3ε heterodimeric Bi-Fc or the single chain molecule initially experienced tumor growth, although tumor cell luminescence approximately equal to levels seen in naïve mice were observed in these groups by the end of the study. Mice that received the monomeric anti-CD33/CD3ε Bi-Fc also experienced initial tumor growth followed by tumor cell luminescence that was intermediate between that observed in vehicle-treated mice and that observed in naïve mice by the end of the study. Figure 17. The difference between tumor growth in mice treated with vehicle versus those treated with the monomeric Bi-Fc was statistically significant ($p < -.0001$). Thus, the monomeric Bi-Fc did elicit tumor cell killing *in vivo.*

### Example 15: Effect of the Fc alteration N297G on the in vivo anti-tumor efficacy of a monomeric anti-CD33/CD3 Bi-Fc

The following experiment compared the *in vivo* activity of a monomeric anti-CD33/CD3ε Bi-Fc that had the N297G alteration in the Fc polypeptide chain portion of the Bi-Fc to the activity of one that had the wild type N297. Methods are essentially the same as those described in Example 14. Molm-13-luc cells ($1 \times 10^6$) were injected subcutaneously (SC) into the right flank of 10 week old female NSG mice (day 0), and $20 \times 10^6$ pre-activated human pan-T cells were administered to each mouse by IP injection on day 3. On days 4 and 11, an FcγR block as described in Example 13 was administered. One hour following the day 4 FcγR block, the mice (N=10/group) received daily IP injections of one of the following: vehicle (25 mM lysine-hydrochloride, 0.002% Tween 80 in 0.9% NaCl, pH 7.0); a monomeric anti-CD33/CD3ε Bi-Fc comprising the amino acid sequence of SEQ ID NO:34 (which has the N297G alteration); or a monomeric anti-CD33/CD3ε Bi-Fc comprising the amino acid sequence of SEQ ID NO:84 (which has the wild type N297). Naïve control mice did not receive an injection of tumor cells and received no treatment injections. Results are shown in Figure 18.

Vehicle-treated mice exhibited tumor growth. Small but significant ($p < 0.0005$) differences in tumor growth existed between vehicle-treated mice and mice treated with the monomeric anti-CD33/CD3ε Bi-Fc having the N297G alteration. Figure 18. Mice treated with the monomeric anti-CD33/CD3ε Bi-Fc with the wild type N297 had significantly ($p < 0.0001$) lower levels of tumor bioluminescence by the end of the study than vehicle-treated mice. Naive mice had, as expected, low levels of bioluminescence. Figure 18. At the least, these results suggest that a monomeric Bi-Fc having the wild type N297 is as active, if not more active, than one having N297G in an *in vivo* tumor cell killing assay.

**Claims**

1.  A bispecific Fc (Bi-Fc) polypeptide, which is monomeric and comprises

    (a) a polypeptide chain comprising an amino acid sequence having the following formula: V1-L1-V2-L2-V3-L3-V4-L4-Fc; wherein Fc is a human IgG Fc polypeptide chain which is C-terminal relative to the four immunoglobulin variable regions;

    wherein two of V1, V2, V3, and V4 are immunoglobulin heavy chain variable (VH) regions and the other two are immunoglobulin light chain variable (VL) regions;
    wherein either V1 is a VH region and V2 is a VL region or vice versa and either V3 is a VH region and V4 is a VL region or vice versa;
    wherein L1, L2, L3, and L4 are linkers, wherein L2 is present and wherein L2 is not more than 12 amino acids long;
    wherein L1 and L3 are each at least 15 amino acids long; and
    wherein L4 can be present or absent; or

    (b) a polypeptide chain comprising an amino acid sequence having the following formula: Fc-L4-V1-L1-V2-L2-V3-L3-V4; wherein Fc is a human IgG Fc polypeptide chain which is N-terminal relative to the four immunoglobulin variable regions;

wherein either V1 is a VH region and V2 is a VL region or vice versa and either V3 is a VH region and V4 is a VL region or vice versa;
wherein two of V1, V2, V3, and V4 are VH regions and the other two are VL regions;
wherein L1, L2, L3, and L4 are linkers, wherein L2 is present and wherein L2 is not more than 12 amino acids long;
wherein L1 and L3 are each at least 15 amino acids long; and
wherein L4 can be present or absent;

wherein the Bi-Fc binds to a target cell and an immune effector cell and/or mediates cytolysis of a target cell by an immune effector cell, wherein the polypeptide chain targeting the effector cell binds to human and/or cynomolgus CD3ε,
wherein the Fc polypeptide chain of the Bi-Fc comprises an insertion of the amino acid sequence of any of SEQ ID NOs:36-47 between positions 384 and 385, wherein these position numbers are assigned according to the EU numbering scheme.

2. The Bi-Fc polypeptide of claim 1, wherein the Fc polypeptide chain of (a) or (b) comprises one or more of the following alterations: K392D, K392E, K409D, K409E, Y349T, L351T, L368T, L398T, F405T, Y407T, and Y407R, wherein these position numbers are assigned according to the EU numbering scheme.

3. The Bi-Fc polypeptide of any one of claims 1 to 2, which binds to a cell expressing human HER2, human FOLR1 or human CD33.

4. A bispecific (Bi-Fc) polypeptide, which is heterodimeric comprising:

(a)

(i) a first polypeptide chain comprising an amino acid sequence having the following formula: V1-L1-V2-L2-V3-L3-V4-L4-Fc; wherein Fc is a human IgG Fc polypeptide chain which is C-terminal relative to the four immunoglobulin variable regions; wherein V1, V2, V3, and V4 are each immunoglobulin variable regions; wherein L1, L2, L3, and L4 are linkers; and wherein L4 can be present or absent; and
(ii) a second polypeptide chain that comprises a human IgG Fc polypeptide chain; or

(b)

(i) a first polypeptide chain having the following formula: Fc-L4-V1-L1-V2-L2-V3-L3-V4; wherein Fc is a human IgG Fc polypeptide chain which is N-terminal relative to the four immunoglobulin variable regions; wherein V1, V2, V3, and V4 are each immunoglobulin variable regions; wherein L1, L2, L3, and L4 are linkers; and wherein L4 can be present or absent; and
(ii) a second polypeptide chain that comprises a human IgG Fc polypeptide chain;

wherein the Bi-Fc binds to a target cell and an immune effector cell and/or mediates cytolysis of a target cell by an immune effector cell,
wherein L1 and L3 are at least 15 amino acids long,
wherein L2 is less than 12 amino acids long,
wherein either V1 is a VH region and V2 is a VL region or vice versa,
wherein either V3 is a VH region and V4 is a VL region or vice versa,
wherein the Bi-Fc binds to human and/or cynomolgus CD3ε,
wherein the Fc polypeptide chain(s) comprise(s) an insertion of the amino acid sequence of any of SEQ ID NOs:36-47 between positions 384 and 385 of each Fc polypeptide chain, wherein positions 384 and 385 are positions assigned according to the EU numbering scheme.

5. The Bi-Fc polypeptide of claim 4, which binds to a cell expressing human CD33, human FOLR1, or human HER2.

6. The Bi-Fc polypeptide of claim 1 or 4, which comprises:

(a) a heavy chain CDR1 comprising the amino acid sequence of SEQ ID NO:60,

a heavy chain CDR2 comprising the amino acid sequence of SEQ ID NO:61,
a heavy chain CDR3 comprising the amino acid sequence of SEQ ID NO:62,
a light chain CDR1 comprising the amino acid sequence of SEQ ID NO:63,
a light chain CDR2 comprising the amino acid sequence of SEQ ID NO:64, and
a light chain CDR3 comprising the amino acid sequence of SEQ ID NO:65;

(b) a heavy chain CDR1 comprising the amino acid sequence of SEQ ID NO:66,

a heavy chain CDR2 comprising the amino acid sequence of SEQ ID NO:67,
a heavy chain CDR3 comprising the amino acid sequence of SEQ ID NO:68,
a light chain CDR1 comprising the amino acid sequence of SEQ ID NO:69,
a light chain CDR2 comprising the amino acid sequence of SEQ ID NO:70, and
a light chain CDR3 comprising the amino acid sequence of SEQ ID NO:71; or

(c) a heavy chain CDR1 comprising the amino acid sequence of SEQ ID NO:72;

a heavy chain CDR2 comprising the amino acid sequence of SEQ ID NO:73;
a heavy chain CDR3 comprising the amino acid sequence of SEQ ID NO:74;
a light chain CDR1 comprising the amino acid sequence of SEQ ID NO:75;
a light chain CDR2 comprising the amino acid sequence of SEQ ID NO:76; and
a light chain CDR3 comprising the amino acid sequence of SEQ ID NO:77.

7. The Bi-Fc polypeptide of any one of claims 4-6, wherein the Fc polypeptide chains of the first and second polypeptide chains comprise one or more alteration that inhibits FcγR binding selected from the group consisting of: L234A, L235A, and any substitution at N297.

8. The Bi-Fc polypeptide of claim 4, wherein V3 comprises the amino acid sequence of SEQ ID NO:7 or 29 and V4 comprises the amino acid sequence of SEQ ID NO:8 or 31.

9. The Bi-Fc polypeptide of any one of claims 1 to 8, wherein the target cell is a cancer cell.

10. The Bi-Fc polypeptide of any one of claims 1, 2, 4 and 6-8, wherein the target cell is a cell infected by a pathogen or a cell that mediates a disease.

11. The Bi-Fc polypeptide of claim 10, wherein

(a) the pathogen is virus including human immunodeficiency virus, hepatitis virus, human papilloma virus, or cytomegalovirus, or a bacterium of the genus Listeria, Mycobacterium, Staphylococcus, or Streptococcus; or
(b) the cell that mediates a disease is a fibrotic cell that mediates a fibrotic disease.

12. The Bi-Fc polypeptide of any one of claims 1 to 11, wherein the Bi-Fc binds to an amino acid sequence within the first 27 amino acids of human or cynomolgus CD3ε as determined by alanine scanning.

13. The Bi-Fc polypeptide of any one of claims 1 to 12 , wherein the Bi-Fc comprises (1) a VH region comprising a CDR1, a CDR2, and a CDR3 comprising, respectively, the amino acid sequences of SEQ ID NO:48, SEQ ID NO:49, and SEQ ID NO:50 and a VL region comprising a CDR1. a CDR2, and a CDR3 comprising, respectively, the amino acid sequences of SEQ ID NO:51, SEQ ID NO:52, and SEQ ID NO:53

14. A pharmaceutical formulation comprising the Bi-Fc polypeptide of any one of claims 1 to 13 and a physiologically acceptable excipient.

15. One or more nucleic acid(s) encoding the Bi-Fc polypeptide of any one of claims 1 to 13.

16. One or more vector(s) comprising the nucleic acid(s) of claim 15.

17. A host cell containing the nucleic acid(s) of claim 15 and/or the vector(s) of claim 16.

**18.** A method of making a Bi-Fc polypeptide comprising
culturing the host cell of claim 17 under conditions such that the nucleic acid is expressed, and recovering the Bi-Fc polypeptide from the cell mass or the culture medium.

**19.** A pharmaceutical composition for use in a method of treatment of a cancer, an infectious disease, an autoimmune or inflammatory disease or a fibrotic disease comprising the Bi-Fc polypeptide of any one of claims 1 to 13.

**Patentansprüche**

**1.** Bispezifisches Fc (Bi-Fc)-Polypeptid, das monomer ist und Folgendes umfasst

(a) eine Polypeptidkette, die eine Aminosäuresequenz mit der folgenden Formel umfasst: V1-L1-V2-L2-V3-L3-V4-L4-Fc; wobei Fc eine humane IgG-Fc-Polypeptidkette ist, die relativ zu den vier variablen Immunglobulin-Regionen C-terminal ist;

wobei zwei von V1, V2, V3 und V4 Immunoglobulinschwere-Kette-variable (VH)-Regionen sind und die anderen beiden Immunoglobulin-leichte-Kettevariable (VH)-Regionen sind;
wobei entweder V1 eine VH-Region und V2 eine VL-Region ist oder umgekehrt und entweder V3 eine VH-Region und V4 eine VL-Region ist oder umgekehrt;
wobei L1, L2, L3 und L4 Linker sind, wobei L2 vorhanden ist und wobei L2 nicht mehr als 12 Aminosäuren lang ist;
wobei L1 und L3 jeweils mindestens 15 Aminosäuren lang sind; und
wobei L4 vorhanden oder abwesend sein kann; oder

(b) eine Polypeptidkette, die eine Aminosäuresequenz mit der folgenden Formel umfasst: Fc-L4-V1-L1-V2-L2-V3-L3-V4; wobei Fc eine humane IgG-Fc-Polypeptidkette ist, die N-terminal zu den vier variablen ImmunglobulinRegionen ist;

wobei entweder V1 eine VH-Region und V2 eine VL-Region ist oder umgekehrt und entweder V3 eine VH-Region und V4 eine VL-Region ist oder umgekehrt;
wobei zwei von V1, V2, V3 und V4 VH-Regionen sind und die anderen beiden VL-Regionen sind;
wobei L1, L2, L3 und L4 Linker sind, wobei L2 vorhanden ist und wobei L2 nicht mehr als 12 Aminosäuren lang ist;
wobei L1 und L3 jeweils mindestens 15 Aminosäuren lang sind; und
wobei L4 vorhanden oder abwesend sein kann;
wobei das Bi-Fc an eine Zielzelle und eine Immuneffektorzelle bindet und/oder die Zytolyse einer Zielzelle durch eine Immuneffektorzelle vermittelt, wobei die Polypeptidkette, die auf die Effektorzelle zielt, an humanes und/oder Cynomolgus-CD3s bindet,
wobei die Fc-Polypeptidkette des Bi-Fc eine Insertion der Aminosäuresequenz einer der SEQ ID NOs: 36-47 zwischen den Positionen 384 und 385 umfasst, wobei diese Positionsnummern gemäß dem EU-Nummerierungsschema zugeordnet werden.

**2.** Bi-Fc-Polypeptid nach Anspruch 1, wobei die Fc-Polypeptidkette von (a) oder (b) eine oder mehrere der folgenden Änderungen umfasst: K392D, K392E, K409D, K409E, Y349T, L351T, L368T, L398T, F405T, Y407T und Y407R, wobei diese Positionsnummern gemäß dem EU-Nummerierungsschema zugeordnet werden.

**3.** Bi-Fc-Polypeptid nach einem der Ansprüche 1 bis 2, das an eine Zelle bindet, die humanes HER2, humanes FOLR1 oder humanes CD33 exprimiert.

**4.** Bispezifisches (Bi-Fc) Polypeptid, das heterodimer ist und Folgendes umfasst:

(a)

(i) eine erste Polypeptidkette, die eine Aminosäuresequenz mit der folgenden Formel umfasst: VI-LI-V2-L2-V3-L3-V4-L4-Fc; wobei Fc eine humane IgG-Fc-Polypeptidkette ist, die relativ zu den vier variablen Immunoglobulin-Regionen C-terminal ist; wobei V1, V2, V3 und V4 jeweils variable Immunoglobulin-Regionen sind; wobei L1, L2, L3 und L4 Linker sind; und wobei L4 vorhanden oder abwesend sein kann; und

(ii) eine zweite Polypeptidkette, die eine humane IgG-Fc-Polypeptidkette umfasst; oder

(b)

(i) eine erste Polypeptidkette mit der folgenden Formel: FC-L4-V1-L1-V2-L2-V3-L3-V4; wobei Fc eine humane IgG-Fc-Polypeptidkette ist, die N-terminal in Bezug auf die vier variablen Immunglobulinregionen ist; wobei V1, V2, V3 und V4 jeweils variable Immunglobulinregionen sind; wobei L1, L2, L3 und L4 Linker sind; und wobei L4 vorhanden oder abwesend sein kann; und
(ii) eine zweite Polypeptidkette, die eine humane IgG-Fc-Polypeptidkette umfasst;

wobei das Bi-Fc an eine Zielzelle und eine Immuneffektorzelle bindet und/oder die Zytolyse einer Zielzelle durch eine Immuneffektorzelle vermittelt,
wobei L1 und L3 mindestens 15 Aminosäuren lang sind, wobei L2 weniger als 12 Aminosäuren lang ist, wobei entweder V1 eine VH-Region ist und V2 eine VL-Region ist oder umgekehrt,
wobei entweder V3 eine VH-Region ist und V4 eine VL-Region oder umgekehrt,
wobei das Bi-Fc an humanes und/oder Cynomolgus-CD3s bindet,
wobei die Fc-Polypeptidkette(n) eine Insertion der Aminosäuresequenz einer der SEQ ID NOs: 36-47 zwischen den Positionen 384 und 385 jeder Fc-Polypeptidkette umfasst (umfassen), wobei die Positionen 384 und 385 Positionen sind, die gemäß dem EU-Nummerierungsschema zugeordnet sind.

5. Bi-Fc-Polypeptid nach Anspruch 4, das an eine Zelle bindet, die humanes CD33, humanes FOLR1 oder humanes HER2 exprimiert.

6. Bi-Fc-Polypeptid nach Anspruch 1 oder 4, das Folgendes umfasst:

(a) eine Schwere-Kette-CDR1, umfassend die Aminosäuresequenz von SEQ ID NO:60,

eine Schwere-Kette-CDR2, umfassend die Aminosäuresequenz von SEQ ID NO:61,
eine Schwere-Kette-CDR3, umfassend die Aminosäuresequenz von SEQ ID NO:62,
eine Leichte-Kette-CDR1, umfassend die Aminosäuresequenz von SEQ ID NO:63,
eine Leichte-Kette-CDR2, umfassend die Aminosäuresequenz von SEQ ID NO:64, und
eine Leichte-Kette-CDR3, umfassend die Aminosäuresequenz von SEQ ID NO:65;

(b) eine Schwere-Kette-CDR1, umfassend die Aminosäuresequenz von SEQ ID NO:66,

eine Schwere-Kette-CDR2, umfassend die Aminosäuresequenz von SEQ ID NO:67,
eine Schwere-Kette-CDR3, umfassend die Aminosäuresequenz von SEQ ID NO:68,
eine Leichte-Kette-CDR1, umfassend die Aminosäuresequenz von SEQ ID NO:69,
eine Leichte-Kette-CDR2, umfassend die Aminosäuresequenz von SEQ ID NO:70, und
eine Leichte-Kette-CDR3, umfassend die Aminosäuresequenz von SEQ ID NO:71; oder

(c) eine Schwere-Kette-CDR1, umfassend die Aminosäuresequenz von SEQ ID NO:72;

eine Schwere-Kette-CDR2, umfassend die Aminosäuresequenz von SEQ ID NO:73;
eine Schwere-Kette-CDR3, umfassend die Aminosäuresequenz von SEQ ID NO:74;
eine Leichte-Kette-CDR1, umfassend die Aminosäuresequenz von SEQ ID NO:75;
eine Leichte-Kette-CDR2, umfassend die Aminosäuresequenz von SEQ ID NO:76; und
eine Leichte-Kette-CDR3, umfassend die Aminosäuresequenz von SEQ ID NO:77.

7. Bi-Fc-Polypeptid nach einem der Ansprüche 4-6, wobei die Fc-Polypeptidketten der ersten und zweiten Polypeptidketten eine oder mehrere Änderungen umfassen, die das FcγR-Binden hemmen, ausgewählt aus der Gruppe bestehend aus: L234A, L235A und jede Substitution an N297.

8. Bi-Fc-Polypeptid nach Anspruch 4, wobei V3 die Aminosäuresequenz von SEQ ID NO: 7 oder 29 umfasst und V4 die Aminosäuresequenz von SEQ ID NO: 8 oder 31 umfasst.

9. Bi-Fc-Polypeptid nach einem der Ansprüche 1 bis 8, wobei die Zielzelle eine Krebszelle ist.

**10.** Bi-Fc-Polypeptid nach einem der Ansprüche 1, 2, 4 und 6-8, wobei die Zielzelle eine mit einem Pathogen infizierte Zelle oder eine Zelle ist, die eine Krankheit vermittelt.

**11.** Bi-Fc-Polypeptid nach Anspruch 10, wobei

(a) der Erreger ein Virus ist, einschließlich humanes Immunschwäche-Virus, HepatitisVirus, humanes Papillom-Virus oder Cytomegalo-Virus, oder ein Bakterium der Gattung Listeria, Mycobacterium, Staphylococcus oder Streptococcus; oder
(b) die Zelle, die eine Krankheit vermittelt, eine fibrotische Zelle ist, die eine fibrotische Krankheit vermittelt.

**12.** Bi-Fc-Polypeptid nach einem der Ansprüche 1 bis 11, wobei das Bi-Fc an eine Aminosäuresequenz innerhalb der ersten 27 Aminosäuren von humanem oder Cynomolgus-CD3s bindet, wie durch Alanin-Scanning bestimmt.

**13.** Bi-Fc-Polypeptid nach einem der Ansprüche 1 bis 12, wobei das Bi-Fc (1) eine VH-Region umfasst, umfassend eine CDR1, eine CDR2 und eine CDR3, umfassend jeweils die Aminosäuresequenzen SEQ ID NO: 48, SEQ ID NO: 49 und SEQ ID NO: 50, und eine VL-Region, umfassend eine CDR1, eine CDR2 und eine CDR3, umfassend jeweils die Aminosäuresequenzen SEQ ID NO: 51, SEQ ID NO: 52, und SEQ ID NO: 53.

**14.** Pharmazeutische Formulierung, umfassend das Bi-Fc-Polypeptid nach einem der Ansprüche 1 bis 13 und einen physiologisch akzeptablen Exzipienten.

**15.** Eine oder mehrere Nukleinsäure(n), die das Bi-Fc-Polypeptid nach einem der Ansprüche 1 bis 13 codiert (codieren).

**16.** Ein oder mehrere Vektor(en), umfassend die Nukleinsäure(n) nach Anspruch 15.

**17.** Wirtszelle, enthaltend die Nukleinsäure(n) nach Anspruch 15 und/oder den/die Vektor(en) nach Anspruch 16.

**18.** Verfahren zur Herstellung eines Bi-Fc-Polypeptids, umfassend

Kultivieren der Wirtszelle nach Anspruch 17 unter solchen Bedingungen, dass die Nukleinsäure exprimiert wird, und
Gewinnen des Bi-Fc-Polypeptids aus der Zellmasse oder dem Kulturmedium.

**19.** Pharmazeutische Zusammensetzung zur Verwendung in einem Verfahren zur Behandlung von Krebs, einer Infektionskrankheit, einer Autoimmun- oder Entzündungskrankheit oder einer fibrotischen Krankheit, umfassend das Bi-Fc-Polypeptid nach einem der Ansprüche 1 bis 13.


**Revendications**

**1.** Polypeptide Fc bispécifique (Bi-Fc), qui est monomérique et comprend

(a) une chaîne polypeptidique comprenant une séquence d'acides aminés ayant la formule suivante : V1-L1-V2-L2-V3-L3-V4-L4-Fc ; Fc étant une chaîne polypeptidique Fc d'IgG humaine qui est C-terminale par rapport aux quatre régions variables immunoglobuline ;

deux parmi V1, V2, V3, et V4 étant des régions variables de chaîne lourde (VH) d'immunoglobuline et les deux autres étant des régions variables de chaîne légère (VL) d'immunoglobuline ;
soit V1 est une région VH et V2 est une région VL ou vice versa et soit V3 est une région VH et V4 est une région VL ou vice versa ;
L1, L2, L3, et L4 étant des lieurs, L2 étant présent et, L2 n'étant pas de plus de 12 acides aminés de longueur ;
L1 et L3 étant chacun d'une longueur d'au moins 15 acides aminés ; et
L4 pouvant être présent ou absent ; ou

(b) une chaîne polypeptidique comprenant une séquence d'acides aminés ayant la formule suivante : Fc-L4-V1-L1-V2-L2-V3-L3-V4 ; Fc étant une chaîne polypeptidique Fc d'IgG humaine qui est N-terminale par rapport aux quatre régions variables immunoglobuline ;

soit V1 est une région VH et V2 est une région VL ou vice versa et soit V3 est une région VH et V4 est une région VL ou vice versa ;

deux parmi V1, V2, V3, et V4 étant des régions VH et les deux autres étant des régions VL ;

L1, L2, L3, et L4 étant des lieurs, L2 étant présent et, L2 n'étant pas de plus de 12 acides aminés de longueur ;

L1 et L3 étant chacun d'une longueur d'au moins 15 acides aminés ; et

L4 pouvant être présent ou absent ;

le Bi-Fc se liant à une cellule cible et une cellule effectrice immunitaire et/ou médiant une cytolyse d'une cellule cible par une cellule effectrice immunitaire, la chaîne polypeptidique ciblant la cellule effectrice se liant à CD3ε humaine et/ou de cynomolgus,

la chaîne polypeptidique Fc du Bi-Fc comprenant une insertion de la séquence d'acides aminés de l'une quelconque parmi les SEQ ID NO: 36 à 47 entre les positions 384 et 385, ces numéros de position étant assignés selon le schéma de numérotation de l'UE.

2. Polypeptide Bi-Fc selon la revendication 1, la chaîne polypeptidique Fc de (a) ou (b) comprenant l'une ou plusieurs des modifications suivantes : K392D, K392E, K409D, K409E, Y349T, L351T, L368T, L398T, F405T, Y407T, et Y407R, ces numéros de position étant assignés selon le schéma de numérotation de l'UE.

3. Polypeptide Bi-Fc selon l'une quelconque des revendications 1 à 2, qui se lie à une cellule exprimant HER2 humaine, FLOR1 humaine ou CD33 humaine.

4. Polypeptide (Bi-Fc) bispécifique, qui est hétérodimérique comprenant :

(a)

(i) une première chaîne polypeptidique comprenant une séquence d'acides aminés ayant la formule suivante : V1-L1-V2-L2-V3-L3-V4-L4-Fc ; Fc étant une chaîne polypeptidique Fc d'IgG humaine qui est C-terminale par rapport aux quatre régions variables immunoglobuline ; V1, V2, V3, et V4 étant chacune des régions variables globuline L1, L2, L3, et L4 étant des lieurs ; et L4 pouvant être présent ou absent ; et

(ii) une deuxième chaîne polypeptidique qui comprend une chaîne polypeptidique Fc d'IgG humaine ; ou

(b)

(i) une première chaîne polypeptidique comprenant une séquence d'acides aminés ayant la formule suivante : Fc-L4-V1-L1-V2-L2-V3-L3-V4 ; Fc étant une chaîne polypeptidique Fc d'IgG humaine qui est N-terminale par rapport aux quatre régions variables immunoglobuline ; V1, V2, V3, et V4 étant chacune des régions variables globuline L1, L2, L3, et L4 étant des lieurs ; et L4 pouvant être présent ou absent ; et

(ii) une deuxième chaîne polypeptidique qui comprend une chaîne polypeptidique Fc d'IgG humaine ;

le Bi-Fc se liant à une cellule cible et une cellule effectrice immunitaire et/ou médiant une cytolyse d'une cellule cible par une cellule effectrice immunitaire,

L1 et L3 étant d'une longueur d'au moins 15 acides aminés,

L2 étant d'une longueur de moins de 12 acides aminés ;

soit V1 est une région VH et V2 est une région VL ou vice versa,

soit V3 est une région VH et V4 est une région VL ou vice versa,

le Bi-Fc se liant à CD3ε humaine et/ou de cynomolgus,

la ou les chaîne polypeptidique Fc comprenant une insertion de la séquence d'acides aminés de l'une quelconque parmi les SEQ ID NO: 36 à 47 entre les positions 384 et 385 de chaque chaîne polypeptidique Fc, les positions 384 et 385 étant des positions assignées selon le schéma de numérotation de l'UE.

5. Polypeptide Bi-Fc selon la revendication 4, qui se lie à une cellule exprimant CD33 humaine, FLOR1 humaine ou HER2 humaine.

6. Polypeptide Bi-Fc selon la revendication 1 ou 4, qui comprend :

(a) une CDR1 de chaîne lourde comprenant la séquence d'acides aminés de la SEQ ID NO: 60,

une CDR2 de chaîne lourde comprenant la séquence d'acides aminés de la SEQ ID NO: 61,

une CDR3 de chaîne lourde comprenant la séquence d'acides aminés de la SEQ ID NO: 62,
une CDR1 de chaîne légère comprenant la séquence d'acides aminés de la SEQ ID NO: 63,
une CDR2 de chaîne légère comprenant la séquence d'acides aminés de la SEQ ID NO: 64, et
une CDR3 de chaîne légère comprenant la séquence d'acides aminés de la SEQ ID NO: 65 ;

(b) une CDR1 de chaîne lourde comprenant la séquence d'acides aminés de la SEQ ID NO: 66,

une CDR2 de chaîne lourde comprenant la séquence d'acides aminés de la SEQ ID NO: 67,
une CDR3 de chaîne lourde comprenant la séquence d'acides aminés de la SEQ ID NO: 68,
une CDR1 de chaîne légère comprenant la séquence d'acides aminés de la SEQ ID NO: 69,
une CDR2 de chaîne légère comprenant la séquence d'acides aminés de la SEQ ID NO: 70, et
une CDR3 de chaîne légère comprenant la séquence d'acides aminés de la SEQ ID NO: 71 ;

(c) une CDR1 de chaîne lourde comprenant la séquence d'acides aminés de la SEQ ID NO: 72,

une CDR2 de chaîne lourde comprenant la séquence d'acides aminés de la SEQ ID NO: 73,
une CDR3 de chaîne lourde comprenant la séquence d'acides aminés de la SEQ ID NO: 74,
une CDR1 de chaîne légère comprenant la séquence d'acides aminés de la SEQ ID NO: 75,
une CDR2 de chaîne légère comprenant la séquence d'acides aminés de la SEQ ID NO: 76, et
une CDR3 de chaîne légère comprenant la séquence d'acides aminés de la SEQ ID NO: 77.

7. Polypeptide Bi-Fc selon l'une quelconque des revendications 4-6, les chaînes polypeptidiques Fc de la première et deuxième chaînes polypeptidiques comprenant une ou plusieurs modifications qui inhibent une liaison à FcγR choisies dans le groupe constitué par : L234A, L235A, et une quelconque substitution au niveau de N297.

8. Polypeptide Bi-Fc selon la revendication 4, V3 comprenant la séquence d'acides aminés de la SEQ ID NO: 7 ou 29 et V4 comprenant la séquence d'acides aminés de la SEQ ID NO: 8 ou 31.

9. Polypeptide Bi-Fc selon l'une quelconque des revendications 1 à 8, la cellule cible étant une cellule cancéreuse.

10. Polypeptide Bi-Fc selon l'une quelconque des revendications 1, 2, 4 et 6 à 8, la cellule cible étant une cellule infectée par un pathogène ou une cellule qui médie une maladie.

11. Polypeptide Bi-Fc selon la revendication 10,

(a) le pathogène étant un virus comprenant un virus de l'immunodéficience humaine, un virus de l'hépatite, un papillomavirus humain, ou un cytomégalovirus, ou une bactérie du genre Listeria, Mycobacterium, Staphylococcus ou Streptococcus ; ou
(b) la cellule qui médie une maladie étant une cellule fibrotique qui médie une maladie fibrotique.

12. Polypeptide Bi-Fc selon l'une quelconque des revendications 1 à 11, le Bi-Fc se liant à une séquence d'acides aminés dans les premiers 27 acides aminés de CD3ε humaine ou de cynomolgus comme déterminé par balayage d'alanine.

13. Polypeptide Bi-Fc selon l'une quelconque des revendications 1 à 12, le Bi-Fc comprenant (1) une région VH comprenant une CDR1, une CDR2 et une CDR3 comprenant, respectivement, les séquences d'acides aminés de la SEQ ID NO: 48, SEQ ID NO: 49, et SEQ ID NO: 50 et une région VL comprenant une CDR1, une CDR2 et une CDR3 comprenant, respectivement, les séquences d'acides aminés de la SEQ ID NO: 51, SEQ ID NO: 52 et SEQ ID NO: 53.

14. Formulation pharmaceutique comprenant le polypeptide Bi-Fc selon l'une quelconque des revendications 1 à 13 et un excipient physiologiquement acceptable.

15. Acide(s) nucléique(s) codant pour le polypeptide Bi-Fc selon l'une quelconque des revendications 1 à 13.

16. Vecteur(s) comprenant l'acide ou les acides nucléiques selon la revendication 15.

17. Cellule hôte contenant l'acide ou les acides nucléiques selon la revendication 15 et/ou le ou les vecteurs selon la revendication 16.

18. Procédé de préparation d'un polypeptide Bi-Fc comprenant

la culture de la cellule hôte selon la revendication 17 dans des conditions telles que l'acide nucléique est exprimé, et
la récupération du polypeptide Bi-Fc de la masse cellulaire ou du milieu de culture.

19. Composition pharmaceutique pour une utilisation dans un procédé de traitement d'un cancer, d'une maladie infectieuse, d'une maladie auto-immune ou inflammatoire ou d'une maladie fibrotique comprenant le polypeptide Bi-Fc selon l'une quelconque des revendications 1 à 13.

**A**    **B**    **C**    **D**

**Figure 1**

EP 2 970 477 B1

**Figure 2**

Figure 3

**Figure 4**

45

**Figure 5A**

**Figure 5B**

**Figure 6A**

**Figure 6B**

**Figure 7**

**Figure 8**

**Figure 9**

**Figure 9** (cont.)

**Figure 10**

**A** Molm-13

**B** Namalwa

Concentration (pM)

## Figure 11

**Figure 12**

Figure 13

Figure 14

Figure 15

**Figure 15** (cont.)

# Figure 16

**Figure 17**

**Figure 18**

## REFERENCES CITED IN THE DESCRIPTION

### Patent documents cited in the description

- WO 2013026837 A
- WO 2009089004 A
- US 20070105199 A
- US 20090311253 A
- US 2012244578
- US 8592562 B
- US 7083784 B
- US 20100234575
- US 7670600 B
- US 2012070146 W
- WO 2013096221 A
- US 20120244578 A
- US 7695936 B
- US 20030078385
- WO 2012125850 A
- US 20030114659
- US 2010183615
- US 20120244578
- US 2012244162 A

### Non-patent literature cited in the description

- **BARGOU et al.** *Science,* 2008, vol. 321 (5891), 974-977
- **CARAYANNOPOULOS ; CAPRA.** FUNDAMENTAL IMMUNOLOGY. Raven Press, 1993, 284-286
- **MULDERMANS et al.** *J. Biotechnol.,* 2001, vol. 74, 277-302
- **DESMYTER et al.** *J. Biol. Chem.,* 2001, vol. 276, 26285-90
- **STRELTSOV et al.** *Protein Science,* 2005, vol. 14, 2901-2909
- BISPECIFIC ANTIBODIES. Springer, 2011
- **DEVITA et al.** Cancer: Principles and Practice of Oncology. J.B. Lippincott Co, 1993
- Principles of Cancer Therapy. **MERCK.** Manual of Diagnosis and Therapy. 1999, vol. 144
- **EDELMAN et al.** *Proc. Natl. Acad. Sci.,* 1969, vol. 63, 78-85
- **XU et al.** *Cellular Immunol.,* 2000, vol. 200, 16-26
- **KABAT et al.** SEQUENCES OF IMMUNOLOGICAL INTEREST. Public Health Service N.I.H, 1991
- **HONEGGER ; PLÜCKTHUN.** *J. Mol. Biol.,* 2001, vol. 309, 657-670
- **KABAT et al.** SEQUENCES OF PROTEINS OF IMMUNOLOGICAL INTEREST. Public Health Service N.I.H, 1991
- **DEVEREUX et al.** *Nucleic Acids Res.,* 1984, vol. 12, 387-95
- **GRIBSKOV ; BURGESS.** *Nucleic Acids Res.,* 1986, vol. 14, 6745
- Atlas of Polypeptide Sequence and Structure. National Biomedical Research Foundation, 1979, 353-358
- **SCHELLER et al.** *Nature Biotechnol.,* 2001, vol. 19, 573-577
- **ZHU et al.** *Nature Biotechnol.,* 2005, vol. 23, 1159-1169
- **LAIBLE et al.** *Reprod. Fertil. Dev.,* 2012, vol. 25 (1), 315
- Cancer, Principles and Practice of Oncology. J. B. Lippincott Co, 1993
- **LÖFFLER et al.** *Blood,* 2000, vol. 95 (6), 2098-2103
- **WARRENS et al.** *Gene,* 1997, vol. 186, 29-35